# EUROPEAN PATENT SPECIFICATION

(11) **EP 2 134 159 B1**
(45) Date of publication and mention of the grant of the patent: **21.06.2017**
(21) Application number: 08733094.0
(22) Date of filing: 03.04.2008
(51) Int. Cl.: A01H 5/00, A01H 5/10, C12N 15/82, C12N 9/88

(54) **AHAS MUTANTS**
AHAS-MUTANTEN
MUTANTS AHAS

(30) Priority: 04.04.2007 US 910028 P
(43) Date of publication of application: 23.12.2009
(60) Divisional application: 12180252.4
(73) Proprietor: BASF Plant Science GmbH, 67056 Ludwigshafen (DE)
(72) Inventor: MCELVER, John, A., Durham, NC 27712 (US); SINGH, Bijay, Cary, NC 27519 (US)
(74) Representative: Reitstötter Kinzebach
(86) International application number: PCT/US2008/059257
(87) International publication number: WO 2008/124495

(56) References cited:
- EP-A- 1 754 786
- WO-A-03/014356
- WO-A-2007/005581
- US-A1- 2003 097 692
- US-A1- 2006 010 514

### SEQUENCE LISTING

<110> McElver, John
   Singh, Bijay
<120> AHAS MUTANTS
<130> 038867/341792
<160> 34
<150> US 60/ 910,028
   <151> 2007-04-04
<170> PatentIn version 3.4
<210> 1
   <211> 670
   <212> PRT
   <213> Arabidopsis thaliana
<220>
   <221> MSC_FEATURE
   <223> Arabidopsis thaliana acetohydroxyacid synthase large subunit (At AHASL)
<400> 1
   Met Ala Ala Ala Thr Thr Thr Thr Thr Thr Ser Ser Ser Ile Ser Phe
   1               5                   10                  15
   Ser Thr Lys Pro Ser Pro Ser Ser Ser Lys Ser Pro Leu Pro Ile Ser
               20                  25                  30
   Arg Phe Ser Leu Pro Phe Ser Leu Asn Pro Asn Lys Ser Ser Ser Ser
           35                  40                  45
   Ser Arg Arg Arg Gly Ile Lys Ser Ser Ser Pro Ser Ser Ile Ser Ala
       50                  55                  60
   Val Leu Asn Thr Thr Thr Asn Val Thr Thr Thr Pro Ser Pro Thr Lys
   65                  70                  75                  80
   Pro Thr Lys Pro Glu Thr Phe Ile Ser Arg Phe Ala Pro Asp Gln Pro
                   85                  90                  95
   Arg Lys Gly Ala Asp Ile Leu Val Glu Ala Leu Glu Arg Gln Gly Val
               100                 105                 110
   Glu Thr Val Phe Ala Tyr Pro Gly Gly Ala Ser Met Glu Ile His Gln
           115                 120                 125
   Ala Leu Thr Arg Ser Ser Ser Ile Arg Asn Val Leu Pro Arg His Glu
       130                 135                 140
   Gln Gly Gly Val Phe Ala Ala Glu Gly Tyr Ala Arg Ser Ser Gly Lys
   145                 150                 155                 160
   Pro Gly Ile Cys Ile Ala Thr Ser Gly Pro Gly Ala Thr Asn Leu Val
   165 170 175
   Ser Gly Leu Ala Asp Ala Leu Leu Asp Ser Val Pro Leu Val Ala Ile
   180 185 190
   Thr Gly Gln Val Pro Arg Arg Met Ile Gly Thr Asp Ala Phe Gln Glu
   195 200 205
   Thr Pro Ile Val Glu Val Thr Arg Ser Ile Thr Lys His Asn Tyr Leu
   210 215 220
   Val Met Asp Val Glu Asp Ile Pro Arg Ile Ile Glu Glu Ala Phe Phe
   225 230 235 240
   Leu Ala Thr Ser Gly Arg Pro Gly Pro Val Leu Val Asp Val Pro Lys
   245 250 255
   Asp Ile Gln Gln Gln Leu Ala Ile Pro Asn Trp Glu Gln Ala Met Arg
   260 265 270
   Leu Pro Gly Tyr Met Ser Arg Met Pro Lys Pro Pro Glu Asp Ser His
   275 280 285
   Leu Glu Gln Ile Val Arg Leu Ile Ser Glu Ser Lys Lys Pro Val Leu
   290 295 300
   Tyr Val Gly Gly Gly Cys Leu Asn Ser Ser Asp Glu Leu Gly Arg Phe
   305 310 315 320
   Val Glu Leu Thr Gly Ile Pro Val Ala Ser Thr Leu Met Gly Leu Gly
   325 330 335
   Ser Tyr Pro Cys Asp Asp Glu Leu Ser Leu His Met Leu Gly Met His
   340 345 350
   Gly Thr Val Tyr Ala Asn Tyr Ala Val Glu His Ser Asp Leu Leu Leu
   355 360 365
   Ala Phe Gly Val Arg Phe Asp Asp Arg Val Thr Gly Lys Leu Glu Ala
   370 375 380
   Phe Ala Ser Arg Ala Lys Ile Val His Ile Asp Ile Asp Ser Ala Glu
   385 390 395 400
   Ile Gly Lys Asn Lys Thr Pro His Val Ser Val Cys Gly Asp Val Lys
   405 410 415
   Leu Ala Leu Gln Gly Met Asn Lys Val Leu Glu Asn Arg Ala Glu Glu
   420 425 430
   Leu Lys Leu Asp Phe Gly Val Trp Arg Asn Glu Leu Asn Val Gln Lys
   435 440 445
   Gln Lys Phe Pro Leu Ser Phe Lys Thr Phe Gly Glu Ala Ile Pro Pro
   450 455 460
   Gln Tyr Ala Ile Lys Val Leu Asp Glu Leu Thr Asp Gly Lys Ala Ile
   465 470 475 480
   Ile Ser Thr Gly Val Gly Gln His Gln Met Trp Ala Ala Gln Phe Tyr
   485 490 495
   Asn Tyr Lys Lys Pro Arg Gln Trp Leu Ser Ser Gly Gly Leu Gly Ala
   500 505 510
   Met Gly Phe Gly Leu Pro Ala Ala Ile Gly Ala Ser Val Ala Asn Pro
   515 520 525
   Asp Ala Ile Val Val Asp Ile Asp Gly Asp Gly Ser Phe Ile Met Asn
   530 535 540
   Val Gln Glu Leu Ala Thr Ile Arg Val Glu Asn Leu Pro Val Lys Val
   545 550 555 560
   Leu Leu Leu Asn Asn Gln His Leu Gly Met Val Met Gln Trp Glu Asp
   565 570 575
   Arg Phe Tyr Lys Ala Asn Arg Ala His Thr Phe Leu Gly Asp Pro Ala
   580 585 590
   Gln Glu Asp Glu Ile Phe Pro Asn Met Leu Leu Phe Ala Ala Ala Cys
   595 600 605
   Gly Ile Pro Ala Ala Arg Val Thr Lys Lys Ala Asp Leu Arg Glu Ala
   610 615 620
   Ile Gln Thr Met Leu Asp Thr Pro Gly Pro Tyr Leu Leu Asp Val Ile
   625 630 635 640
   Cys Pro His Gln Glu His Val Leu Pro Met Ile Pro Ser Gly Gly Thr
   645 650 655
   Phe Asn Asp Val Ile Thr Glu Gly Asp Gly Arg Ile Lys Tyr
   660 665 670
<210> 2
   <211> 638
   <212> PRT
   <213> Zea mays
<220>
   <221> MSC_FEATURE
   <223> Zea mays acetohydroxyacid synthase large subunit number two (ZmAHASL2)
<400> 2
   Met Ala Thr Ala Ala Ala Ala Ser Thr Ala Leu Thr Gly Ala Thr Thr
   1               5                   10                  15
   Ala Ala Pro Lys Ala Arg Arg Arg Ala His Leu Leu Ala Thr Arg Arg
               20                  25                  30
   Ala Leu Ala Ala Pro Ile Arg Cys Ser Ala Ala Ser Pro Ala Met Pro
           35                  40                  45
   Met Ala Pro Pro Ala Thr Pro Leu Arg Pro Trp Gly Pro Thr Asp Pro
       50                  55                  60
   Arg Lys Gly Ala Asp Ile Leu Val Glu Ser Leu Glu Arg Cys Gly Val
   65                  70                  75                  80
   Arg Asp Val Phe Ala Tyr Pro Gly Gly Ala Ser Met Glu Ile His Gln
                   85                  90                  95
   Ala Leu Thr Arg Ser Pro Val Ile Ala Asn His Leu Phe Arg His Glu
               100                 105                 110
   Gln Gly Glu Ala Phe Ala Ala Ser Gly Tyr Ala Arg Ser Ser Gly Arg
           115                 120                 125
   Val Gly Val Cys Ile Ala Thr Ser Gly Pro Gly Ala Thr Asn Leu Val
       130                 135                 140
   Ser Ala Leu Ala Asp Ala Leu Leu Asp Ser Val Pro Met Val Ala Ile
   145                 150                 155                 160
   Thr Gly Gln Val Pro Arg Arg Met Ile Gly Thr Asp Ala Phe Gln Glu
                   165                 170                 175
   Thr Pro Ile Val Glu Val Thr Arg Ser Ile Thr Lys His Asn Tyr Leu
               180                 185                 190
   Val Leu Asp Val Asp Asp Ile Pro Arg Val Val Gln Glu Ala Phe Phe
           195                 200                 205
   Leu Ala Ser Ser Gly Arg Pro Gly Pro Val Leu Val Asp Ile Pro Lys
       210                 215                 220
   Asp Ile Gln Gln Gln Met Ala Val Pro Val Trp Asp Lys Pro Met Ser
   225                 230                 235                 240
   Leu Pro Gly Tyr Ile Ala Arg Leu Pro Lys Pro Pro Ala Thr Glu Leu
                   245                 250                 255
   Leu Glu Gln Val Leu Arg Leu Val Gly Glu Ser Arg Arg Pro Val Leu
               260                 265                 270
   Tyr Val Gly Gly Gly Cys Ala Ala Ser Gly Glu Glu Leu Arg Arg Phe
   275 280 285
   Val Glu Leu Thr Gly Ile Pro Val Thr Thr Thr Leu Met Gly Leu Gly
   290 295 300
   Asn Phe Pro Ser Asp Asp Pro Leu Ser Leu Arg Met Leu Gly Met His
   305 310 315 320
   Gly Thr Val Tyr Ala Asn Tyr Ala Val Asp Lys Ala Asp Leu Leu Leu
   325 330 335
   Ala Leu Gly Val Arg Phe Asp Asp Arg Val Thr Gly Lys Ile Glu Ala
   340 345 350
   Phe Ala Ser Arg Ala Lys Ile Val His Val Asp Ile Asp Pro Ala Glu
   355 360 365
   Ile Gly Lys Asn Lys Gln Pro His Val Ser Ile Cys Ala Asp Val Lys
   370 375 380
   Leu Ala Leu Gln Gly Met Asn Ala Leu Leu Glu Gly Ser Thr Ser Lys
   385 390 395 400
   Lys Ser Phe Asp Phe Gly Ser Trp Asn Asp Glu Leu Asp Gln Gln Lys
   405 410 415
   Arg Glu Phe Pro Leu Gly Tyr Lys Thr Ser Asn Glu Glu Ile Gln Pro
   420 425 430
   Gln Tyr Ala Ile Gln Val Leu Asp Glu Leu Thr Lys Gly Glu Ala Ile
   435 440 445
   Ile Gly Thr Gly Val Gly Gln His Gln Met Trp Ala Ala Gln Tyr Tyr
   450 455 460
   Thr Tyr Lys Arg Pro Arg Gln Trp Leu Ser Ser Ala Gly Leu Gly Ala
   465 470 475 480
   Met Gly Phe Gly Leu Pro Ala Ala Ala Gly Ala Ser Val Ala Asn Pro
   485 490 495
   Gly Val Thr Val Val Asp Ile Asp Gly Asp Gly Ser Phe Leu Met Asn
   500 505 510
   Val Gln Glu Leu Ala Met Ile Arg Ile Glu Asn Leu Pro Val Lys Val
   515 520 525
   Phe Val Leu Asn Asn Gln His Leu Gly Met Val Val Gln Trp Glu Asp
   530 535 540
   Arg Phe Tyr Lys Ala Asn Arg Ala His Thr Tyr Leu Gly Asn Pro Glu
   545 550 555 560
   Asn Glu Ser Glu Ile Tyr Pro Asp Phe Val Thr Ile Ala Lys Gly Phe
   565 570 575
   Asn Ile Pro Ala Val Arg Val Thr Lys Lys Asn Glu Val Arg Ala Ala
   580 585 590
   Ile Lys Lys Met Leu Glu Thr Pro Gly Pro Tyr Leu Leu Asp Ile Ile
   595 600 605
   Val Pro His Gln Glu His Val Leu Pro Met Ile Pro Ser Gly Gly Ala
   610 615 620
   Phe Lys Asp Met Ile Leu Asp Gly Asp Gly Arg Thr Val Tyr
   625 630 635
<210> 3
   <211> 652
   <212> PRT
   <213> Brassica napus
<220>
   <221> MSC_FEATURE
   <223> Brassica napus A genome acetohydroxyacid synthase large subunit number one (BnAHASL1A)
<400> 3
   Met Ala Ala Ala Thr Ser Ser Ser Pro Ile Ser Leu Thr Ala Lys Pro
   1 5 10 15
   Ser Ser Lys Ser Pro Leu Pro Ile Ser Arg Phe Ser Leu Pro Phe Ser
   20 25 30
   Leu Thr Pro Gln Lys Pro Ser Ser Arg Leu His Arg Pro Leu Ala Ile
   35 40 45
   Ser Ala Val Leu Asn Ser Pro Val Asn Val Ala Pro Glu Lys Thr Asp
   50 55 60
   Lys Ile Lys Thr Phe Ile Ser Arg Tyr Ala Pro Asp Glu Pro Arg Lys
   65 70 75 80
   Gly Ala Asp Ile Leu Val Glu Ala Leu Glu Arg Gln Gly Val Glu Thr
   85 90 95
   Val Phe Ala Tyr Pro Gly Gly Ala Ser Met Glu Ile His Gln Ala Leu
   100 105 110
   Thr Arg Ser Ser Thr Ile Arg Asn Val Leu Pro Arg His Glu Gln Gly
   115 120 125
   Gly Val Phe Ala Ala Glu Gly Tyr Ala Arg Ser Ser Gly Lys Pro Gly
   130 135 140
   Ile Cys Ile Ala Thr Ser Gly Pro Gly Ala Thr Asn Leu Val Ser Gly
   145 150 155 160
   Leu Ala Asp Ala Met Leu Asp Ser Val Pro Leu Val Ala Ile Thr Gly
   165 170 175
   Gln Val Pro Arg Arg Met Ile Gly Thr Asp Ala Phe Gln Glu Thr Pro
   180 185 190
   Ile Val Glu Val Thr Arg Ser Ile Thr Lys His Asn Tyr Leu Val Met
   195 200 205
   Asp Val Asp Asp Ile Pro Arg Ile Val Gln Glu Ala Phe Phe Leu Ala
   210 215 220
   Thr Ser Gly Arg Pro Gly Pro Val Leu Val Asp Val Pro Lys Asp Ile
   225 230 235 240
   Gln Gln Gln Leu Ala Ile Pro Asn Trp Asp Gln Pro Met Arg Leu Pro
   245 250 255
   Gly Tyr Met Ser Arg Leu Pro Gln Pro Pro Glu Val Ser Gln Leu Gly
   260 265 270
   Gln Ile Val Arg Leu Ile Ser Glu Ser Lys Arg Pro Val Leu Tyr Val
   275 280 285
   Gly Gly Gly Ser Leu Asn Ser Ser Glu Glu Leu Gly Arg Phe Val Glu
   290 295 300
   Leu Thr Gly Ile Pro Val Ala Ser Thr Leu Met Gly Leu Gly Ser Tyr
   305 310 315 320
   Pro Cys Asn Asp Glu Leu Ser Leu Gln Met Leu Gly Met His Gly Thr
   325 330 335
   Val Tyr Ala Asn Tyr Ala Val Glu His Ser Asp Leu Leu Leu Ala Phe
   340 345 350
   Gly Val Arg Phe Asp Asp Arg Val Thr Gly Lys Leu Glu Ala Phe Ala
   355 360 365
   Ser Arg Ala Lys Ile Val His Ile Asp Ile Asp Ser Ala Glu Ile Gly
   370 375 380
   Lys Asn Lys Thr Pro His Val Ser Val Cys Gly Asp Val Lys Leu Ala
   385 390 395 400
   Leu Gln Gly Met Asn Lys Val Leu Glu Asn Arg Ala Glu Glu Leu Lys
   405 410 415
   Leu Asp Phe Gly Val Trp Arg Ser Glu Leu Ser Glu Gln Lys Gln Lys
   420 425 430
   Phe Pro Leu Ser Phe Lys Thr Phe Gly Glu Ala Ile Pro Pro Gln Tyr
   435 440 445
   Ala Ile Gln Val Leu Asp Glu Leu Thr Gln Gly Lys Ala Ile Ile Ser
   450 455 460
   Thr Gly Val Gly Gln His Gln Met Trp Ala Ala Gln Phe Tyr Lys Tyr
   465 470 475 480
   Arg Lys Pro Arg Gln Trp Leu Ser Ser Ser Gly Leu Gly Ala Met Gly
   485 490 495
   Phe Gly Leu Pro Ala Ala Ile Gly Ala Ser Val Ala Asn Pro Asp Ala
   500 505 510
   Ile Val Val Asp Ile Asp Gly Asp Gly Ser Phe Ile Met Asn Val Gln
   515 520 525
   Glu Leu Ala Thr Ile Arg Val Glu Asn Leu Pro Val Lys Ile Leu Leu
   530 535 540
   Leu Asn Asn Gln His Leu Gly Met Val Met Gln Trp Glu Asp Arg Phe
   545 550 555 560
   Tyr Lys Ala Asn Arg Ala His Thr Tyr Leu Gly Asp Pro Ala Arg Glu
   565 570 575
   Asn Glu Ile Phe Pro Asn Met Leu Gln Phe Ala Gly Ala Cys Gly Ile
   580 585 590
   Pro Ala Ala Arg Val Thr Lys Lys Glu Glu Leu Arg Glu Ala Ile Gln
   595 600 605
   Thr Met Leu Asp Thr Pro Gly Pro Tyr Leu Leu Asp Val Ile Cys Pro
   610 615 620
   His Gln Glu His Val Leu Pro Met Ile Pro Ser Gly Gly Thr Phe Lys
   625 630 635 640
   Asp Val Ile Thr Glu Gly Asp Gly Arg Thr Lys Tyr
   645 650
<210> 4
   <211> 644
   <212> PRT
   <213> Oryza sativa
<220>
   <221> MSC_FEATURE
   <223> Rice acetohydroxy acid synthase large subunit (OSAHASL)
<400> 4
   Met Ala Thr Thr Ala Ala Ala Ala Ala Ala Ala Leu Ser Ala Ala Ala
   1 5 10 15
   Thr Ala Lys Thr Gly Arg Lys Asn His Gln Arg His His Val Leu Pro
   20 25 30
   Ala Arg Gly Arg Val Gly Ala Ala Ala Val Arg Cys Ser Ala Val Ser
   35 40 45
   Pro Val Thr Pro Pro Ser Pro Ala Pro Pro Ala Thr Pro Leu Arg Pro
   50 55 60
   Trp Gly Pro Ala Glu Pro Arg Lys Gly Ala Asp Ile Leu Val Glu Ala
   65 70 75 80
   Leu Glu Arg Cys Gly Val Ser Asp Val Phe Ala Tyr Pro Gly Gly Ala
   85 90 95
   Ser Met Glu Ile His Gln Ala Leu Thr Arg Ser Pro Val Ile Thr Asn
   100 105 110
   His Leu Phe Arg His Glu Gln Gly Glu Ala Phe Ala Ala Ser Gly Tyr
   115 120 125
   Ala Arg Ala Ser Gly Arg Val Gly Val Cys Val Ala Thr Ser Gly Pro
   130 135 140
   Gly Ala Thr Asn Leu Val Ser Ala Leu Ala Asp Ala Leu Leu Asp Ser
   145 150 155 160
   Val Pro Met Val Ala Ile Thr Gly Gln Val Pro Arg Arg Met Ile Gly
   165 170 175
   Thr Asp Ala Phe Gln Glu Thr Pro Ile Val Glu Val Thr Arg Ser Ile
   180 185 190
   Thr Lys His Asn Tyr Leu Val Leu Asp Val Glu Asp Ile Pro Arg Val
   195 200 205
   Ile Gln Glu Ala Phe Phe Leu Ala Ser Ser Gly Arg Pro Gly Pro Val
   210 215 220
   Leu Val Asp Ile Pro Lys Asp Ile Gln Gln Gln Met Ala Val Pro Val
   225 230 235 240
   Trp Asp Thr Ser Met Asn Leu Pro Gly Tyr Ile Ala Arg Leu Pro Lys
   245 250 255
   Pro Pro Ala Thr Glu Leu Leu Glu Gln Val Leu Arg Leu Val Gly Glu
   260 265 270
   Ser Arg Arg Pro Ile Leu Tyr Val Gly Gly Gly Cys Ser Ala Ser Gly
   275 280 285
   Asp Glu Leu Arg Trp Phe Val Glu Leu Thr Gly Ile Pro Val Thr Thr
   290 295 300
   Thr Leu Met Gly Leu Gly Asn Phe Pro Ser Asp Asp Pro Leu Ser Leu
   305 310 315 320
   Arg Met Leu Gly Met His Gly Thr Val Tyr Ala Asn Tyr Ala Val Asp
   325 330 335
   Lys Ala Asp Leu Leu Leu Ala Phe Gly Val Arg Phe Asp Asp Arg Val
   340 345 350
   Thr Gly Lys Ile Glu Ala Phe Ala Ser Arg Ala Lys Ile Val His Ile
   355 360 365
   Asp Ile Asp Pro Ala Glu Ile Gly Lys Asn Lys Gln Pro His Val Ser
   370 375 380
   Ile Cys Ala Asp Val Lys Leu Ala Leu Gln Gly Leu Asn Ala Leu Leu
   385 390 395 400
   Gln Gln Ser Thr Thr Lys Thr Ser Ser Asp Phe Ser Ala Trp His Asn
   405 410 415
   Glu Leu Asp Gln Gln Lys Arg Glu Phe Pro Leu Gly Tyr Lys Thr Phe
   420 425 430
   Gly Glu Glu Ile Pro Pro Gln Tyr Ala Ile Gln Val Leu Asp Glu Leu
   435 440 445
   Thr Lys Gly Glu Ala Ile Ile Ala Thr Gly Val Gly Gln His Gln Met
   450 455 460
   Trp Ala Ala Gln Tyr Tyr Thr Tyr Lys Arg Pro Arg Gln Trp Leu Ser
   465 470 475 480
   Ser Ala Gly Leu Gly Ala Met Gly Phe Gly Leu Pro Ala Ala Ala Gly
   485 490 495
   Ala Ser Val Ala Asn Pro Gly Val Thr Val Val Asp Ile Asp Gly Asp
   500 505 510
   Gly Ser Phe Leu Met Asn Ile Gln Glu Leu Ala Leu Ile Arg Ile Glu
   515 520 525
   Asn Leu Pro Val Lys Val Met Val Leu Asn Asn Gln His Leu Gly Met
   530 535 540
   Val Val Gln Trp Glu Asp Arg Phe Tyr Lys Ala Asn Arg Ala His Thr
   545 550 555 560
   Tyr Leu Gly Asn Pro Glu Cys Glu Ser Glu Ile Tyr Pro Asp Phe Val
   565 570 575
   Thr Ile Ala Lys Gly Phe Asn Ile Pro Ala Val Arg Val Thr Lys Lys
   580 585 590
   Ser Glu Val Arg Ala Ala Ile Lys Lys Met Leu Glu Thr Pro Gly Pro
   595 600 605
   Tyr Leu Leu Asp Ile Ile Val Pro His Gln Glu His Val Leu Pro Met
   610 615 620
   Ile Pro Ser Gly Gly Ala Phe Lys Asp Met Ile Leu Asp Gly Asp Gly
   625 630 635 640
   Arg Thr Val Tyr
<210> 5
   <211> 655
   <212> PRT
   <213> Helianthus annuus
<220>
   <221> MSC_FEATURE
   <223> Sunflower acetohydroxy acid synthase large subunit number 1(HaAHASL1)
<400> 5
   Met Ala Ala Pro Pro Asn Pro Ser Ile Ser Phe Lys Pro Pro Ser Pro
   1 5 10 15
   Ala Ala Ala Leu Pro Pro Arg Ser Ala Phe Leu Pro Arg Phe Ala Leu
   20 25 30
   Pro Ile Thr Ser Thr Thr Gln Lys Arg His Arg Leu His Ile Ser Asn
   35 40 45
   Val Leu Ser Asp Ser Lys Ser Thr Thr Thr Thr Thr Thr Thr Thr Gln
   50 55 60
   Arg Pro Leu Pro Val Gln Pro Phe Val Ser Arg Tyr Ala Pro Asp Gln
   65 70 75 80
   Pro Arg Lys Gly Ala Asp Val Leu Val Glu Ala Leu Glu Arg Glu Gly
   85 90 95
   Val Thr Asp Val Phe Ala Tyr Pro Gly Gly Ala Ser Met Glu Ile His
   100 105 110
   Gln Ala Leu Thr Arg Ser Ser Thr Ile Arg Asn Val Leu Pro Arg His
   115 120 125
   Glu Gln Gly Gly Val Phe Ala Ala Glu Gly Tyr Ala Arg Ala Ser Gly
   130 135 140
   Leu Pro Gly Val Cys Ile Ala Thr Ser Gly Pro Gly Ala Thr Asn Leu
   145 150 155 160
   Val Ser Gly Leu Ala Asp Ala Leu Leu Asp Ser Val Pro Met Val Ala
   165 170 175
   Ile Thr Gly Gln Val Pro Arg Arg Met Ile Gly Thr Asp Ala Phe Gln
   180 185 190
   Glu Thr Pro Ile Val Glu Val Thr Arg Ser Ile Thr Lys His Asn Tyr
   195 200 205
   Leu Val Leu Asp Val Glu Asp Ile Pro Arg Ile Val Arg Glu Ala Phe
   210 215 220
   Tyr Leu Ala Ser Ser Gly Arg Pro Gly Pro Val Leu Ile Asp Val Pro
   225 230 235 240
   Lys Asp Ile Gln Gln Gln Leu Val Val Pro Lys Trp Asp Glu Pro Met
   245 250 255
   Arg Leu Pro Gly Tyr Leu Ser Arg Met Pro Lys Pro Gln Tyr Asp Gly
   260 265 270
   His Leu Glu Gln Ile Val Arg Leu Val Gly Glu Ala Lys Arg Pro Val
   275 280 285
   Leu Tyr Val Gly Gly Gly Cys Leu Asn Ser Asp Asp Glu Leu Arg Arg
   290 295 300
   Phe Val Glu Leu Thr Gly Ile Pro Val Ala Ser Thr Leu Met Gly Leu
   305 310 315 320
   Gly Ala Tyr Pro Ala Ser Ser Asp Leu Ser Leu His Met Leu Gly Met
   325 330 335
   His Gly Thr Val Tyr Ala Asn Tyr Ala Val Asp Lys Ser Asp Leu Leu
   340 345 350
   Leu Ala Phe Gly Val Arg Phe Asp Asp Arg Val Thr Gly Lys Leu Glu
   355 360 365
   Ala Phe Ala Ser Arg Ala Lys Ile Val His Ile Asp Ile Asp Pro Ala
   370 375 380
   Glu Ile Gly Lys Asn Lys Gln Pro His Val Ser Ile Cys Gly Asp Ile
   385 390 395 400
   Lys Val Ala Leu Gln Gly Leu Asn Lys Ile Leu Glu Glu Lys Asn Ser
   405 410 415
   Val Thr Asn Leu Asp Phe Ser Thr Trp Arg Lys Glu Leu Asp Glu Gln
   420 425 430
   Lys Met Lys Phe Pro Leu Ser Phe Lys Thr Phe Gly Glu Ala Ile Pro
   435 440 445
   Pro Gln Tyr Ala Ile Gln Val Leu Asp Glu Leu Thr Gly Gly Asn Ala
   450 455 460
   Ile Ile Ser Thr Gly Val Gly Gln His Gln Met Trp Ala Ala Gln Phe
   465 470 475 480
   Tyr Lys Tyr Asn Lys Pro Arg Gln Trp Leu Thr Ser Gly Gly Leu Gly
   485 490 495
   Ala Met Gly Phe Gly Leu Pro Ala Ala Ile Gly Ala Ala Val Ala Arg
   500 505 510
   Pro Asp Ala Val Val Val Asp Ile Asp Gly Asp Gly Ser Phe Met Met
   515 520 525
   Asn Val Gln Glu Leu Ala Thr Ile Arg Val Glu Asn Leu Pro Val Lys
   530 535 540
   Ile Leu Leu Leu Asn Asn Gln His Leu Gly Met Val Val Gln Trp Glu
   545 550 555 560
   Asp Arg Phe Tyr Lys Ala Asn Arg Ala His Thr Tyr Leu Gly Asn Pro
   565 570 575
   Ser Lys Glu Ser Glu Ile Phe Pro Asn Met Val Lys Phe Ala Glu Ala
   580 585 590
   Cys Asp Ile Pro Ala Ala Arg Val Thr Gln Lys Ala Asp Leu Arg Ala
   595 600 605
   Ala Ile Gln Lys Met Leu Asp Thr Pro Gly Pro Tyr Leu Leu Asp Val
   610 615 620
   Ile Val Pro His Gln Glu His Val Leu Pro Met Ile Pro Ala Gly Gly
   625 630 635 640
   Gly Phe Ser Asp Val Ile Thr Glu Gly Asp Gly Arg Thr Lys Tyr
   645 650 655
<210> 6
   <211> 646
   <212> PRT
   <213> Helianthus annuus
<220>
   <221> MSC_FEATURE
   <223> Sunflower acetohydroxy acid synthase large subunit number 2(HaAHASL2)
<400> 6
   Met Ala Ala Ile His Pro Pro His Pro Ser Ile Thr Ala Lys Pro Pro
   1               5                   10                  15
   Pro Ser Ser Ala Ala Ala Val Ala Leu Pro Pro His Phe Ala Phe Ser
               20                  25                  30
   Ile Thr Ser Thr Ser His Lys Arg His Arg Leu His Ile Ser Asn Val
           35                  40                  45
   Leu Ser Asp Ser Thr Thr Thr Thr Gly Ala Thr Thr Ile His Pro Pro
       50                  55                  60
   Pro Phe Val Ser Arg Tyr Ala Pro Asp Gln Pro Arg Lys Gly Ala Asp
   65                  70                  75                  80
   Val Leu Val Glu Ala Leu Glu Arg Glu Gly Val Thr Asp Val Phe Ala
                   85                  90                  95
   Tyr Pro Gly Gly Ala Ser Met Glu Ile His Gln Ala Leu Thr Arg Ser
               100                 105                 110
   Asn Thr Ile Arg Asn Val Leu Pro Arg His Glu Gln Gly Gly Val Phe
           115                 120                 125
   Ala Ala Glu Gly Tyr Ala Arg Ala Ser Gly Val Pro Gly Val Cys Ile
       130                 135                 140
   Ala Thr Ser Gly Pro Gly Ala Thr Asn Leu Val Ser Gly Leu Ala Asp
   145                 150                 155                 160
   Ala Leu Leu Asp Ser Val Pro Met Val Ala Ile Thr Gly Gln Val Pro
                   165                 170                 175
   Arg Arg Met Ile Gly Thr Asp Ala Phe Gln Glu Thr Pro Ile Val Glu
               180                 185                 190
   Val Thr Arg Ser Ile Thr Lys His Asn Tyr Leu Val Leu Asp Val Glu
           195                 200                 205
   Asp Ile Pro Arg Ile Val Arg Glu Ala Phe Tyr Leu Ala Ser Ser Gly
   210 215 220
   Arg Pro Gly Pro Val Leu Ile Asp Val Pro Lys Asp Ile Gln Gln Gln
   225 230 235 240
   Leu Val Val Pro Lys Trp Asp Glu Pro Met Arg Leu Pro Gly Tyr Leu
   245 250 255
   Ser Arg Met Pro Lys Pro Gln Tyr Asp Gly His Leu Glu Gln Ile Val
   260 265 270
   Arg Leu Val Gly Glu Ala Lys Arg Pro Val Leu Tyr Val Gly Gly Gly
   275 280 285
   Cys Leu Asn Ser Asp Asp Glu Leu Arg Arg Phe Val Glu Leu Thr Gly
   290 295 300
   Ile Pro Val Ala Ser Thr Leu Met Gly Leu Gly Ala Tyr Pro Ala Ser
   305 310 315 320
   Ser Asp Leu Ser Leu His Met Leu Gly Met His Gly Thr Val Tyr Ala
   325 330 335
   Asn Tyr Ala Val Asp Lys Ser Asp Leu Leu Leu Ala Phe Gly Val Arg
   340 345 350
   Phe Asp Asp Arg Val Thr Gly Lys Leu Glu Ala Phe Ala Ser Arg Ala
   355 360 365
   Lys Ile Val His Ile Asp Ile Asp Pro Ala Glu Ile Gly Lys Asn Lys
   370 375 380
   Gln Pro His Val Ser Ile Cys Gly Asp Ile Lys Val Ala Leu Gln Gly
   385 390 395 400
   Leu Asn Lys Ile Leu Glu Glu Lys Asn Ser Leu Asp Phe Ser Asn Trp
   405 410 415
   Arg Lys Glu Leu Asp Glu Gln Lys Val Lys Phe Pro Leu Ser Phe Lys
   420 425 430
   Thr Phe Gly Glu Ala Ile Pro Pro Gln Tyr Ala Ile His Val Leu Asp
   435 440 445
   Glu Leu Thr Gly Gly Asn Ala Ile Ile Ser Thr Gly Val Gly Gln His
   450 455 460
   Gln Met Trp Ala Ala Gln Phe Tyr Lys Tyr Asn Lys Pro Arg Gln Trp
   465 470 475 480
   Leu Thr Ser Gly Gly Leu Gly Ala Met Gly Phe Gly Leu Pro Ala Ala
   485 490 495
   Ile Gly Ala Ala Val Ala Arg Pro Asp Ala Val Val Val Asp Ile Asp
   500 505 510
   Gly Asp Gly Ser Phe Met Met Asn Val Gln Glu Leu Ala Thr Ile Arg
   515 520 525
   Val Glu Asn Leu Pro Val Lys Ile Leu Leu Leu Asn Asn Gln His Leu
   530 535 540
   Gly Met Val Val Gln Trp Glu Asp Arg Phe Tyr Lys Ala Asn Arg Ala
   545 550 555 560
   His Thr Tyr Leu Gly Asn Pro Ser Lys Glu Ser Glu Ile Phe Pro Asn
   565 570 575
   Met Leu Lys Phe Ala Glu Ala Cys Asp Ile Pro Ala Ala Arg Val Thr
   580 585 590
   Arg Lys Gly Asp Leu Arg Ala Ala Ile Gln Lys Met Leu Asp Thr Pro
   595 600 605
   Gly Pro Tyr Leu Leu Asp Val Ile Val Pro His Gln Glu His Val Leu
   610 615 620
   Pro Met Ile Pro Ala Gly Gly Gly Phe Ser Asp Val Ile Thr Glu Gly
   625 630 635 640
   Asp Gly Arg Met Lys Tyr
   645
<210> 7
   <211> 646
   <212> PRT
   <213> Helianthus annuus
<220>
   <221> misc_feature
   <223> Sunflower acetohydroxy acid synthase large subunit number 3(HaAHASL3)
<400> 7
   Met Ala Val Pro Leu Thr Phe Ile Ser Gly Lys Pro Pro Phe Ser Ala
   1 5 10 15
   Thr Pro Ser Gln Leu Thr Thr Asn Leu Ser Leu Pro Leu Thr Leu Leu
   20 25 30
   Pro Ile Ile Pro Ser Lys Thr His Val Ser Lys His Leu Ile Ile Thr
   35 40 45
   Asn Ala Ile Ala Lys His Ser His Ser His Lys Ala Phe Val Val Ser
   50 55 60
   Arg Phe Gly Pro Asp Glu Pro Arg Lys Gly Ser Asp Val Leu Val Glu
   65 70 75 80
   Ala Leu Glu Arg Glu Gly Val Thr Asn Val Phe Ala Tyr Pro Gly Gly
   85 90 95
   Ala Thr Leu Glu Ile His Gln Ala Leu Thr Arg Ser Thr Ile Ile Arg
   100 105 110
   Asn Ile Leu Pro Arg His Glu Gln Gly Gly Val Phe Ala Ala Glu Gly
   115 120 125
   Tyr Ala Arg Ala Ser Gly Leu Thr Gly Val Cys Ile Ser Thr Ser Gly
   130 135 140
   Pro Gly Ala Thr Asn Leu Val Ser Gly Leu Ala Asp Ala Leu Leu Asp
   145 150 155 160
   Ser Val Pro Ile Val Ala Ile Thr Gly Gln Val Pro Arg Asn Met Ile
   165 170 175
   Gly Thr Asp Ala Phe Gln Glu Thr Pro Ile Val Glu Val Ser Arg Ser
   180 185 190
   Ile Thr Lys His Asn Tyr Leu Val Leu Asn Val Gln Asp Ile Pro Arg
   195 200 205
   Ile Val His Glu Ala Phe Phe Leu Ala Ser Ser Gly Arg Pro Gly Pro
   210 215 220
   Val Leu Ile Asp Ile Pro Lys Asp Ile Gln Gln Gln Leu Val Val Pro
   225 230 235 240
   Asn Trp Asp Glu Gln Gln Gln Pro Met Arg Leu Asp Gly Tyr Ile Ser
   245 250 255
   Arg Leu Pro Lys Pro Pro Asn Glu Thr His Leu Arg Gln Ile Val Arg
   260 265 270
   Phe Ile Lys Glu Ser Lys Arg Pro Val Leu Tyr Val Gly Gly Gly Cys
   275 280 285
   Met Asn Ser Ser Asp Glu Leu Gly Arg Phe Val Glu Leu Thr Gly Ile
   290 295 300
   Pro Val Ala Asn Thr Leu Met Gly Leu Gly Thr Tyr Pro Gly Ser His
   305 310 315 320
   Asp Leu Ser Leu His Met Leu Gly Met His Gly Thr Val Tyr Ala Asn
   325 330 335
   Tyr Ala Ile Asp Lys Ser Asp Leu Leu Leu Ala Phe Gly Val Arg Phe
   340 345 350
   Asp Asp Arg Val Thr Gly Lys Ile Glu Ala Phe Ala Ser Arg Ala Lys
   355 360 365
   Ile Val His Ile Asp Ile Asp Pro Ala Glu Ile Gly Lys Asn Lys Gln
   370 375 380
   Pro His Phe Ser Ile Cys Gly Asp Ile Lys Ala Ala Leu Gln Gly Leu
   385 390 395 400
   Asn Lys Ile Leu Glu Arg Gly Glu Asp Leu Asp Phe Asp Phe Ser Pro
   405 410 415
   Trp Lys Glu Glu Val Met Asn Gln Lys Ala Ser Asn Pro Leu Ser Tyr
   420 425 430
   Lys Thr Phe Gly Asp Ala Ile Pro Pro Gln Tyr Ala Ile Gln Val Leu
   435 440 445
   Asn Glu Val Thr Gly Gly Asn Ala Ile Ile Thr Thr Gly Val Gly Gln
   450 455 460
   His Gln Met Trp Ser Ala Gln Phe Tyr Lys Tyr Asn Arg Pro Arg Gln
   465 470 475 480
   Trp Leu Thr Ser Ala Gly Leu Gly Ala Met Gly Phe Gly Leu Pro Ala
   485 490 495
   Ala Ile Gly Ala Ala Val Ala Arg Pro Asp Ala Val Val Val Asp Ile
   500 505 510
   Asp Gly Asp Gly Ser Phe Met Met Asn Val Gln Glu Leu Ala Thr Ile
   515 520 525
   Arg Val Glu Asn Leu Pro Val Lys Met Met Val Leu Asn Asn Gln His
   530 535 540
   Leu Gly Met Val Val Gln Trp Glu Asp Arg Phe Tyr Lys Ala Asn Arg
   545 550 555 560
   Ala His Thr Tyr Leu Gly Asn Pro Thr Asn Glu Ser Gly Ile Phe Pro
   565 570 575
   Asn Met Leu Lys Phe Ala Glu Ala Cys Asp Ile Pro Ala Ala Arg Val
   580 585 590
   Thr Lys Lys Gly Asp Val Arg Thr Ala Ile Gln Lys Met Leu Asp Thr
   595 600 605
   Pro Gly Pro Tyr Leu Leu Asp Val Ile Val Pro His Gln Glu His Val
   610 615 620
   Leu Pro Met Ile Pro Ala Gly Gly Gly Phe Asn Asp Ile Ile Thr Asp
   625 630 635 640
   Gly Asp Gly Arg Thr Gln
   645
<210> 8
   <211> 638
   <212> PRT
   <213> Zea mays
<220>
   <221> MSC_FEATURE
   <223> Zea mays acetohydroxy acid synthase large subunit number 1(ZmAHASL1)
<400> 8
   Met Ala Thr Ala Ala Thr Ala Ala Ala Ala Leu Thr Gly Ala Thr Thr
   1 5 10 15
   Ala Thr Pro Lys Ser Arg Arg Arg Ala His His Leu Ala Thr Arg Arg
   20 25 30
   Ala Leu Ala Ala Pro Ile Arg Cys Ser Ala Leu Ser Arg Ala Thr Pro
   35 40 45
   Thr Ala Pro Pro Ala Thr Pro Leu Arg Pro Trp Gly Pro Asn Glu Pro
   50 55 60
   Arg Lys Gly Ser Asp Ile Leu Val Glu Ala Leu Glu Arg Cys Gly Val
   65 70 75 80
   Arg Asp Val Phe Ala Tyr Pro Gly Gly Ala Ser Met Glu Ile His Gln
   85 90 95
   Ala Leu Thr Arg Ser Pro Val Ile Ala Asn His Leu Phe Arg His Glu
   100 105 110
   Gln Gly Glu Ala Phe Ala Ala Ser Ala Tyr Ala Arg Ser Ser Gly Arg
   115 120 125
   Val Gly Val Cys Ile Ala Thr Ser Gly Pro Gly Ala Thr Asn Leu Val
   130 135 140
   Ser Ala Leu Ala Asp Ala Leu Leu Asp Ser Val Pro Met Val Ala Ile
   145 150 155 160
   Thr Gly Gln Val Pro Arg Arg Met Ile Gly Thr Asp Ala Phe Gln Glu
   165 170 175
   Thr Pro Ile Val Glu Val Thr Arg Ser Ile Thr Lys His Asn Tyr Leu
   180 185 190
   Val Leu Asp Val Asp Asp Ile Pro Arg Val Val Gln Glu Ala Phe Phe
   195 200 205
   Leu Ala Ser Ser Gly Arg Pro Gly Pro Val Leu Val Asp Ile Pro Lys
   210 215 220
   Asp Ile Gln Gln Gln Met Ala Val Pro Ala Trp Asp Thr Pro Met Ser
   225 230 235 240
   Leu Pro Gly Tyr Ile Ala Arg Leu Pro Lys Pro Pro Ala Thr Glu Phe
   245 250 255
   Leu Glu Gln Val Leu Arg Leu Val Gly Glu Ser Arg Arg Pro Val Leu
   260 265 270
   Tyr Val Gly Gly Gly Cys Ala Ala Ser Gly Glu Glu Leu Cys Arg Phe
   275 280 285
   Val Glu Leu Thr Gly Ile Pro Val Thr Thr Thr Leu Met Gly Leu Gly
   290 295 300
   Asn Phe Pro Ser Asp Asp Pro Leu Ser Leu Arg Met Leu Gly Met His
   305 310 315 320
   Gly Thr Val Tyr Ala Asn Tyr Ala Val Asp Lys Ala Asp Leu Leu Leu
   325 330 335
   Ala Phe Gly Val Arg Phe Asp Asp Arg Val Thr Gly Lys Ile Glu Ala
   340 345 350
   Phe Ala Gly Arg Ala Lys Ile Val His Ile Asp Ile Asp Pro Ala Glu
   355 360 365
   Ile Gly Lys Asn Lys Gln Pro His Val Ser Ile Cys Ala Asp Val Lys
   370 375 380
   Leu Ala Leu Gln Gly Met Asn Thr Leu Leu Glu Gly Ser Thr Ser Lys
   385 390 395 400
   Lys Ser Phe Asp Phe Gly Ser Trp His Asp Glu Leu Asp Gln Gln Lys
   405 410 415
   Arg Glu Phe Pro Leu Gly Tyr Lys Ile Phe Asn Glu Glu Ile Gln Pro
   420 425 430
   Gln Tyr Ala Ile Gln Val Leu Asp Glu Leu Thr Lys Gly Glu Ala Ile
   435 440 445
   Ile Ala Thr Gly Val Gly Gln His Gln Met Trp Ala Ala Gln Tyr Tyr
   450 455 460
   Thr Tyr Lys Arg Pro Arg Gln Trp Leu Ser Ser Ala Gly Leu Gly Ala
   465 470 475 480
   Met Gly Phe Gly Leu Pro Ala Ala Ala Gly Ala Ala Val Ala Asn Pro
   485 490 495
   Gly Val Thr Val Val Asp Ile Asp Gly Asp Gly Ser Phe Leu Met Asn
   500 505 510
   Ile Gln Glu Leu Ala Met Ile Arg Ile Glu Asn Leu Pro Val Lys Val
   515 520 525
   Phe Val Leu Asn Asn Gln His Leu Gly Met Val Val Gln Trp Glu Asp
   530 535 540
   Arg Phe Tyr Lys Ala Asn Arg Ala His Thr Phe Leu Gly Asn Pro Glu
   545 550 555 560
   Asn Glu Ser Glu Ile Tyr Pro Asp Phe Val Ala Ile Ala Lys Gly Phe
   565 570 575
   Asn Ile Pro Ala Val Arg Val Thr Lys Lys Ser Glu Val His Ala Ala
   580 585 590
   Ile Lys Lys Met Leu Glu Ala Pro Gly Pro Tyr Leu Leu Asp Ile Ile
   595 600 605
   Val Pro His Gln Glu His Val Leu Pro Met Ile Pro Ser Gly Gly Ala
   610 615 620
   Phe Lys Asp Met Ile Leu Asp Gly Asp Gly Arg Thr Val Tyr
   625 630 635
<210> 9
   <211> 665
   <212> PRT
   <213> Amaranthus sp.
<220>
   <221> MSC_FEATURE
   <223> Amaranthus acetohydroxy acid synthase large subunit (AsAHASL)
<400> 9
   Met Val Thr Leu Asn His Ile Ser Ser Phe Thr Lys Pro Asn Lys Thr
   1               5                   10                  15
   Tyr Leu Gln Ser Ser Ile Tyr Ala Ile Pro Phe Ser Asn Ser Leu Lys
               20                  25                  30
   Pro Thr Ser Ser Ser Ser Ile Leu Ala Ala Leu Phe Lys Ser His His
           35                  40                  45
   Leu Leu Leu Gln Ser Pro Lys Pro Lys Pro Pro Ser Ala Thr Ile Thr
   50 55 60
   Gln Ser Pro Ser Ser Leu Thr Asp Asp Lys Pro Ser Ser Phe Val Ser
   65 70 75 80
   Arg Phe Ser Pro Glu Glu Pro Arg Lys Gly Cys Asp Val Leu Val Glu
   85 90 95
   Ala Leu Glu Arg Glu Gly Val Thr Asp Val Phe Ala Tyr Pro Gly Gly
   100 105 110
   Ala Ser Met Glu Ile His Gln Ala Leu Thr Arg Ser Asn Ile Ile Arg
   115 120 125
   Asn Val Leu Pro Arg His Glu Gln Gly Gly Val Phe Ala Ala Gln Gly
   130 135 140
   Tyr Ala Arg Ala Thr Gly Arg Val Gly Val Cys Ile Ala Thr Ser Gly
   145 150 155 160
   Pro Gly Ala Thr Asn Leu Val Ser Gly Phe Ala Asp Ala Leu Leu Asp
   165 170 175
   Ser Val Pro Leu Val Ala Ile Thr Gly Gln Val Pro Arg Arg Met Ile
   180 185 190
   Gly Thr Asp Ala Phe Gln Glu Thr Pro Ile Val Glu Val Thr Arg Ser
   195 200 205
   Ile Thr Lys His Asn Tyr Leu Val Leu Asp Val Glu Asp Ile Pro Arg
   210 215 220
   Ile Val Lys Glu Ala Phe Phe Leu Ala Asn Ser Gly Arg Pro Gly Pro
   225 230 235 240
   Val Leu Ile Asp Ile Pro Lys Asp Ile Gln Gln Gln Leu Val Val Pro
   245 250 255
   Asn Trp Glu Gln Pro Ile Lys Leu Gly Gly Tyr Leu Ser Arg Leu Pro
   260 265 270
   Lys Pro Thr Phe Ser Ala Asn Glu Glu Gly Leu Leu Asp Gln Ile Val
   275 280 285
   Arg Leu Val Gly Glu Ser Lys Arg Pro Val Leu Tyr Thr Gly Gly Gly
   290 295 300
   Cys Leu Asn Ser Ser Glu Glu Leu Arg Lys Phe Val Glu Leu Thr Gly
   305 310 315 320
   Ile Pro Val Ala Ser Thr Leu Met Gly Leu Gly Ala Phe Pro Cys Thr
   325 330 335
   Asp Asp Leu Ser Leu Gln Met Leu Gly Met His Gly Thr Val Tyr Ala
   340 345 350
   Asn Tyr Ala Val Asp Lys Ala Asp Leu Leu Leu Ala Phe Gly Val Arg
   355 360 365
   Phe Asp Asp Arg Val Thr Gly Lys Leu Glu Ala Phe Ala Ser Arg Ala
   370 375 380
   Lys Ile Val His Ile Asp Ile Asp Ser Ala Glu Ile Gly Lys Asn Lys
   385 390 395 400
   Gln Pro His Val Ser Ile Cys Gly Glu Ile Lys Val Ala Leu Gln Gly
   405 410 415
   Leu Asn Lys Ile Leu Glu Ser Arg Lys Gly Lys Leu Lys Leu Asp Phe
   420 425 430
   Ser Asn Trp Arg Glu Glu Leu Asn Glu Gln Lys Lys Lys Phe Pro Leu
   435 440 445
   Ser Phe Lys Ser Phe Gly Asp Ala Ile Pro Pro Gln Tyr Ala Ile Gln
   450 455 460
   Val Leu Asp Glu Leu Thr Leu Gly Asp Ala Ile Val Ser Thr Gly Val
   465 470 475 480
   Gly Gln His Gln Met Trp Ala Ala Gln Phe Tyr Lys Tyr Arg Asn Pro
   485 490 495
   Arg Gln Trp Leu Thr Ser Gly Gly Leu Gly Ala Met Gly Phe Gly Leu
   500 505 510
   Pro Ala Cys Tyr Gly Ala Ala Val Ala Arg Pro Asp Ala Val Val Val
   515 520 525
   Asp Ile Asp Gly Asp Gly Ser Phe Ile Met Asn Val Gln Glu Leu Ala
   530 535 540
   Thr Ile Arg Val Glu Asn Leu Pro Val Lys Ile Met Leu Leu Asn Asn
   545 550 555 560
   Gln His Leu Gly Met Val Val Gln Trp Glu Asp Arg Phe Tyr Lys Ala
   565 570 575
   Asn Arg Ala His Thr Tyr Leu Gly Asn Pro Ser Asn Ser Ser Glu Ile
   580 585 590
   Phe Pro Asp Met Leu Lys Phe Ala Glu Ala Cys Asp Ile Pro Ala Ala
   595 600 605
   Arg Val Thr Lys Val Ser Asp Leu Arg Ala Ala Ile Gln Thr Met Leu
   610 615 620
   Asp Thr Pro Gly Pro Tyr Leu Leu Asp Val Ile Val Pro His Gln Glu
   625 630 635 640
   His Val Leu Pro Met Ile Pro Ser Gly Ala Ala Phe Lys Asp Thr Ile
   645 650 655
   Thr Glu Gly Asp Gly Arg Arg Ala Tyr
   660 665
<210> 10
   <211> 655
   <212> PRT
   <213> Brassica napus
<220>
   <221> MSC_FEATURE
   <223> Brassica napus acetohydroxy acid synthase C genome large subunit number 1 (BnAHASL1C)
<400> 10
   Met Ala Ala Ala Thr Ser Ser Ser Pro Ile Ser Leu Thr Ala Lys Pro
   1 5 10 15
   Ser Ser Lys Ser Pro Leu Pro Ile Ser Arg Phe Ser Leu Pro Phe Ser
   20 25 30
   Leu Thr Pro Gln Lys Asp Ser Ser Arg Leu His Arg Pro Leu Ala Ile
   35 40 45
   Ser Ala Val Leu Asn Ser Pro Val Asn Val Ala Pro Pro Ser Pro Glu
   50 55 60
   Lys Thr Asp Lys Asn Lys Thr Phe Val Ser Arg Tyr Ala Pro Asp Glu
   65 70 75 80
   Pro Arg Lys Gly Ala Asp Ile Leu Val Glu Ala Leu Glu Arg Gln Gly
   85 90 95
   Val Glu Thr Val Phe Ala Tyr Pro Gly Gly Ala Ser Met Glu Ile His
   100 105 110
   Gln Ala Leu Thr Arg Ser Ser Thr Ile Arg Asn Val Leu Pro Arg His
   115 120 125
   Glu Gln Gly Gly Val Phe Ala Ala Glu Gly Tyr Ala Arg Ser Ser Gly
   130 135 140
   Lys Pro Gly Ile Cys Ile Ala Thr Ser Gly Pro Gly Ala Thr Asn Leu
   145 150 155 160
   Val Ser Gly Leu Ala Asp Ala Met Leu Asp Ser Val Pro Leu Val Ala
   165 170 175
   Ile Thr Gly Gln Val Pro Arg Arg Met Ile Gly Thr Asp Ala Phe Gln
   180 185 190
   Glu Thr Pro Ile Val Glu Val Thr Arg Ser Ile Thr Lys His Asn Tyr
   195 200 205
   Leu Val Met Asp Val Asp Asp Ile Pro Arg Ile Val Gln Glu Ala Phe
   210 215 220
   Phe Leu Ala Thr Ser Gly Arg Pro Gly Pro Val Leu Val Asp Val Pro
   225 230 235 240
   Lys Asp Ile Gln Gln Gln Leu Ala Ile Pro Asn Trp Asp Gln Pro Met
   245 250 255
   Arg Leu Pro Gly Tyr Met Ser Arg Leu Pro Gln Pro Pro Glu Val Ser
   260 265 270
   Gln Leu Gly Gln Ile Val Arg Leu Ile Ser Glu Ser Lys Arg Pro Val
   275 280 285
   Leu Tyr Val Gly Gly Gly Ser Leu Asn Ser Ser Glu Glu Leu Gly Arg
   290 295 300
   Phe Val Glu Leu Thr Gly Ile Pro Val Ala Ser Thr Leu Met Gly Leu
   305 310 315 320
   Gly Ser Tyr Pro Cys Asn Asp Glu Leu Ser Leu Gln Met Leu Gly Met
   325 330 335
   His Gly Thr Val Tyr Ala Asn Tyr Ala Val Glu His Ser Asp Leu Leu
   340 345 350
   Leu Ala Phe Gly Val Arg Phe Asp Asp Arg Val Thr Gly Lys Leu Glu
   355 360 365
   Ala Phe Ala Ser Arg Ala Lys Ile Val His Ile Asp Ile Asp Ser Ala
   370 375 380
   Glu Ile Gly Lys Asn Lys Thr Pro His Val Ser Val Cys Gly Asp Val
   385 390 395 400
   Lys Leu Ala Leu Gln Gly Met Asn Lys Val Leu Glu Asn Arg Ala Glu
   405 410 415
   Glu Leu Lys Leu Asp Phe Gly Val Trp Arg Ser Glu Leu Ser Glu Gln
   420 425 430
   Lys Gln Lys Phe Pro Leu Ser Phe Lys Thr Phe Gly Glu Ala Ile Pro
   435 440 445
   Pro Gln Tyr Ala Ile Gln Ile Leu Asp Glu Leu Thr Glu Gly Lys Ala
   450 455 460
   Ile Ile Ser Thr Gly Val Gly Gln His Gln Met Trp Ala Ala Gln Phe
   465 470 475 480
   Tyr Lys Tyr Arg Lys Pro Arg Gln Trp Leu Ser Ser Ser Gly Leu Gly
   485 490 495
   Ala Met Gly Phe Gly Leu Pro Ala Ala Ile Gly Ala Ser Val Ala Asn
   500 505 510
   Pro Asp Ala Ile Val Val Asp Ile Asp Gly Asp Gly Ser Phe Ile Met
   515 520 525
   Asn Val Gln Glu Leu Ala Thr Ile Arg Val Glu Asn Leu Pro Val Lys
   530 535 540
   Ile Leu Leu Leu Asn Asn Gln His Leu Gly Met Val Met Gln Trp Glu
   545 550 555 560
   Asp Arg Phe Tyr Lys Ala Asn Arg Ala His Thr Tyr Leu Gly Asp Pro
   565 570 575
   Ala Arg Glu Asn Glu Ile Phe Pro Asn Met Leu Gln Phe Ala Gly Ala
   580 585 590
   Cys Gly Ile Pro Ala Ala Arg Val Thr Lys Lys Glu Glu Leu Arg Glu
   595 600 605
   Ala Ile Gln Thr Met Leu Asp Thr Pro Gly Pro Tyr Leu Leu Asp Val
   610 615 620
   Ile Cys Pro His Gln Glu His Val Leu Pro Met Ile Pro Ser Gly Gly
   625 630 635 640
   Thr Phe Lys Asp Val Ile Thr Glu Gly Asp Gly Arg Thr Lys Tyr
   645 650 655
<210> 11
   <211> 637
   <212> PRT
   <213> Brassica napus
<220>
   <221> MSC_FEATURE
   <223> Brassica napus acetohydroxy acid synthase A genome large subunit number 2 (BnAHASL2A)
<400> 11
   Met Ala Ser Phe Ser Phe Phe Gly Thr Ile Pro Ser Ser Pro Thr Lys
   1               5                   10                  15
   Ala Ser Val Phe Ser Leu Pro Val Ser Val Thr Thr Leu Pro Ser Phe
               20                  25                  30
   Pro Arg Arg Arg Ala Thr Arg Val Ser Val Ser Ala Asn Ser Lys Lys
           35                  40                  45
   Asp Gln Asp Arg Thr Ala Ser Arg Arg Glu Asn Pro Ser Thr Phe Ser
       50                  55                  60
   Ser Lys Tyr Ala Pro Asn Val Pro Arg Ser Gly Ala Asp Ile Leu Val
   65                  70                  75                  80
   Glu Ala Leu Glu Arg Gln Gly Val Asp Val Val Phe Ala Tyr Pro Gly
                   85                  90                  95
   Gly Ala Ser Met Glu Ile His Gln Ala Leu Thr Arg Ser Asn Thr Ile
               100                 105                 110
   Arg Asn Val Leu Pro Arg His Glu Gln Gly Gly Ile Phe Ala Ala Glu
           115                 120                 125
   Gly Tyr Ala Arg Ser Ser Gly Lys Pro Gly Ile Cys Ile Ala Thr Ser
       130                 135                 140
   Gly Pro Gly Ala Met Asn Leu Val Ser Gly Leu Ala Asp Ala Leu Phe
   145                 150                 155                 160
   Asp Ser Val Pro Leu Ile Ala Ile Thr Gly Gln Val Pro Arg Arg Met
                   165                 170                 175
   Ile Gly Thr Met Ala Phe Gln Glu Thr Pro Val Val Glu Val Thr Arg
               180                 185                 190
   Thr Ile Thr Lys His Asn Tyr Leu Val Met Glu Val Asp Asp Ile Pro
           195                 200                 205
   Arg Ile Val Arg Glu Ala Phe Phe Leu Ala Thr Ser Val Arg Pro Gly
       210                 215                 220
   Pro Val Leu Ile Asp Val Pro Lys Asp Val Gln Gln Gln Phe Ala Ile
   225                 230                 235                 240
   Pro Asn Trp Glu Gln Pro Met Arg Leu Pro Leu Tyr Met Ser Thr Met
                   245                 250                 255
   Pro Lys Pro Pro Lys Val Ser His Leu Glu Gln Ile Leu Arg Leu Val
               260                 265                 270
   Ser Glu Ser Lys Arg Pro Val Leu Tyr Val Gly Gly Gly Cys Leu Asn
   275 280 285
   Ser Ser Glu Glu Leu Arg Arg Phe Val Glu Leu Thr Gly Ile Pro Val
   290 295 300
   Ala Ser Thr Phe Met Gly Leu Gly Ser Tyr Pro Cys Asp Asp Glu Glu
   305 310 315 320
   Phe Ser Leu Gln Met Leu Gly Met His Gly Thr Val Tyr Ala Asn Tyr
   325 330 335
   Ala Val Glu Tyr Ser Asp Leu Leu Leu Ala Phe Gly Val Arg Phe Asp
   340 345 350
   Asp Arg Val Thr Gly Lys Leu Glu Ala Phe Ala Ser Arg Ala Lys Ile
   355 360 365
   Val His Ile Asp Ile Asp Ser Thr Glu Ile Gly Lys Asn Lys Thr Pro
   370 375 380
   His Val Ser Val Cys Cys Asp Val Gln Leu Ala Leu Gln Gly Met Asn
   385 390 395 400
   Glu Val Leu Glu Asn Arg Arg Asp Val Leu Asp Phe Gly Glu Trp Arg
   405 410 415
   Cys Glu Leu Asn Glu Gln Arg Leu Lys Phe Pro Leu Arg Tyr Lys Thr
   420 425 430
   Phe Gly Glu Glu Ile Pro Pro Gln Tyr Ala Ile Gln Leu Leu Asp Glu
   435 440 445
   Leu Thr Asp Gly Lys Ala Ile Ile Thr Thr Gly Val Gly Gln His Gln
   450 455 460
   Met Trp Ala Ala Gln Phe Tyr Arg Phe Lys Lys Pro Arg Gln Trp Leu
   465 470 475 480
   Ser Ser Gly Gly Leu Gly Ala Met Gly Phe Gly Leu Pro Ala Ala Met
   485 490 495
   Gly Ala Ala Ile Ala Asn Pro Gly Ala Val Val Val Asp Ile Asp Gly
   500 505 510
   Asp Gly Ser Phe Ile Met Asn Ile Gln Glu Leu Ala Thr Ile Arg Val
   515 520 525
   Glu Asn Leu Pro Val Lys Val Leu Leu Ile Asn Asn Gln His Leu Gly
   530 535 540
   Met Val Leu Gln Trp Glu Asp His Phe Tyr Ala Ala Asn Arg Ala Asp
   545 550 555 560
   Ser Phe Leu Gly Asp Pro Ala Asn Pro Glu Ala Val Phe Pro Asp Met
   565 570 575
   Leu Leu Phe Ala Ala Ser Cys Gly Ile Pro Ala Ala Arg Val Thr Arg
   580 585 590
   Arg Glu Asp Leu Arg Glu Ala Ile Gln Thr Met Leu Asp Thr Pro Gly
   595 600 605
   Pro Phe Leu Leu Asp Val Val Cys Pro His Gln Asp His Val Leu Pro
   610 615 620
   Leu Ile Pro Ser Gly Gly Thr Phe Lys Asp Ile Ile Val
   625 630 635
<210> 12
   <211> 668
   <212> PRT
   <213> Camelina microcarpa
<220>
   <221> MSC_FEATURE
   <223> Carmelina microcarpa acetohydroxy acid synthase large subunit number 1 (CmAHASL1)
<400> 12
   Met Ala Ala Ala Thr Thr Thr Ser Ser Ser Ser Ile Pro Phe Ser Thr
   1 5 10 15
   Lys His Ser Ser Ser Lys Ser Pro Leu Pro Ile Ser Arg Phe Thr Leu
   20 25 30
   Pro Phe Ser Leu Asn Pro Asn Lys Ser Ser Ser Ser Ser Ser Arg Arg
   35 40 45
   Arg Gly Ile Lys Ser Thr Ala Leu Ser Ile Ser Ala Val Leu Asn Thr
   50 55 60
   Thr Thr Asn Val Ser Thr Thr Thr Pro Gln Ser Lys Pro Thr Lys Pro
   65 70 75 80
   Glu Lys Lys Lys Phe Val Ser Arg Phe Ala Pro Asp Gln Pro Arg Lys
   85 90 95
   Gly Ala Asp Ile Leu Val Glu Ala Leu Glu Arg Gln Gly Val Glu Thr
   100 105 110
   Ala Phe Ala Tyr Pro Gly Gly Ala Ser Met Glu Ile His Gln Ala Leu
   115 120 125
   Thr Arg Ser Ser Ser Ile Arg Asn Val Leu Pro Arg His Glu Gln Gly
   130 135 140
   Gly Val Phe Ala Ala Glu Gly Tyr Ala Arg Ser Thr Gly Lys Pro Gly
   145 150 155 160
   Ile Cys Ile Ala Thr Ser Gly Pro Gly Ala Thr Asn Leu Val Ser Gly
   165 170 175
   Leu Ala Asp Ala Leu Leu Asp Ser Val Pro Leu Val Ala Ile Thr Gly
   180 185 190
   Gln Val Pro Arg Arg Met Ile Gly Thr Asp Ala Phe Gln Glu Thr Pro
   195 200 205
   Ile Val Glu Val Thr Arg Ser Ile Thr Lys His Asn Tyr Leu Val Met
   210 215 220
   Asp Val Glu Asp Ile Pro Arg Ile Val Glu Glu Ala Phe Phe Leu Ala
   225 230 235 240
   Thr Ser Gly Arg Pro Gly Pro Val Leu Val Asp Val Pro Lys Asp Ile
   245 250 255
   Gln Gln Gln Leu Ala Ile Pro Asn Trp Glu Gln Ser Met Arg Leu Pro
   260 265 270
   Gly Tyr Met Ser Arg Met Pro Lys Pro Pro Glu Asp Ser His Leu Glu
   275 280 285
   Gln Ile Val Arg Leu Ile Ser Glu Ser Lys Lys Pro Val Leu Tyr Val
   290 295 300
   Gly Gly Gly Cys Leu Asn Ser Ser Glu Glu Leu Gly Arg Phe Val Glu
   305 310 315 320
   Leu Thr Gly Ile Pro Val Ala Ser Thr Leu Met Gly Leu Gly Ala Tyr
   325 330 335
   Pro Cys Asp Asp Glu Leu Ser Leu His Met Leu Gly Met His Gly Thr
   340 345 350
   Val Tyr Ala Asn Tyr Ser Val Glu His Ser Asp Leu Leu Leu Ala Phe
   355 360 365
   Gly Val Arg Phe Asp Asp Arg Val Thr Gly Lys Leu Glu Ala Phe Ala
   370 375 380
   Ser Arg Ala Lys Ile Val His Ile Asp Ile Asp Ser Ala Glu Ile Gly
   385 390 395 400
   Lys Asn Lys Thr Pro His Val Ser Val Cys Gly Asp Val Lys Leu Ala
   405 410 415
   Leu Gln Gly Met Asn Lys Val Leu Glu Asn Arg Ala Glu Glu Leu Lys
   420 425 430
   Leu Asp Phe Gly Val Trp Arg Ser Glu Leu Asn Glu Gln Lys Gln Lys
   435 440 445
   Phe Pro Leu Ser Phe Lys Thr Phe Gly Glu Ala Ile Pro Pro Gln Tyr
   450 455 460
   Ala Ile Gln Val Leu Asp Glu Leu Thr Asp Gly Arg Ala Ile Ile Ser
   465 470 475 480
   Thr Gly Val Gly Gln His Gln Met Trp Ala Ala Gln Phe Tyr Lys Tyr
   485 490 495
   Lys Lys Pro Arg Gln Trp Leu Ser Ser Ala Gly Leu Gly Ala Met Gly
   500 505 510
   Phe Gly Leu Pro Ala Ala Ile Gly Ala Ser Val Ala Asn Pro Asp Ser
   515 520 525
   Ile Val Val Asp Ile Asp Gly Asp Gly Ser Phe Ile Met Asn Val Gln
   530 535 540
   Glu Leu Ala Thr Ile Arg Val Glu Asn Leu Pro Val Lys Ile Leu Ile
   545 550 555 560
   Leu Asn Asn Gln His Leu Gly Met Val Met Gln Trp Glu Asp Arg Phe
   565 570 575
   Tyr Lys Ala Asn Arg Ala His Thr Tyr Leu Gly Asn Pro Ala Glu Glu
   580 585 590
   Asp Glu Ile Phe Pro Asn Met Leu Gln Phe Ala Ser Ala Cys Gly Ile
   595 600 605
   Pro Ala Ala Arg Val Thr Lys Ile Ala Glu Leu Arg Glu Ala Ile Gln
   610 615 620
   Lys Met Leu Asp Thr Pro Gly Pro Tyr Leu Leu Asp Val Ile Cys Pro
   625 630 635 640
   His Gln Glu His Val Leu Pro Met Ile Pro Ser Gly Gly Thr Phe Asn
   645 650 655
   Asp Val Ile Thr Glu Gly Asp Gly Arg Thr Lys Tyr
   660 665
<210> 13
   <211> 665
   <212> PRT
   <213> Carmelina microcarpa
<220>
   <221> MSC_FEATURE
   <223> Carmelina microcarpa acetohydroxy acid synthase large subunit number 2 (CmAHASL2)
<400> 13
   Met Ala Ala Ala Thr Thr Thr Ser Ser Ser Ser Ile Pro Phe Ser Thr
   1 5 10 15
   Lys Pro Ser Ser Ser Lys Ser Pro Leu Pro Ile Ser Arg Phe Thr Leu
   20 25 30
   Pro Phe Ser Leu Asn Pro Asn Lys Ser Ser Ser Arg Arg Arg Gly Ile
   35 40 45
   Lys Ser Thr Ser Leu Ser Ile Ser Ala Val Leu Asn Thr Thr Thr Asn
   50 55 60
   Val Ser Thr Thr Thr Pro Pro Ser Lys Pro Thr Lys Pro Glu Lys Lys
   65 70 75 80
   Lys Phe Val Ser Arg Phe Ala Pro Asp Gln Pro Arg Lys Gly Ala Asp
   85 90 95
   Ile Leu Val Glu Ala Leu Glu Arg Gln Gly Val Glu Thr Val Phe Ala
   100 105 110
   Tyr Pro Gly Gly Ala Ser Met Glu Ile His Gln Ala Leu Thr Arg Ser
   115 120 125
   Ser Ser Ile Arg Asn Val Leu Pro Arg His Glu Gln Gly Gly Val Phe
   130 135 140
   Ala Ala Glu Gly Tyr Ala Arg Ser Thr Gly Lys Pro Gly Ile Cys Ile
   145 150 155 160
   Ala Thr Ser Gly Pro Gly Ala Thr Asn Leu Val Ser Gly Leu Ala Asp
   165 170 175
   Ala Leu Leu Asp Ser Val Pro Leu Val Ala Ile Thr Gly Gln Val Pro
   180 185 190
   Arg Arg Met Ile Gly Thr Asp Ala Phe Gln Glu Thr Pro Ile Val Glu
   195 200 205
   Val Thr Arg Ser Ile Thr Lys His Asn Tyr Leu Val Met Asp Val Glu
   210 215 220
   Asp Ile Pro Arg Ile Val Glu Glu Ala Phe Phe Leu Ala Thr Ser Gly
   225 230 235 240
   Arg Pro Gly Pro Val Leu Val Asp Val Pro Lys Asp Ile Gln Gln Gln
   245 250 255
   Leu Ala Ile Pro Asn Trp Glu Gln Ser Met Arg Leu Pro Gly Tyr Met
   260 265 270
   Ser Arg Met Pro Lys Pro Pro Glu Asp Ser His Leu Glu Gln Ile Val
   275 280 285
   Arg Leu Ile Ser Glu Ser Lys Lys Pro Val Leu Tyr Val Gly Gly Gly
   290 295 300
   Cys Leu Asn Ser Ser Glu Glu Leu Gly Arg Phe Val Glu Leu Thr Gly
   305 310 315 320
   Ile Pro Val Ala Ser Thr Leu Met Gly Leu Gly Ala Tyr Pro Cys Asp
   325 330 335
   Asp Glu Leu Ser Leu His Met Leu Gly Met His Gly Thr Val Tyr Ala
   340 345 350
   Asn Tyr Ser Val Glu His Ser Asp Leu Leu Leu Ala Phe Gly Val Arg
   355 360 365
   Phe Asp Asp Arg Val Thr Gly Lys Leu Glu Ala Phe Ala Ser Arg Ala
   370 375 380
   Lys Ile Val His Ile Asp Ile Asp Ser Ala Glu Ile Gly Lys Asn Lys
   385 390 395 400
   Thr Pro His Val Ser Val Cys Gly Asp Val Lys Leu Ala Leu Gln Gly
   405 410 415
   Met Asn Lys Val Leu Glu Asn Arg Gly Glu Glu Leu Lys Leu Asp Phe
   420 425 430
   Gly Val Trp Arg Ser Glu Leu Asn Glu Gln Lys Gln Lys Phe Pro Leu
   435 440 445
   Ser Phe Lys Thr Phe Gly Glu Ala Ile Pro Pro Gln Tyr Ala Ile Gln
   450 455 460
   Val Leu Asp Glu Leu Thr Asp Gly Lys Ala Ile Ile Ser Thr Gly Val
   465 470 475 480
   Gly Gln His Gln Met Trp Ala Ala Gln Phe Tyr Lys Tyr Lys Lys Pro
   485 490 495
   Arg Gln Trp Leu Ser Ser Ala Gly Leu Gly Ala Met Gly Phe Gly Leu
   500 505 510
   Pro Ala Ala Ile Gly Ala Ser Val Ala Asn Pro Asp Ala Val Val Val
   515 520 525
   Asp Ile Asp Gly Asp Gly Ser Phe Ile Met Asn Val Gln Glu Leu Ala
   530 535 540
   Thr Ile Arg Val Glu Asn Leu Pro Val Lys Ile Leu Ile Leu Asn Asn
   545 550 555 560
   Gln His Leu Gly Met Val Met Gln Trp Glu Asp Arg Phe Tyr Lys Ala
   565 570 575
   Asn Arg Ala His Thr Tyr Leu Gly Asn Pro Ala Thr Glu Asp Glu Ile
   580 585 590
   Phe Pro Asn Met Leu Gln Phe Ala Ser Ala Cys Gly Ile Pro Ser Ala
   595 600 605
   Arg Val Thr Lys Lys Ala Glu Leu Arg Glu Ala Ile Gln Lys Met Leu
   610 615 620
   Asp Thr Pro Gly Pro Tyr Leu Leu Asp Val Ile Cys Pro His Gln Glu
   625 630 635 640
   His Val Leu Pro Met Ile Pro Ser Gly Gly Thr Phe Asn Asp Val Ile
   645 650 655
   Thr Glu Gly Asp Gly Arg Thr Lys Tyr
   660 665
<210> 14
   <211> 567
   <212> PRT
   <213> Solanumptychant hum
<220>
   <221> MSC_FEATURE
   <223> Partial Sol anum pt ychant humacetohydroxy acid synthase large subunit (SpAHASL)
<400> 14
   Pro Arg Lys Gly Cys Asp Val Leu Val Glu Ala Leu Glu Arg Glu Gly
   1 5 10 15
   Val Thr Asp Val Phe Ala Tyr Pro Gly Gly Thr Ser Met Glu Ile His
   20 25 30
   Gln Ala Leu Thr Arg Ser Asn Ile Ile Arg Asn Val Leu Pro Arg His
   35 40 45
   Glu Gln Gly Gly Val Phe Ala Ala Glu Gly Tyr Ala Arg Ala Thr Gly
   50 55 60
   Phe Pro Gly Val Cys Ile Ala Thr Ser Gly Pro Gly Ala Thr Asn Leu
   65 70 75 80
   Val Ser Gly Leu Ala Asp Ala Leu Leu Asp Ser Ile Pro Ile Val Ala
   85 90 95
   Ile Thr Gly Gln Val Pro Arg Arg Met Ile Gly Thr Asp Ala Phe Gln
   100 105 110
   Glu Thr Pro Ile Val Glu Val Thr Arg Ser Ile Thr Lys His Asn Tyr
   115 120 125
   Leu Val Met Asp Val Glu Asp Ile Pro Arg Ile Val Arg Glu Ala Phe
   130 135 140
   Phe Leu Ala Lys Ser Gly Arg Pro Gly Pro Val Leu Ile Asp Val Pro
   145 150 155 160
   Lys Asp Ile Gln Gln Gln Leu Val Ile Pro Asn Trp Asp Gln Pro Met
   165 170 175
   Arg Leu Pro Gly Tyr Met Ser Arg Leu Pro Lys Leu Pro Asn Glu Met
   180 185 190
   Leu Leu Glu Gln Ile Val Arg Leu Ile Ser Glu Ser Lys Lys Pro Val
   195 200 205
   Leu Tyr Val Gly Gly Gly Cys Ser Gln Ser Ser Glu Glu Leu Arg Arg
   210 215 220
   Phe Val Glu Leu Thr Gly Ile Pro Val Ala Ser Thr Leu Met Gly Leu
   225 230 235 240
   Gly Ala Phe Pro Thr Gly Asp Glu Leu Ser Leu Gln Met Leu Gly Met
   245 250 255
   His Gly Thr Val Tyr Ala Asn Tyr Ala Val Asp Ser Ser Asp Leu Leu
   260 265 270
   Leu Ala Phe Gly Val Arg Phe Asp Asp Arg Val Thr Gly Lys Leu Glu
   275 280 285
   Ala Phe Ala Ser Arg Ala Lys Ile Val His Ile Asp Ile Asp Ser Ala
   290 295 300
   Glu Ile Gly Lys Asn Lys Leu Pro His Ala Ser Ile Cys Ala Asp Ile
   305 310 315 320
   Lys Leu Ala Leu Gln Gly Leu Asn Ser Ile Leu Glu Gly Lys Glu Gly
   325 330 335
   Lys Leu Lys Leu Asp Phe Ser Ala Trp Arg Gln Glu Leu Asn Glu Gln
   340 345 350
   Lys Val Lys Tyr Pro Leu Asn Phe Lys Thr Phe Gly Glu Ala Ile Pro
   355 360 365
   Pro Gln Tyr Ala Ile Gln Val Leu Asp Glu Leu Thr Asn Gly Asn Ala
   370 375 380
   Ile Ile Ser Thr Gly Val Gly Gln His Gln Met Trp Ala Ala Gln Phe
   385 390 395 400
   Tyr Lys Tyr Lys Lys Pro Arg Gln Trp Leu Thr Ser Gly Gly Leu Gly
   405 410 415
   Ala Met Gly Phe Gly Leu Pro Ala Ala Met Gly Ala Ala Val Gly Arg
   420 425 430
   Pro Gly Glu Ile Val Val Asp Ile Asp Gly Asp Gly Ser Phe Met Met
   435 440 445
   Asn Val Gln Glu Leu Ala Thr Ile Lys Val Glu Asn Leu Pro Val Lys
   450 455 460
   Ile Met Leu Leu Asn Asn Gln His Leu Gly Met Val Val Gln Trp Glu
   465 470 475 480
   Asp Arg Phe Tyr Lys Ala Asn Arg Ala His Thr Tyr Leu Gly Asp Pro
   485 490 495
   Ala Asn Glu Glu Glu Ile Phe Pro Asn Met Leu Lys Phe Ala Glu Ala
   500 505 510
   Cys Gly Val Pro Ala Ala Arg Val Thr His Arg Asp Glu Leu Arg Ala
   515 520 525
   Ala Ile Gln Lys Met Leu Asp Thr Pro Gly Pro Tyr Leu Leu Asp Val
   530 535 540
   Ile Val Pro His Gln Glu His Val Leu Pro Met Ile Pro Ser Gly Gly
   545 550 555 560
   Ala Phe Lys Asp Val Ile Thr
   565
<210> 15
   <211> 641
   <212> PRT
   <213> Sorghum bicolor
<220>
   <221> MSC_FEATURE
   <223> Sorghum bicolor acetohydroxy acid synthase large subunit (SbAHASL)
<400> 15
   Met Ala Thr Thr Ala Ala Ala Ala Ala Ala Ala Leu Ala Gly Ala Thr
   1               5                   10                  15
   Thr Ala Ala Pro Lys Ala Arg Arg Arg Ala His Leu Leu Ala Ala Arg
               20                  25                  30
   Arg Ala Leu Ala Ala Pro Ile Arg Cys Ser Ala Ala Pro Pro Ala Thr
           35                  40                  45
   Leu Thr Val Thr Ala Pro Pro Ala Thr Pro Leu Arg Pro Trp Gly Pro
       50                  55                  60
   Thr Asp Pro Arg Lys Gly Ala Asp Ile Leu Val Glu Ala Leu Glu Arg
   65                  70                  75                  80
   Cys Gly Val Arg Asp Val Phe Ala Tyr Pro Gly Gly Ala Ser Met Glu
                   85                  90                  95
   Ile His Gln Ala Leu Thr Arg Ser Pro Val Ile Ala Asn His Leu Phe
               100                 105                 110
   Arg His Glu Gln Gly Glu Ala Phe Ala Ala Ser Gly Phe Ala Arg Ser
           115                 120                 125
   Ser Gly Arg Val Gly Val Cys Val Ala Thr Ser Gly Pro Gly Ala Thr
       130                 135                 140
   Asn Leu Val Ser Ala Leu Ala Asp Ala Leu Leu Asp Ser Val Pro Met
   145                 150                 155                 160
   Val Ala Ile Thr Gly Gln Val Pro Arg Arg Met Ile Gly Thr Asp Ala
                   165                 170                 175
   Phe Gln Glu Thr Pro Ile Val Glu Val Thr Arg Ser Ile Thr Lys His
               180                 185                 190
   Asn Tyr Leu Val Leu Asp Val Asp Asp Ile Pro Arg Val Val Gln Glu
           195                 200                 205
   Ala Phe Phe Leu Ala Ser Ser Gly Arg Pro Gly Pro Val Leu Val Asp
       210                 215                 220
   Ile Pro Lys Asp Ile Gln Gln Gln Met Ala Val Pro Val Trp Asp Thr
   225                 230                 235                 240
   Pro Met Ser Leu Pro Gly Tyr Ile Ala Arg Leu Pro Lys Pro Pro Ala
                   245                 250                 255
   Thr Glu Leu Leu Glu Gln Val Leu Arg Leu Val Gly Glu Ser Arg Arg
   260 265 270
   Pro Val Leu Tyr Val Gly Gly Gly Cys Ala Ala Ser Gly Glu Glu Leu
   275 280 285
   Arg Arg Phe Val Glu Met Thr Gly Ile Pro Val Thr Thr Thr Leu Met
   290 295 300
   Gly Leu Gly Asn Phe Pro Gly Asp Asp Pro Leu Ser Leu Arg Met Leu
   305 310 315 320
   Gly Met His Gly Thr Val Tyr Ala Asn Tyr Ala Val Asp Lys Ala Asp
   325 330 335
   Leu Leu Leu Ala Phe Gly Val Arg Phe Asp Asp Arg Val Thr Gly Lys
   340 345 350
   Ile Glu Ala Phe Ala Ser Arg Ala Lys Ile Val His Ile Asp Ile Asp
   355 360 365
   Pro Ala Glu Ile Gly Lys Asn Lys Gln Pro His Val Ser Ile Cys Ala
   370 375 380
   Asp Val Lys Leu Ala Leu Gln Gly Met Asn Ala Leu Leu Glu Gly Ser
   385 390 395 400
   Thr Ser Lys Lys Ser Phe Asp Phe Gly Ser Trp Gln Ala Glu Leu Asp
   405 410 415
   Gln Gln Lys Arg Glu Phe Pro Leu Gly Tyr Lys Thr Phe Asp Asp Glu
   420 425 430
   Ile Gln Pro Gln Tyr Ala Ile Gln Val Leu Asp Glu Leu Thr Lys Gly
   435 440 445
   Glu Ala Ile Ile Ala Thr Gly Val Gly Gln His Gln Met Trp Ala Ala
   450 455 460
   Gln Tyr Tyr Thr Tyr Lys Arg Pro Arg Gln Trp Leu Ser Ser Ala Gly
   465 470 475 480
   Leu Gly Ala Met Gly Phe Gly Leu Pro Ala Ala Ala Gly Ala Ala Val
   485 490 495
   Ala Asn Pro Gly Ile Thr Val Val Asp Ile Asp Gly Asp Gly Ser Phe
   500 505 510
   Leu Met Asn Ile Gln Glu Leu Ala Met Ile Arg Ile Glu Asn Leu Pro
   515 520 525
   Val Lys Val Phe Val Leu Asn Asn Gln His Leu Gly Met Val Val Gln
   530 535 540
   Trp Glu Asp Arg Phe Tyr Lys Ala Asn Arg Ala His Thr Tyr Leu Gly
   545 550 555 560
   Asn Pro Glu Asn Glu Ser Glu Ile Tyr Pro Asp Phe Val Thr Ile Ala
   565 570 575
   Lys Gly Phe Asn Ile Pro Ala Val Arg Val Thr Lys Lys Ser Glu Val
   580 585 590
   His Ala Ala Ile Lys Lys Met Leu Glu Thr Pro Gly Pro Tyr Leu Leu
   595 600 605
   Asp Ile Ile Val Pro His Gln Glu His Val Leu Pro Met Ile Pro Ser
   610 615 620
   Gly Gly Ala Phe Lys Asp Met Ile Leu Asp Gly Asp Gly Arg Thr Val
   625 630 635 640
   Tyr
<210> 16
   <211> 657
   <212> PRT
   <213> Solanum tuber osum
<220>
   <221> MSC_FEATURE
   <223> Potatoacetohydroxy acid synthase large subunit number 1 (StAHASL1)
<400> 16
   Met Ala Ala Ala Ser Pro Ser Pro Cys Phe Ser Lys Asn Leu Pro Pro
   1 5 10 15
   Ser Ser Ser Lys Ser Ser Ile Leu Leu Pro Lys Ser Thr Phe Thr Phe
   20 25 30
   His Asn His Pro Lys Lys Thr Ser Pro Leu His Leu Thr His Thr Gln
   35 40 45
   His His Ser Arg Phe Thr Val Ser Asn Val Ile Leu Ser Thr Thr Thr
   50 55 60
   His Asn Asp Val Ser Glu Pro Glu Ile Phe Val Ser Arg Phe Ala Pro
   65 70 75 80
   Asp Glu Pro Arg Lys Gly Cys Asp Val Leu Val Glu Ala Leu Glu Arg
   85 90 95
   Glu Gly Val Lys Asp Val Phe Ala Tyr Pro Gly Gly Ala Ser Met Glu
   100 105 110
   Ile His Gln Ala Leu Thr Arg Ser Asn Ile Ile Arg Asn Val Leu Pro
   115 120 125
   Arg His Glu Gln Gly Gly Val Phe Ala Ala Glu Gly Tyr Ala Arg Ala
   130 135 140
   Thr Gly Phe Pro Gly Val Cys Ile Ala Thr Ser Gly Pro Gly Ala Thr
   145 150 155 160
   Asn Leu Val Ser Gly Leu Ala Asp Ala Leu Leu Asp Ser Ile Pro Ile
   165 170 175
   Val Ala Ile Thr Gly Gln Val Pro Arg Arg Met Ile Gly Thr Asp Ala
   180 185 190
   Phe Gln Glu Thr Pro Ile Val Glu Val Thr Arg Ser Ile Thr Lys His
   195 200 205
   Asn Tyr Leu Val Met Asp Val Glu Asp Ile Pro Arg Val Val Arg Glu
   210 215 220
   Ala Phe Phe Leu Ala Lys Ser Gly Arg Pro Gly Pro Val Leu Ile Asp
   225 230 235 240
   Val Pro Lys Asp Ile Gln Gln Pro Leu Val Ile Pro Asn Trp Asp Gln
   245 250 255
   Pro Met Arg Leu Pro Gly Tyr Met Ser Arg Leu Pro Lys Leu Pro Asn
   260 265 270
   Glu Met Leu Leu Glu Gln Ile Val Arg Leu Ile Ser Glu Ser Lys Lys
   275 280 285
   Pro Val Leu Tyr Val Gly Gly Gly Cys Thr Gln Ser Ser Glu Glu Leu
   290 295 300
   Arg Arg Phe Val Glu Leu Thr Gly Ile Pro Val Ala Ser Thr Leu Met
   305 310 315 320
   Gly Leu Gly Ala Phe Pro Val Gly Asp Glu Leu Ser Leu Gln Met Leu
   325 330 335
   Gly Met His Gly Thr Val Tyr Ala Asn Tyr Ala Val Asp Ser Ser Asp
   340 345 350
   Leu Leu Leu Ala Phe Gly Val Arg Phe Asp Asp Arg Val Thr Gly Lys
   355 360 365
   Leu Glu Ala Phe Ala Ser Arg Ala Lys Ile Val His Ile Asp Ile Asp
   370 375 380
   Ser Ala Glu Ile Gly Lys Asn Lys Gln Pro His Val Ser Ile Cys Ala
   385                 390                 395                 400
   Asp Ile Lys Leu Ala Leu Gln Gly Leu Asn Ser Ile Leu Glu Gly Lys
                   405                 410                 415
   Glu Gly Lys Leu Lys Leu Asp Phe Ser Ala Trp Arg Gln Glu Leu Thr
               420                 425                 430
   Glu Gln Lys Val Lys Tyr Pro Leu Asn Tyr Lys Thr Phe Gly Glu Ala
           435                 440                 445
   Ile Pro Pro Gln Tyr Ala Ile Gln Val Leu Asp Glu Leu Thr Asn Gly
       450                 455                 460
   Asn Ala Ile Ile Ser Thr Gly Val Gly Gln His Gln Met Trp Ala Ala
   465                 470                 475                 480
   Gln Tyr Tyr Lys Tyr Lys Lys Pro Arg Gln Trp Leu Thr Ser Gly Gly
                   485                 490                 495
   Leu Gly Ala Met Gly Phe Gly Leu Pro Ala Ala Ile Gly Ala Ala Val
               500                 505                 510
   Gly Arg Pro Gly Glu Ile Val Val Asp Ile Asp Gly Asp Gly Ser Phe
           515                 520                 525
   Ile Met Asn Val Gln Glu Leu Ala Thr Ile Lys Val Glu Asn Leu Pro
       530                 535                 540
   Val Lys Ile Met Leu Leu Asn Asn Gln His Leu Gly Met Val Val Gln
   545                 550                 555                 560
   Trp Glu Asp Arg Phe Tyr Lys Ala Asn Arg Ala His Thr Tyr Leu Gly
                   565                 570                 575
   Asp Pro Ala Asn Glu Glu Glu Ile Phe Pro Asn Met Leu Lys Phe Ala
               580                 585                 590
   Glu Ala Cys Gly Val Pro Ala Ala Arg Val Ser His Arg Asp Asp Leu
           595                 600                 605
   Arg Ala Ala Ile Gln Lys Met Leu Asp Thr Pro Gly Pro Tyr Leu Leu
       610                 615                 620
   Asp Val Ile Val Pro His Gln Glu His Val Leu Pro Met Ile Pro Ser
   625                 630                 635                 640
   Gly Gly Ala Phe Lys Asp Val Ile Thr Glu Gly Asp Gly Arg Arg Ser
                   645                 650                 655
   Tyr
<210> 17
   <211> 659
   <212> PRT
   <213> Solanum tuber osum
<220>
   <221> MSC_FEATURE
   <223> Potato acetohydroxy acid synthase large subunit number 2 (St AHASL2)
<220>
   <221> misc_feature
   <222> (406)..(406)
   <223> Xaa can be any naturally occurring amino acid
<400> 17
   Met Ala Ala Ala Ala Ser Pro Ser Pro Cys Phe Ser Lys Thr Leu Pro
   1               5                   10                  15
   Pro Ser Ser Ser Lys Ser Ser Thr Ile Leu Pro Arg Ser Thr Phe Pro
               20                  25                  30
   Phe His Asn His Pro Gln Lys Ala Ser Pro Leu His Leu Thr His Thr
           35                  40                  45
   His His His Arg Arg Gly Phe Ala Val Ser Asn Val Val Ile Ser Thr
       50                  55                  60
   Thr Thr His Asn Asp Val Ser Glu Pro Glu Thr Phe Val Ser Arg Phe
   65                  70                  75                  80
   Ala Pro Asp Glu Pro Arg Lys Gly Cys Asp Val Leu Val Glu Ala Leu
                   85                  90                  95
   Glu Arg Glu Gly Val Thr Asp Val Phe Ala Tyr Pro Gly Gly Ala Ser
               100                 105                 110
   Met Glu Ile His Gln Ala Leu Thr Arg Ser Asn Ile Ile Arg Asn Val
           115                 120                 125
   Leu Pro Arg His Glu Gln Gly Gly Val Phe Ala Ala Glu Gly Tyr Ala
       130                 135                 140
   Arg Ala Thr Gly Phe Pro Gly Val Cys Ile Ala Thr Ser Gly Pro Gly
   145                 150                 155                 160
   Ala Thr Asn Leu Val Ser Gly Leu Ala Asp Ala Leu Leu Asp Ser Ile
                   165                 170                 175
   Pro Ile Val Ala Ile Thr Gly Gln Val Pro Arg Arg Met Ile Gly Thr
               180                 185                 190
   Asp Ala Phe Gln Glu Thr Pro Ile Val Glu Val Thr Arg Ser Ile Thr
           195                 200                 205
   Lys His Asn Tyr Leu Val Met Asp Val Glu Asp Ile Pro Arg Val Val
       210                 215                 220
   Arg Glu Ala Phe Phe Leu Ala Lys Ser Gly Arg Pro Gly Pro Val Leu
   225                 230                 235                 240
   Ile Asp Val Pro Lys Asp Ile Gln Gln Gln Leu Val Ile Pro Asn Trp
                   245                 250                 255
   Asp Gln Pro Met Arg Leu Pro Gly Tyr Met Ser Arg Leu Pro Lys Leu
               260                 265                 270
   Pro Asn Glu Met Leu Leu Glu Gln Ile Ile Arg Leu Ile Ser Glu Ser
           275                 280                 285
   Lys Lys Pro Val Leu Tyr Val Gly Gly Gly Cys Leu Gln Ser Ser Glu
       290                 295                 300
   Glu Leu Arg Arg Phe Val Glu Leu Thr Gly Ile Ser Val Ala Ser Thr
   305                 310                 315                 320
   Leu Met Gly Leu Gly Ala Phe Pro Thr Gly Asp Glu Leu Ser Leu Gln
                   325                 330                 335
   Met Leu Gly Met His Gly Thr Val Tyr Ala Asn Tyr Ala Val Asp Gly
               340                 345                 350
   Ser Asp Leu Leu Leu Ala Phe Gly Val Arg Phe Asp Asp Arg Val Thr
           355                 360                 365
   Gly Lys Leu Glu Ala Phe Ala Ser Arg Ala Lys Ile Val His Ile Asp
       370                 375                 380
   Ile Asp Ser Ala Glu Ile Gly Lys Asn Lys Gln Pro His Val Ser Ile
   385                 390                 395                 400
   Cys Ala Asp Ile Lys Xaa Ala Leu Gln Gly Leu Asn Ser Ile Leu Glu
                   405                 410                 415
   Gly Lys Glu Gly Lys Leu Lys Leu Asp Phe Ser Ala Trp Arg Gln Glu
               420                 425                 430
   Leu Thr Glu Gln Lys Val Lys Tyr Pro Leu Ser Phe Lys Thr Phe Gly
           435                 440                 445
   Glu Ala Ile Pro Pro Gln Tyr Ala Ile Gln Val Leu Asp Glu Leu Thr
       450                 455                 460
   Asn Gly Asn Ala Ile Ile Ser Thr Gly Val Gly Gln His Gln Met Trp
   465 470 475 480
   Ala Ala Gln Tyr Tyr Lys Tyr Lys Lys Pro Arg Gln Trp Leu Thr Ser
   485 490 495
   Gly Gly Leu Gly Ala Met Gly Phe Gly Leu Pro Ala Ala Ile Gly Ala
   500 505 510
   Ala Val Gly Arg Pro Gly Glu Ile Val Val Asp Ile Asp Gly Asp Gly
   515 520 525
   Ser Phe Ile Met Asn Val Gln Glu Leu Ala Thr Ile Lys Val Glu Asn
   530 535 540
   Leu Pro Val Lys Ile Met Leu Leu Asn Asn Gln His Leu Gly Met Val
   545 550 555 560
   Val Gln Trp Glu Asp Arg Phe Tyr Lys Ala Asn Arg Ala His Thr Tyr
   565 570 575
   Leu Gly Asp Pro Ala Asn Glu Glu Glu Ile Phe Pro Asn Met Leu Lys
   580 585 590
   Phe Ala Glu Ala Cys Gly Val Pro Ala Ala Arg Val Ser His Arg Asp
   595 600 605
   Asp Leu Arg Ala Ala Ile Gln Lys Met Leu Asp Thr Pro Gly Pro Tyr
   610 615 620
   Leu Leu Asp Val Ile Val Pro His Gln Glu His Val Leu Pro Met Ile
   625 630 635 640
   Pro Ser Gly Gly Ala Phe Lys Asp Val Ile Thr Glu Gly Asp Gly Arg
   645 650 655
   Arg Ser Tyr
<210> 18
   <211> 651
   <212> PRT
   <213> Glycine max
<220>
   <221> MSC_FEATURE
   <223> Soybean acetohydroxy acid synthase large subunit (GmAHASL)
<400> 18
   Met Ala Ala Thr Ala Ser Arg Thr Thr Arg Phe Ser Ser Ser Ser Ser
   1 5 10 15
   His Pro Thr Phe Pro Lys Arg Ile Thr Arg Ser Thr Leu Pro Leu Ser
   20 25 30
   His Gln Thr Leu Thr Lys Pro Asn His Ala Leu Lys Ile Lys Cys Ser
   35 40 45
   Ile Ser Lys Pro Pro Thr Ala Ala Pro Phe Thr Lys Glu Ala Pro Thr
   50 55 60
   Thr Glu Pro Phe Val Ser Arg Phe Ala Ser Gly Glu Pro Arg Lys Gly
   65 70 75 80
   Ala Asp Ile Leu Val Glu Ala Leu Glu Arg Gln Gly Val Thr Thr Val
   85 90 95
   Phe Ala Tyr Pro Gly Gly Ala Ser Met Glu Ile His Gln Ala Leu Thr
   100 105 110
   Arg Ser Ala Ala Ile Arg Asn Val Leu Pro Arg His Glu Gln Gly Gly
   115 120 125
   Val Phe Ala Ala Glu Gly Tyr Ala Arg Ser Ser Gly Leu Pro Gly Val
   130 135 140
   Cys Ile Ala Thr Ser Gly Pro Gly Ala Thr Asn Leu Val Ser Gly Leu
   145 150 155 160
   Ala Asp Ala Leu Met Asp Ser Val Pro Val Val Ala Ile Thr Gly Gln
   165 170 175
   Val Pro Arg Arg Met Ile Gly Thr Asp Ala Phe Gln Glu Thr Pro Ile
   180 185 190
   Val Glu Val Ser Arg Ser Ile Thr Lys His Asn Tyr Leu Ile Leu Asp
   195 200 205
   Val Asp Asp Ile Pro Arg Val Val Ala Glu Ala Phe Phe Val Ala Thr
   210 215 220
   Ser Gly Arg Pro Gly Pro Val Leu Ile Asp Ile Pro Lys Asp Val Gln
   225 230 235 240
   Gln Gln Leu Ala Val Pro Asn Trp Asp Glu Pro Val Asn Leu Pro Gly
   245 250 255
   Tyr Leu Ala Arg Leu Pro Arg Pro Pro Ala Glu Ala Gln Leu Glu His
   260 265 270
   Ile Val Arg Leu Ile Met Glu Ala Gln Lys Pro Val Leu Tyr Val Gly
   275 280 285
   Gly Gly Ser Leu Asn Ser Ser Ala Glu Leu Arg Arg Phe Val Glu Leu
   290 295 300
   Thr Gly Ile Pro Val Ala Ser Thr Leu Met Gly Leu Gly Thr Phe Pro
   305 310 315 320
   Ile Gly Asp Glu Tyr Ser Leu Gln Met Leu Gly Met His Gly Thr Val
   325 330 335
   Tyr Ala Asn Tyr Ala Val Asp Asn Ser Asp Leu Leu Leu Ala Phe Gly
   340 345 350
   Val Arg Phe Asp Asp Arg Val Thr Gly Lys Leu Glu Ala Phe Ala Ser
   355 360 365
   Arg Ala Lys Ile Val His Ile Asp Ile Asp Ser Ala Glu Ile Gly Lys
   370 375 380
   Asn Lys Gln Ala His Val Ser Val Cys Ala Asp Leu Lys Leu Ala Leu
   385 390 395 400
   Lys Gly Ile Asn Met Ile Leu Glu Glu Lys Gly Val Glu Gly Lys Phe
   405 410 415
   Asp Leu Gly Gly Trp Arg Glu Glu Ile Asn Val Gln Lys His Lys Phe
   420 425 430
   Pro Leu Gly Tyr Lys Thr Phe Gln Asp Ala Ile Ser Pro Gln His Ala
   435 440 445
   Ile Glu Val Leu Asp Glu Leu Thr Asn Gly Asp Ala Ile Val Ser Thr
   450 455 460
   Gly Val Gly Gln His Gln Met Trp Ala Ala Gln Phe Tyr Lys Tyr Lys
   465 470 475 480
   Arg Pro Arg Gln Trp Leu Thr Ser Gly Gly Leu Gly Ala Met Gly Phe
   485 490 495
   Gly Leu Pro Ala Ala Ile Gly Ala Ala Val Ala Asn Pro Gly Ala Val
   500 505 510
   Val Val Asp Ile Asp Gly Asp Gly Ser Phe Ile Met Asn Val Gln Glu
   515 520 525
   Leu Ala Thr Ile Arg Val Glu Asn Leu Pro Val Lys Ile Leu Leu Leu
   530 535 540
   Asn Asn Gln His Leu Gly Met Val Val Gln Trp Glu Asp Arg Phe Tyr
   545 550 555 560
   Lys Ser Asn Arg Ala His Thr Tyr Leu Gly Asp Pro Ser Ser Glu Ser
   565 570 575
   Glu Ile Phe Pro Asn Met Leu Lys Phe Ala Asp Ala Cys Gly Ile Pro
   580 585 590
   Ala Ala Arg Val Thr Lys Lys Glu Glu Leu Arg Ala Ala Ile Gln Arg
   595 600 605
   Met Leu Asp Thr Pro Gly Pro Tyr Leu Leu Asp Val Ile Val Pro His
   610 615 620
   Gln Glu His Val Leu Pro Met Ile Pro Ser Asn Gly Ser Phe Lys Asp
   625 630 635 640
   Val Ile Thr Glu Gly Asp Gly Arg Thr Arg Tyr
   645 650
<210> 19
   <211> 648
   <212> PRT
   <213> Xanthium sp.
<220>
   <221> MSC_FEATURE
   <223> Cocklebur acetohydroxyacid synthase large subunit (XsAHASL)
<400> 19
   Met Ala Ala Ile Pro His Thr Asn Pro Ser Ile Thr Thr Lys Pro Pro
   1 5 10 15
   Ser Ser Pro Pro Arg Pro Thr Phe Leu Ala Arg Phe Thr Phe Pro Ile
   20 25 30
   Thr Ser Thr Ser His Lys Arg His Arg Leu His Ile Ser Asn Val Leu
   35 40 45
   Ser Asp Ser Lys Pro Thr Ile Thr His Ser Pro Leu Pro Thr Glu Ser
   50 55 60
   Phe Ile Ser Arg Tyr Ala Pro Asp Gln Pro Arg Lys Gly Ala Asp Val
   65 70 75 80
   Leu Val Glu Ala Leu Glu Arg Glu Gly Val Thr Asp Val Phe Ala Tyr
   85 90 95
   Pro Gly Gly Ala Ser Met Glu Ile His Gln Ala Leu Thr Arg Ser Thr
   100 105 110
   Thr Ile Arg Asn Val Leu Pro Arg His Glu Gln Gly Gly Val Phe Ala
   115 120 125
   Ala Glu Gly Tyr Ala Arg Ala Ser Gly Leu Pro Gly Val Cys Ile Ala
   130 135 140
   Thr Ser Gly Pro Gly Ala Thr Asn Leu Val Ser Gly Leu Ala Asp Ala
   145 150 155 160
   Leu Leu Asp Ser Val Pro Met Val Ala Ile Thr Gly Gln Val Pro Arg
   165 170 175
   Arg Met Ile Gly Thr Asp Ala Phe Gln Glu Thr Pro Ile Val Glu Val
   180 185 190
   Thr Arg Ser Ile Thr Lys His Asn Tyr Leu Val Leu Asp Val Glu Asp
   195 200 205
   Ile Pro Arg Ile Val Arg Glu Ala Phe Tyr Leu Ala Ser Ser Gly Arg
   210 215 220
   Pro Gly Pro Val Leu Ile Asp Val Pro Lys Asp Ile Gln Gln Gln Leu
   225 230 235 240
   Val Val Pro Lys Trp Asp Glu Pro Ile Arg Leu Pro Gly Tyr Leu Ser
   245 250 255
   Arg Leu Pro Lys Thr Glu Asn Asn Gly Gln Leu Glu His Ile Val Arg
   260 265 270
   Leu Val Ser Glu Ala Lys Arg Pro Val Leu Tyr Val Gly Gly Gly Cys
   275 280 285
   Leu Asn Ser Gly Asp Glu Leu Arg Arg Phe Val Glu Leu Thr Gly Ile
   290 295 300
   Pro Val Ala Ser Thr Leu Met Gly Leu Gly Ala Tyr Pro Ala Ser Ser
   305 310 315 320
   Asp Leu Ser Leu His Met Leu Gly Met His Gly Thr Val Tyr Ala Asn
   325 330 335
   Tyr Ala Val Asp Lys Ser Asp Leu Leu Leu Ala Phe Gly Val Arg Phe
   340 345 350
   Asp Asp Arg Val Thr Gly Lys Leu Glu Ala Phe Ala Ser Arg Ala Lys
   355 360 365
   Ile Val His Ile Asp Ile Asp Ser Ala Glu Ile Gly Lys Asn Lys Gln
   370 375 380
   Pro His Val Ser Ile Cys Gly Asp Ile Lys Val Ala Leu Gln Gly Leu
   385 390 395 400
   Asn Lys Ile Leu Glu Val Lys Asn Ser Val Thr Asn Leu Asp Phe Ser
   405 410 415
   Asn Trp Arg Lys Glu Leu Asp Glu Gln Lys Val Lys Tyr Pro Leu Ser
   420 425 430
   Phe Lys Thr Phe Gly Glu Ala Ile Pro Pro Gln Tyr Ala Ile Gln Val
   435 440 445
   Leu Asp Glu Leu Thr Gly Gly Asn Ala Ile Ile Ser Thr Gly Val Gly
   450 455 460
   Gln His Gln Met Trp Ala Ala Gln Phe Tyr Lys Tyr Asn Lys Pro Arg
   465 470 475 480
   Gln Trp Leu Thr Ser Gly Gly Leu Gly Ala Met Gly Phe Gly Leu Pro
   485 490 495
   Ala Ala Ile Gly Ala Ala Val Ala Arg Pro Asp Ala Val Val Val Asp
   500 505 510
   Ile Asp Gly Asp Gly Ser Phe Ile Met Ser Val Gln Glu Leu Ala Thr
   515 520 525
   Ile Arg Val Glu Asn Leu Pro Val Lys Ile Leu Leu Leu Asn Asn Gln
   530 535 540
   His Leu Gly Met Val Val Gln Trp Glu Asp Arg Phe Tyr Lys Ala Asn
   545 550 555 560
   Arg Ala His Thr Tyr Leu Gly Asn Pro Ser Lys Glu Ser Glu Ile Phe
   565 570 575
   Pro Asn Met Leu Lys Phe Ala Glu Ala Cys Asp Ile Pro Ala Ala Arg
   580 585 590
   Val Thr Arg Lys Ala Asp Leu Arg Ala Ala Ile Gln Lys Met Leu Asp
   595 600 605
   Thr Pro Gly Pro Tyr Leu Leu Asp Val Ile Val Pro His Gln Glu His
   610 615 620
   Val Leu Pro Met Ile Pro Ala Gly Gly Gly Phe Met Asp Val Ile Thr
   625 630 635 640
   Glu Gly Asp Gly Arg Met Lys Tyr
   645
<210> 20
   <211> 640
   <212> PRT
   <213> Lolium multiflorum
<220>
   <221> MSC_FEATURE
   <223> Ryegrass acetohydroxyacid synthase large subunit (LmAHASL)
<210> 21
   <211> 607
   <212> PRT
   <213> Triticum aestivum
<220>
   <221> MSC_FEATURE
   <223> Wheat A genome acetohydroxyacid synthase large subunit (TaAHASL1A), partial
<400> 21
   Leu Pro Ala Arg Val Val Arg Cys Cys Ala Ala Ser Pro Ala Ala Thr
   1 5 10 15
   Ser Ala Ala Pro Pro Ala Thr Ala Leu Arg Pro Trp Gly Pro Ser Glu
   20 25 30
   Pro Arg Lys Gly Ala Asp Ile Leu Val Glu Ala Leu Glu Arg Cys Gly
   35 40 45
   Ile Val Asp Val Phe Ala Tyr Pro Gly Gly Ala Ser Met Glu Ile His
   50 55 60
   Gln Ala Leu Thr Arg Ser Pro Val Ile Thr Asn His Leu Phe Arg His
   65 70 75 80
   Glu Gln Gly Glu Ala Phe Ala Ala Ser Gly Tyr Ala Arg Ala Ser Gly
   85 90 95
   Arg Val Gly Val Cys Val Ala Thr Ser Gly Pro Gly Ala Thr Asn Leu
   100 105 110
   Val Ser Ala Leu Ala Asp Ala Leu Leu Asp Ser Ile Pro Met Val Ala
   115 120 125
   Ile Thr Gly Gln Val Pro Arg Arg Met Ile Gly Thr Asp Ala Phe Gln
       130                 135                 140
   Glu Thr Pro Ile Val Glu Val Thr Arg Ser Ile Thr Lys His Asn Tyr
   145                 150                 155                 160
   Leu Val Leu Asp Val Glu Asp Ile Pro Arg Val Ile Gln Glu Ala Phe
                   165                 170                 175
   Phe Leu Ala Ser Ser Gly Arg Pro Gly Pro Val Leu Val Asp Ile Pro
               180                 185                 190
   Lys Asp Ile Gln Gln Gln Met Ala Val Pro Ile Trp Asp Thr Pro Met
           195                 200                 205
   Ser Leu Pro Gly Tyr Ile Ala Arg Leu Pro Lys Pro Pro Ser Thr Glu
       210                 215                 220
   Ser Leu Glu Gln Val Leu Arg Leu Val Gly Glu Ser Arg Arg Pro Ile
   225                 230                 235                 240
   Leu Tyr Val Gly Gly Gly Cys Ala Ala Ser Gly Glu Glu Leu Arg Arg
                   245                 250                 255
   Phe Val Glu Leu Thr Gly Ile Pro Val Thr Thr Thr Leu Met Gly Leu
               260                 265                 270
   Gly Asn Phe Pro Ser Asp Asp Pro Leu Ser Leu Arg Met Leu Gly Met
           275                 280                 285
   His Gly Thr Val Tyr Ala Asn Tyr Ala Val Asp Lys Ala Asp Leu Leu
       290                 295                 300
   Leu Ala Phe Gly Val Arg Phe Asp Asp Arg Val Thr Gly Lys Ile Glu
   305                 310                 315                 320
   Ala Phe Ala Ser Arg Ser Lys Ile Val His Ile Asp Ile Asp Pro Ala
                   325                 330                 335
   Glu Ile Gly Lys Asn Lys Gln Pro His Val Ser Ile Cys Ala Asp Val
               340                 345                 350
   Lys Leu Ala Leu Gln Gly Leu Asn Ala Leu Leu Asn Gly Ser Lys Ala
           355                 360                 365
   Gln Gln Gly Leu Asp Phe Gly Pro Trp His Lys Glu Leu Asp Gln Gln
       370                 375                 380
   Lys Arg Glu Phe Pro Leu Gly Phe Lys Thr Phe Gly Glu Ala Ile Pro
   385                 390                 395                 400
   Pro Gln Tyr Ala Ile Gln Val Leu Asp Glu Leu Thr Lys Gly Glu Ala
   405 410 415
   Ile Ile Ala Thr Gly Val Gly Gln His Gln Met Trp Ala Ala Gln Tyr
   420 425 430
   Tyr Thr Tyr Lys Arg Pro Arg Gln Trp Leu Ser Ser Ser Gly Leu Gly
   435 440 445
   Ala Met Gly Phe Gly Leu Pro Ala Ala Ala Gly Ala Ala Val Ala Asn
   450 455 460
   Pro Gly Val Thr Val Val Asp Ile Asp Gly Asp Gly Ser Phe Leu Met
   465 470 475 480
   Asn Ile Gln Glu Leu Ala Leu Ile Arg Ile Glu Asn Leu Pro Val Lys
   485 490 495
   Val Met Ile Leu Asn Asn Gln His Leu Gly Met Val Val Gln Trp Glu
   500 505 510
   Asp Arg Phe Tyr Lys Ala Asn Arg Ala His Thr Tyr Leu Gly Asn Pro
   515 520 525
   Glu Asn Glu Ser Glu Ile Tyr Pro Asp Phe Val Thr Ile Ala Lys Gly
   530 535 540
   Phe Asn Val Pro Ala Val Arg Val Thr Lys Lys Ser Glu Val Thr Ala
   545 550 555 560
   Ala Ile Lys Lys Met Leu Glu Thr Pro Gly Pro Tyr Leu Leu Asp Ile
   565 570 575
   Ile Val Pro His Gln Glu His Val Leu Pro Met Ile Pro Ser Gly Gly
   580 585 590
   Ala Phe Lys Asp Met Ile Met Glu Gly Asp Gly Arg Thr Ser Tyr
   595 600 605
<210> 22
   <211> 607
   <212> PRT
   <213> Triticum aestivum
<220>
   <221> MSC_FEATURE
   <223> Wheat B genome acetohydroxyacid synthase large subunit (TaAHASL1B), partial
<400> 22
   Leu Pro Ala Arg Ile Val Arg Cys Cys Ala Ala Ser Pro Ala Ala Thr
   1               5                   10                  15
   Ser Ala Ala Pro Pro Ala Thr Ala Leu Arg Pro Trp Gly Pro Ser Glu
   20 25 30
   Pro Arg Lys Gly Ala Asp Ile Leu Val Glu Ala Leu Glu Arg Cys Gly
   35 40 45
   Ile Val Asp Val Phe Ala Tyr Pro Gly Gly Ala Ser Met Glu Ile His
   50 55 60
   Gln Ala Leu Thr Arg Ser Pro Val Ile Thr Asn His Leu Phe Arg His
   65 70 75 80
   Glu Gln Gly Glu Ala Phe Ala Ala Ser Gly Tyr Ala Arg Ala Ser Gly
   85 90 95
   Arg Val Gly Val Cys Val Ala Thr Ser Gly Pro Gly Ala Thr Asn Leu
   100 105 110
   Val Ser Ala Leu Ala Asp Ala Leu Leu Asp Ser Ile Pro Met Val Ala
   115 120 125
   Ile Thr Gly Gln Val Pro Arg Arg Met Ile Gly Thr Asp Ala Phe Gln
   130 135 140
   Glu Thr Pro Ile Val Glu Val Thr Arg Ser Ile Thr Lys His Asn Tyr
   145 150 155 160
   Leu Val Leu Asp Val Glu Asp Ile Pro Arg Val Ile Gln Glu Ala Phe
   165 170 175
   Phe Leu Ala Ser Ser Gly Arg Pro Gly Pro Val Leu Val Asp Ile Pro
   180 185 190
   Lys Asp Ile Gln Gln Gln Met Ala Val Pro Val Trp Asp Thr Pro Met
   195 200 205
   Ser Leu Pro Gly Tyr Ile Ala Arg Leu Pro Lys Pro Pro Ser Thr Glu
   210 215 220
   Ser Leu Glu Gln Val Leu Arg Leu Val Gly Glu Ser Arg Arg Pro Ile
   225 230 235 240
   Leu Tyr Val Gly Gly Gly Cys Ala Ala Ser Gly Glu Glu Leu Arg Arg
   245 250 255
   Phe Val Glu Leu Thr Gly Ile Pro Val Thr Thr Thr Leu Met Gly Leu
   260 265 270
   Gly Asn Phe Pro Ser Asp Asp Pro Leu Ser Leu Arg Met Leu Gly Met
   275 280 285
   His Gly Thr Val Tyr Ala Asn Tyr Ala Val Asp Lys Ala Asp Leu Leu
   290 295 300
   Leu Ala Phe Gly Val Arg Phe Asp Asp Arg Val Thr Gly Lys Ile Glu
   305 310 315 320
   Ala Phe Ala Ser Arg Ser Lys Ile Val His Ile Asp Ile Asp Pro Ala
   325 330 335
   Glu Ile Gly Lys Asn Lys Gln Pro His Val Ser Ile Cys Ala Asp Val
   340 345 350
   Lys Leu Ala Leu Gln Gly Leu Asn Ala Leu Leu Asn Gly Ser Lys Ala
   355 360 365
   Gln Gln Gly Leu Asp Phe Gly Pro Trp His Lys Glu Leu Asp Gln Gln
   370 375 380
   Lys Arg Glu Phe Pro Leu Gly Phe Lys Thr Phe Gly Glu Ala Ile Pro
   385 390 395 400
   Pro Gln Tyr Ala Ile Gln Val Leu Asp Glu Leu Thr Lys Gly Glu Ala
   405 410 415
   Ile Ile Ala Thr Gly Val Gly Gln His Gln Met Trp Ala Ala Gln Tyr
   420 425 430
   Tyr Thr Tyr Lys Arg Pro Arg Gln Trp Leu Ser Ser Ser Gly Leu Gly
   435 440 445
   Ala Met Gly Phe Gly Leu Pro Ala Ala Ala Gly Ala Ala Val Ala Asn
   450 455 460
   Pro Gly Val Thr Val Val Asp Ile Asp Gly Asp Gly Ser Phe Leu Met
   465 470 475 480
   Asn Ile Gln Glu Leu Ala Leu Ile Arg Ile Glu Asn Leu Pro Val Lys
   485 490 495
   Val Met Ile Leu Asn Asn Gln His Leu Gly Met Val Val Gln Trp Glu
   500 505 510
   Asp Arg Phe Tyr Lys Ala Asn Arg Ala His Thr Tyr Leu Gly Asn Pro
   515 520 525
   Glu Asn Glu Ser Glu Ile Tyr Pro Asp Phe Val Thr Ile Ala Lys Gly
   530 535 540
   Phe Asn Val Pro Ala Val Arg Val Thr Lys Lys Ser Glu Val Thr Ala
   545 550 555 560
   Ala Ile Lys Lys Met Leu Glu Thr Pro Gly Pro Tyr Leu Leu Asp Ile
   565 570 575
   Ile Val Pro His Gln Glu His Val Leu Pro Met Ile Pro Ser Gly Gly
   580 585 590
   Ala Phe Lys Asp Met Ile Met Glu Gly Asp Gly Arg Thr Ser Tyr
   595 600 605
<210> 23
   <211> 607
   <212> PRT
   <213> Triticum aestivum
<220>
   <221> MSC_FEATURE
   <223> Wheat D genome acetohydroxyacid synthase large subunit (TaAHASL1D), partial
<400> 23
   Leu Pro Ala Arg Ile Val Arg Cys Cys Ala Ala Ser Pro Ala Ala Thr
   1 5 10 15
   Ser Ala Ala Pro Pro Ala Thr Ala Leu Arg Pro Trp Gly Pro Ser Glu
   20 25 30
   Pro Arg Lys Gly Ala Asp Ile Leu Val Glu Ala Leu Glu Arg Cys Gly
   35 40 45
   Ile Val Asp Val Phe Ala Tyr Pro Gly Gly Ala Ser Met Glu Ile His
   50 55 60
   Gln Ala Leu Thr Arg Ser Pro Val Ile Thr Asn His Leu Phe Arg His
   65 70 75 80
   Glu Gln Gly Glu Ala Phe Ala Ala Ser Gly Tyr Ala Arg Ala Ser Gly
   85 90 95
   Arg Val Gly Val Cys Val Ala Thr Ser Gly Pro Gly Ala Thr Asn Leu
   100 105 110
   Val Ser Ala Leu Ala Asp Ala Leu Leu Asp Ser Ile Pro Met Val Ala
   115 120 125
   Ile Thr Gly Gln Val Pro Arg Arg Met Ile Gly Thr Asp Ala Phe Gln
   130 135 140
   Glu Thr Pro Ile Val Glu Val Thr Arg Ser Ile Thr Lys His Asn Tyr
   145 150 155 160
   Leu Val Leu Asp Val Glu Asp Ile Pro Arg Val Ile Gln Glu Ala Phe
   165 170 175
   Phe Leu Ala Ser Ser Gly Arg Pro Gly Pro Val Leu Val Asp Ile Pro
   180 185 190
   Lys Asp Ile Gln Gln Gln Met Ala Val Pro Val Trp Asp Thr Pro Met
   195 200 205
   Ser Leu Pro Gly Tyr Ile Ala Arg Leu Pro Lys Pro Pro Ser Thr Glu
   210 215 220
   Ser Leu Glu Gln Val Leu Arg Leu Val Gly Glu Ser Arg Arg Pro Ile
   225 230 235 240
   Leu Tyr Val Gly Gly Gly Cys Ala Ala Ser Gly Glu Glu Leu Arg Arg
   245 250 255
   Phe Val Glu Leu Thr Gly Ile Pro Val Thr Thr Thr Leu Met Gly Leu
   260 265 270
   Gly Asn Phe Pro Ser Asp Asp Pro Leu Ser Leu Arg Met Leu Gly Met
   275 280 285
   His Gly Thr Val Tyr Ala Asn Tyr Ala Val Asp Lys Ala Asp Leu Leu
   290 295 300
   Leu Ala Phe Gly Val Arg Phe Asp Asp Arg Val Thr Gly Lys Ile Glu
   305 310 315 320
   Ala Phe Ala Ser Arg Ser Lys Ile Val His Ile Asp Ile Asp Pro Ala
   325 330 335
   Glu Ile Gly Lys Asn Lys Gln Pro His Val Ser Ile Cys Ala Asp Val
   340 345 350
   Lys Leu Ala Leu Gln Gly Leu Asn Asp Leu Leu Asn Gly Ser Lys Ala
   355 360 365
   Gln Gln Gly Leu Asp Phe Gly Pro Trp His Lys Glu Leu Asp Gln Gln
   370 375 380
   Lys Arg Glu Phe Pro Leu Gly Phe Lys Thr Phe Gly Glu Ala Ile Pro
   385 390 395 400
   Pro Gln Tyr Ala Ile Gln Val Leu Asp Glu Leu Thr Lys Gly Glu Ala
   405 410 415
   Ile Ile Ala Thr Gly Val Gly Gln His Gln Met Trp Ala Ala Gln Tyr
   420 425 430
   Tyr Thr Tyr Lys Arg Pro Arg Gln Trp Leu Ser Ser Ser Gly Leu Gly
   435 440 445
   Ala Met Gly Phe Gly Leu Pro Ala Ala Ala Gly Ala Ala Val Ala Asn
   450 455 460
   Pro Gly Val Thr Val Val Asp Ile Asp Gly Asp Gly Ser Phe Leu Met
   465 470 475 480
   Asn Ile Gln Glu Leu Ala Leu Ile Arg Ile Glu Asn Leu Pro Val Lys
   485 490 495
   Val Met Ile Leu Asn Asn Gln His Leu Gly Met Val Val Gln Trp Glu
   500 505 510
   Asp Arg Phe Tyr Lys Ala Asn Arg Ala His Thr Tyr Leu Gly Asn Pro
   515 520 525
   Glu Asn Glu Ser Glu Ile Tyr Pro Asp Phe Val Thr Ile Ala Lys Gly
   530 535 540
   Phe Asn Val Pro Ala Val Arg Val Thr Lys Lys Ser Glu Val Thr Ala
   545 550 555 560
   Ala Ile Lys Lys Met Leu Glu Thr Pro Gly Pro Tyr Leu Leu Asp Ile
   565 570 575
   Ile Val Pro His Gln Glu His Val Leu Pro Met Ile Pro Ser Gly Gly
   580 585 590
   Ala Phe Lys Asp Met Ile Met Glu Gly Asp Gly Arg Thr Ser Tyr
   595 600 605
<210> 24
   <211> 659
   <212> PRT
   <213> Gossypium hirsutum
<220>
   <221> MSC_FEATURE
   <223> Cotton acetohydroxyacid synthase large subunit (GHAHASA5)
<400> 24
   Met Ala Ala Ala Thr Ala Asn Ser Ala Leu Pro Lys Leu Ser Thr Leu
   1               5                   10                  15
   Thr Ser Ser Phe Lys Ser Ser Ile Pro Ile Ser Lys Ser Ser Leu Pro
               20                  25                  30
   Phe Ser Thr Thr Pro Gln Lys Pro Thr Pro Tyr Arg Ser Phe Asp Val
           35                  40                  45
   Ser Cys Ser Leu Ser His Ala Ser Ser Asn Pro Arg Ser Ala Ala Thr
       50                  55                  60
   Ser Val Thr Pro Lys Asn Ala Pro Pro His Asp Phe Ile Ser Arg Tyr
   65                  70                  75                  80
   Ala Asp Asp Glu Pro Arg Lys Gly Ala Asp Ile Leu Val Glu Ala Leu
   85 90 95
   Val Arg Glu Gly Val Lys Asp Val Phe Ala Tyr Pro Gly Gly Ala Ser
   100 105 110
   Met Glu Ile His Gln Ala Leu Thr Arg Ser Lys Ile Ile Arg Asn Val
   115 120 125
   Leu Pro Arg His Glu Gln Gly Gly Val Phe Ala Ala Glu Gly Tyr Ala
   130 135 140
   Arg Ser Ser Gly Ile Pro Gly Val Cys Ile Ala Thr Ser Gly Pro Gly
   145 150 155 160
   Ala Thr Asn Leu Val Ser Gly Leu Ala Asp Ala Met Leu Asp Ser Ile
   165 170 175
   Pro Leu Val Ala Ile Thr Gly Gln Val Pro Arg Arg Met Ile Gly Thr
   180 185 190
   Asp Ala Phe Gln Glu Thr Pro Ile Val Glu Val Thr Arg Ser Ile Thr
   195 200 205
   Lys His Asn Tyr Leu Val Leu Asp Val Asp Asp Ile Pro Arg Ile Val
   210 215 220
   Ser Glu Ala Phe Phe Leu Ala Ser Ser Gly Arg Pro Gly Pro Val Leu
   225 230 235 240
   Ile Asp Val Pro Lys Asp Ile Gln Gln Gln Leu Ala Val Pro Lys Trp
   245 250 255
   Asn His Ser Leu Arg Leu Pro Gly Tyr Leu Ser Arg Leu Pro Lys Ala
   260 265 270
   Pro Gly Glu Ala His Leu Glu Gln Ile Val Arg Leu Val Ser Glu Ser
   275 280 285
   Lys Lys Pro Val Leu Tyr Val Gly Gly Gly Cys Leu Asn Ser Ser Glu
   290 295 300
   Glu Leu Lys Arg Phe Val Glu Leu Thr Gly Ile Pro Val Ala Ser Thr
   305 310 315 320
   Leu Met Gly Leu Gly Ala Phe Pro Ile Ser Asp Asp Leu Ser Leu Gln
   325 330 335
   Met Leu Gly Met His Gly Thr Val Tyr Ala Asn Tyr Ala Val Asp Lys
   340 345 350
   Ser Asp Leu Leu Leu Ala Phe Gly Val Arg Phe Asp Asp Arg Val Thr
   355 360 365
   Gly Lys Leu Glu Ala Phe Ala Ser Arg Ala Lys Ile Val His Ile Asp
   370 375 380
   Ile Asp Ser Ala Glu Ile Gly Lys Asn Lys Gln Pro His Val Ser Val
   385 390 395 400
   Cys Ser Asp Val Lys Leu Ala Leu Gln Gly Ile Asn Lys Ile Leu Glu
   405 410 415
   Thr Lys Val Ala Lys Leu Asn Leu Asp Tyr Ser Glu Trp Arg Gln Glu
   420 425 430
   Leu Asn Glu Gln Lys Leu Lys Phe Pro Leu Ser Tyr Lys Thr Phe Gly
   435 440 445
   Glu Ala Ile Pro Pro Gln Tyr Ala Ile Gln Val Leu Asp Glu Leu Thr
   450 455 460
   Gly Gly Asn Ala Ile Ile Ser Thr Gly Val Gly Gln His Gln Met Trp
   465 470 475 480
   Ala Ala Gln Phe Tyr Lys Tyr Lys Lys Pro Arg Gln Trp Leu Thr Ser
   485 490 495
   Gly Gly Leu Gly Ala Met Gly Phe Gly Leu Pro Ala Ala Ile Gly Ala
   500 505 510
   Ala Val Ala Asn Pro Glu Ala Val Val Val Asp Ile Asp Gly Asp Gly
   515 520 525
   Ser Phe Ile Met Asn Val Gln Glu Leu Ala Thr Ile Arg Val Glu Asn
   530 535 540
   Leu Pro Val Lys Ile Leu Leu Leu Asn Asn Gln His Leu Gly Met Val
   545 550 555 560
   Val Gln Trp Glu Asp Arg Phe Tyr Lys Ala Asn Arg Ala His Thr Tyr
   565 570 575
   Leu Gly Asp Pro Ser Asn Glu Ser Glu Ile Phe Pro Asn Met Leu Lys
   580 585 590
   Phe Ala Glu Ala Cys Gly Ile Pro Ala Ala Arg Val Thr Lys Lys Glu
   595 600 605
   Asp Leu Lys Ala Ala Met Gln Lys Met Leu Asp Thr Pro Gly Pro Tyr
   610 615 620
   Leu Leu Asp Val Ile Val Pro His Gln Glu His Val Leu Pro Met Ile
   625 630 635 640
   Pro Ser Gly Gly Ala Phe Lys Asp Val Ile Thr Glu Gly Asp Gly Arg
   645 650 655
   Thr Gln Tyr
<210> 25
   <211> 659
   <212> PRT
   <213> Gossypium hirsutum
<220>
   <221> MSC_FEATURE
   <223> Cotton acetohydroxyacid synthase large subunit (GHAHASA19)
<400> 25
   Met Ala Ala Ala Thr Ser Asn Ser Ala Leu Pro Lys Leu Ser Thr Leu
   1 5 10 15
   Thr Ser Ser Phe Lys Ser Ser Ile Pro Ile Ser Lys Ser Ser Leu Pro
   20 25 30
   Phe Ser Thr Thr Pro Gln Lys Pro Thr Pro Tyr Arg Ser Phe Asp Val
   35 40 45
   Ser Cys Ser Leu Ser His Ala Ser Ser Asn Pro Arg Asn Pro Ala Ala
   50 55 60
   Ser Val Thr Gln Lys Thr Ala Pro Pro His Tyr Phe Ile Ser Arg Tyr
   65 70 75 80
   Ala Asp Asp Glu Pro Arg Lys Gly Ala Asp Ile Leu Val Glu Ala Leu
   85 90 95
   Glu Arg Glu Gly Val Lys Asp Val Phe Ala Tyr Pro Gly Gly Ala Ser
   100 105 110
   Met Glu Ile His Gln Ala Leu Thr Arg Ser Lys Ile Ile Arg Asn Val
   115 120 125
   Leu Pro Arg His Glu Gln Gly Gly Val Phe Ala Ala Glu Gly Tyr Ala
   130 135 140
   Arg Ser Ser Gly Ile Ser Gly Val Cys Ile Ala Thr Ser Gly Pro Gly
   145 150 155 160
   Arg Thr Asn Leu Val Ser Gly Leu Ala Asp Ala Met Leu Asp Ser Ile
   165 170 175
   Pro Leu Val Ala Ile Thr Gly Gln Val Pro Arg Arg Met Ile Gly Thr
   180 185 190
   Asp Ala Phe Gln Glu Thr Pro Ile Val Glu Val Thr Arg Ser Ile Thr
   195 200 205
   Lys His Asn Tyr Leu Val Leu Asp Val Asp Asp Ile Pro Arg Ile Val
   210 215 220
   Ser Glu Ala Phe Phe Leu Ala Ser Ser Gly Arg Pro Gly Pro Val Leu
   225 230 235 240
   Ile Asp Val Pro Lys Asp Ile Gln Gln Gln Leu Ala Val Pro Lys Trp
   245 250 255
   Asn His Ser Leu Arg Leu Pro Gly Tyr Leu Ser Arg Leu Pro Lys Ala
   260 265 270
   Pro Ala Glu Ala His Leu Glu Gln Ile Val Arg Leu Val Ser Glu Ser
   275 280 285
   Lys Lys Pro Val Leu Tyr Val Gly Gly Gly Cys Leu Asn Ser Ser Glu
   290 295 300
   Glu Leu Lys Arg Phe Val Glu Leu Thr Gly Ile Pro Val Ala Ser Thr
   305 310 315 320
   Leu Met Gly Leu Gly Ala Phe Pro Ile Ser Asp Glu Leu Ser Leu Gln
   325 330 335
   Met Leu Gly Met His Gly Thr Val Tyr Ala Asn Tyr Ala Val Asp Lys
   340 345 350
   Ser Asp Leu Leu Leu Ala Phe Gly Val Arg Phe Asp Asp Arg Val Thr
   355 360 365
   Gly Lys Leu Glu Ala Phe Ala Ser Arg Ala Lys Ile Val His Ile Asp
   370 375 380
   Ile Asp Ser Ala Glu Ile Gly Lys Asn Lys Gln Pro His Met Ser Val
   385 390 395 400
   Cys Ser Asp Val Lys Leu Ala Leu Gln Gly Ile Asn Lys Ile Leu Glu
   405 410 415
   Thr Thr Gly Ala Lys Leu Asn Leu Asp Tyr Ser Glu Trp Arg Gln Glu
   420 425 430
   Leu Asn Glu Gln Lys Leu Lys Phe Pro Leu Ser Tyr Lys Thr Phe Gly
   435 440 445
   Glu Ala Ile Pro Pro Gln Tyr Ala Ile Gln Val Leu Asp Glu Leu Thr
   450 455 460
   Gly Gly Asn Ala Ile Ile Ser Thr Gly Val Gly Gln His Gln Met Trp
   465 470 475 480
   Ala Ala Gln Phe Tyr Lys Tyr Lys Lys Pro Arg Gln Trp Leu Thr Ser
   485 490 495
   Gly Gly Leu Gly Ala Met Gly Phe Gly Leu Pro Ala Ala Ile Gly Ala
   500 505 510
   Ala Val Ala Asn Pro Glu Ala Val Val Val Asp Ile Asp Gly Asp Gly
   515 520 525
   Ser Phe Ile Met Asn Val Gln Glu Leu Ala Thr Ile Arg Val Glu Asn
   530 535 540
   Leu Pro Val Lys Ile Leu Leu Leu Asn Asn Gln His Leu Gly Met Val
   545 550 555 560
   Val Gln Trp Glu Asp Arg Phe Tyr Lys Ala Asn Arg Ala His Thr Tyr
   565 570 575
   Leu Gly Asp Pro Ser Asn Glu Ser Glu Ile Phe Pro Asn Met Leu Lys
   580 585 590
   Phe Ala Glu Ala Cys Gly Ile Pro Ala Ala Arg Val Thr Lys Lys Glu
   595 600 605
   Asp Leu Lys Ala Ala Ile Gln Lys Met Leu Asp Thr Pro Gly Pro Tyr
   610 615 620
   Leu Leu Asp Val Ile Val Pro His Gln Glu His Val Leu Pro Met Ile
   625 630 635 640
   Pro Ser Gly Gly Ala Phe Lys Asp Val Ile Thr Glu Gly Asp Gly Arg
   645 650 655
   Thr Gln Tyr
<210> 26
   <211> 562
   <212> PRT
   <213> Escherichia coli
<220>
   <221> MSC_FEATURE
   <223> E. coli i acetohydroxyacid synthase large subunit I (IIvB)
<400> 26
   Met Ala Ser Ser Gly Thr Thr Ser Thr Arg Lys Arg Phe Thr Gly Ala
   1               5                   10                  15
   Glu Phe Ile Val His Phe Leu Glu Gln Gln Gly Ile Lys Ile Val Thr
               20                  25                  30
   Gly Ile Pro Gly Gly Ser Ile Leu Pro Val Tyr Asp Ala Leu Ser Gln
   35 40 45
   Ser Thr Gln Ile Arg His Ile Leu Ala Arg His Glu Gln Gly Ala Gly
   50 55 60
   Phe Ile Ala Gln Gly Met Ala Arg Thr Asp Gly Lys Pro Ala Val Cys
   65 70 75 80
   Met Ala Cys Ser Gly Pro Gly Ala Thr Asn Leu Val Thr Ala Ile Ala
   85 90 95
   Asp Ala Arg Leu Asp Ser Ile Pro Leu Ile Cys Ile Thr Gly Gln Val
   100 105 110
   Pro Ala Ser Met Ile Gly Thr Asp Ala Phe Gln Glu Val Asp Thr Tyr
   115 120 125
   Gly Ile Ser Ile Pro Ile Thr Lys His Asn Tyr Leu Val Arg His Ile
   130 135 140
   Glu Glu Leu Pro Gln Val Met Ser Asp Ala Phe Arg Ile Ala Gln Ser
   145 150 155 160
   Gly Arg Pro Gly Pro Val Trp Ile Asp Ile Pro Lys Asp Val Gln Thr
   165 170 175
   Ala Val Phe Glu Ile Glu Thr Gln Pro Ala Met Ala Glu Lys Ala Ala
   180 185 190
   Ala Pro Ala Phe Ser Glu Glu Ser Ile Arg Asp Ala Ala Ala Met Ile
   195 200 205
   Asn Ala Ala Lys Arg Pro Val Leu Tyr Leu Gly Gly Gly Val Ile Asn
   210 215 220
   Ala Pro Ala Arg Val Arg Glu Leu Ala Glu Lys Ala Gln Leu Pro Thr
   225 230 235 240
   Thr Met Thr Leu Met Ala Leu Gly Met Leu Pro Lys Ala His Pro Leu
   245 250 255
   Ser Leu Gly Met Leu Gly Met His Gly Val Arg Ser Thr Asn Tyr Ile
   260 265 270
   Leu Gln Glu Ala Asp Leu Leu Ile Val Leu Gly Ala Arg Phe Asp Asp
   275 280 285
   Arg Ala Ile Gly Lys Thr Glu Gln Phe Cys Pro Asn Ala Lys Ile Ile
   290 295 300
   His Val Asp Ile Asp Arg Ala Glu Leu Gly Lys Ile Lys Gln Pro His
   305 310 315 320
   Val Ala Ile Gln Ala Asp Val Asp Asp Val Leu Ala Gln Leu Ile Pro
   325 330 335
   Leu Val Glu Ala Gln Pro Arg Ala Glu Trp His Gln Leu Val Ala Asp
   340 345 350
   Leu Gln Arg Glu Phe Pro Cys Pro Ile Pro Lys Ala Cys Asp Pro Leu
   355 360 365
   Ser His Tyr Gly Leu Ile Asn Ala Val Ala Ala Cys Val Asp Asp Asn
   370 375 380
   Ala Ile Ile Thr Thr Asp Val Gly Gln His Gln Met Trp Thr Ala Gln
   385 390 395 400
   Ala Tyr Pro Leu Asn Arg Pro Arg Gln Trp Leu Thr Ser Gly Gly Leu
   405 410 415
   Gly Thr Met Gly Phe Gly Leu Pro Ala Ala Ile Gly Ala Ala Leu Ala
   420 425 430
   Asn Pro Asp Arg Lys Val Leu Cys Phe Ser Gly Asp Gly Ser Leu Met
   435 440 445
   Met Asn Ile Gln Glu Met Ala Thr Ala Ser Glu Asn Gln Leu Asp Val
   450 455 460
   Lys Ile Ile Leu Met Asn Asn Glu Ala Leu Gly Leu Val His Gln Gln
   465 470 475 480
   Gln Ser Leu Phe Tyr Glu Gln Gly Val Phe Ala Ala Thr Tyr Pro Gly
   485 490 495
   Lys Ile Asn Phe Met Gln Ile Ala Ala Gly Phe Gly Leu Glu Thr Cys
   500 505 510
   Asp Leu Asn Asn Glu Ala Asp Pro Gln Ala Ser Leu Gln Glu Ile Ile
   515 520 525
   Asn Arg Pro Gly Pro Ala Leu Ile His Val Arg Ile Asp Ala Glu Glu
   530 535 540
   Lys Val Tyr Pro Met Val Pro Pro Gly Ala Ala Asn Thr Glu Met Val
   545 550 555 560
   Gly Glu
<210> 27
   <211> 548
   <212> PRT
   <213> Escherichia coli
<220>
   <221> MSC_FEATURE
   <223> E. coli acetohydroxyacid synthase large subunit I I (IIvG)
<400> 27
   Met Asn Gly Ala Gln Trp Val Val His Ala Leu Arg Ala Gln Gly Val
   1               5                   10                  15
   Asn Thr Val Phe Gly Tyr Pro Gly Gly Ala Ile Met Pro Val Tyr Asp
               20                  25                  30
   Ala Leu Tyr Asp Gly Gly Val Glu His Leu Leu Cys Arg His Glu Gln
           35                  40                  45
   Gly Ala Ala Met Ala Ala Ile Gly Tyr Ala Arg Ala Thr Gly Lys Thr
       50                  55                  60
   Gly Val Cys Ile Ala Thr Ser Gly Pro Gly Ala Thr Asn Leu Ile Thr
   65                  70                  75                  80
   Gly Leu Ala Asp Ala Leu Leu Asp Ser Ile Pro Val Val Ala Ile Thr
                   85                  90                  95
   Gly Gln Val Ser Ala Pro Phe Ile Gly Thr Asp Ala Phe Gln Glu Val
               100                 105                 110
   Asp Val Leu Gly Leu Ser Leu Ala Cys Thr Lys His Ser Phe Leu Val
           115                 120                 125
   Gln Ser Leu Glu Glu Leu Pro Arg Ile Met Ala Glu Ala Phe Asp Val
       130                 135                 140
   Ala Cys Ser Gly Arg Pro Gly Pro Val Leu Val Asp Ile Pro Lys Asp
   145                 150                 155                 160
   Ile Gln Leu Ala Ser Gly Asp Leu Glu Pro Trp Phe Thr Thr Val Glu
                   165                 170                 175
   Asn Glu Val Thr Phe Pro His Ala Glu Val Glu Gln Ala Arg Gln Met
               180                 185                 190
   Leu Ala Lys Ala Gln Lys Pro Met Leu Tyr Val Gly Gly Gly Val Gly
           195                 200                 205
   Met Ala Gln Ala Val Pro Ala Leu Arg Glu Phe Leu Ala Ala Thr Lys
       210                 215                 220
   Met Pro Ala Thr Cys Thr Leu Lys Gly Leu Gly Ala Val Glu Ala Asp
   225 230 235 240
   Tyr Pro Tyr Tyr Leu Gly Met Leu Gly Met His Gly Thr Lys Ala Ala
   245 250 255
   Asn Phe Ala Val Gln Glu Cys Asp Leu Leu Ile Ala Val Gly Ala Arg
   260 265 270
   Phe Asp Asp Arg Val Thr Gly Lys Leu Asn Thr Phe Ala Pro His Ala
   275 280 285
   Ser Val Ile His Met Asp Ile Asp Pro Ala Glu Met Asn Lys Leu Arg
   290 295 300
   Gln Ala His Val Ala Leu Gln Gly Asp Leu Asn Ala Leu Leu Pro Ala
   305 310 315 320
   Leu Gln Gln Pro Leu Asn Gln Tyr Asp Trp Gln Gln His Cys Ala Gln
   325 330 335
   Leu Arg Asp Glu His Ser Trp Arg Tyr Asp His Pro Gly Asp Ala Ile
   340 345 350
   Tyr Ala Pro Leu Leu Leu Lys Gln Leu Ser Asp Arg Lys Pro Ala Asp
   355 360 365
   Cys Val Val Thr Thr Asp Val Gly Gln His Gln Met Trp Ala Ala Gln
   370 375 380
   His Ile Ala His Thr Arg Pro Glu Asn Phe Ile Thr Ser Ser Gly Leu
   385 390 395 400
   Gly Thr Met Gly Phe Gly Leu Pro Ala Ala Val Gly Ala Gln Val Ala
   405 410 415
   Arg Pro Asn Asp Thr Val Val Cys Ile Ser Gly Asp Gly Ser Phe Met
   420 425 430
   Met Asn Val Gln Glu Leu Gly Thr Val Lys Arg Lys Gln Leu Pro Leu
   435 440 445
   Lys Ile Val Leu Leu Asp Asn Gln Arg Leu Gly Met Val Arg Gln Trp
   450 455 460
   Gln Gln Leu Phe Phe Gln Glu Arg Tyr Ser Glu Thr Thr Leu Thr Asp
   465 470 475 480
   Asn Pro Asp Phe Leu Met Leu Ala Ser Ala Phe Gly Ile His Gly Gln
   485 490 495
   His Ile Thr Arg Lys Asp Gln Val Glu Ala Ala Leu Asp Thr Met Leu
   500 505 510
   Asn Ser Asp Gly Pro Tyr Leu Leu His Val Ser Ile Asp Glu Leu Glu
   515 520 525
   Asn Val Trp Pro Leu Val Pro Pro Gly Ala Ser Asn Ser Glu Met Leu
   530 535 540
   Glu Lys Leu Ser
   545
<210> 28
   <211> 574
   <212> PRT
   <213> Escherichia coli
<220>
   <221> MSC_FEATURE
   <223> E. coli i acetohydroxyacid synthase large subunit III (IIvI)
<400> 28
   Met Glu Met Leu Ser Gly Ala Glu Met Val Val Arg Ser Leu Ile Asp
   1 5 10 15
   Gln Gly Val Lys Gln Val Phe Gly Tyr Pro Gly Gly Ala Val Leu Asp
   20 25 30
   Ile Tyr Asp Ala Leu His Thr Val Gly Gly Ile Asp His Val Leu Val
   35 40 45
   Arg His Glu Gln Ala Ala Val His Met Ala Asp Gly Leu Ala Arg Ala
   50 55 60
   Thr Gly Glu Val Gly Val Val Leu Val Thr Ser Gly Pro Gly Ala Thr
   65 70 75 80
   Asn Ala Ile Thr Gly Ile Ala Thr Ala Tyr Met Asp Ser Ile Pro Leu
   85 90 95
   Val Val Leu Ser Gly Gln Val Ala Thr Ser Leu Ile Gly Tyr Asp Ala
   100 105 110
   Phe Gln Glu Cys Asp Met Val Gly Ile Ser Arg Pro Val Val Lys His
   115 120 125
   Ser Phe Leu Val Lys Gln Thr Glu Asp Ile Pro Gln Val Leu Lys Lys
   130 135 140
   Ala Phe Trp Leu Ala Ala Ser Gly Arg Pro Gly Pro Val Val Val Asp
   145 150 155 160
   Leu Pro Lys Asp Ile Leu Asn Pro Ala Asn Lys Leu Pro Tyr Val Trp
   165 170 175
   Pro Glu Ser Val Ser Met Arg Ser Tyr Asn Pro Thr Thr Thr Gly His
   180 185 190
   Lys Gly Gln Ile Lys Arg Ala Leu Gln Thr Leu Val Ala Ala Lys Lys
   195 200 205
   Pro Val Val Tyr Val Gly Gly Gly Ala Ile Thr Ala Gly Cys His Gln
   210 215 220
   Gln Leu Lys Glu Thr Val Glu Ala Leu Asn Leu Pro Val Val Cys Ser
   225 230 235 240
   Leu Met Gly Leu Gly Ala Phe Pro Ala Thr His Arg Gln Ala Leu Gly
   245 250 255
   Met Leu Gly Met His Gly Thr Tyr Glu Ala Asn Met Thr Met His Asn
   260 265 270
   Ala Asp Val Ile Phe Ala Val Gly Val Arg Phe Asp Asp Arg Thr Thr
   275 280 285
   Asn Asn Leu Ala Lys Tyr Cys Pro Asn Ala Thr Val Leu His Ile Asp
   290 295 300
   Ile Asp Pro Thr Ser Ile Ser Lys Thr Val Thr Ala Asp Ile Pro Ile
   305 310 315 320
   Val Gly Asp Ala Arg Gln Val Leu Glu Gln Met Leu Glu Leu Leu Ser
   325 330 335
   Gln Glu Ser Ala His Gln Pro Leu Asp Glu Ile Arg Asp Trp Trp Gln
   340 345 350
   Gln Ile Glu Gln Trp Arg Ala Arg Gln Cys Leu Lys Tyr Asp Thr His
   355 360 365
   Ser Glu Lys Ile Lys Pro Gln Ala Val Ile Glu Thr Leu Trp Arg Leu
   370 375 380
   Thr Lys Gly Asp Ala Tyr Val Thr Ser Asp Val Gly Gln His Gln Met
   385 390 395 400
   Phe Ala Ala Leu Tyr Tyr Pro Phe Asp Lys Pro Arg Arg Trp Ile Asn
   405 410 415
   Ser Gly Gly Leu Gly Thr Met Gly Phe Gly Leu Pro Ala Ala Leu Gly
   420 425 430
   Val Lys Met Ala Leu Pro Glu Glu Thr Val Val Cys Val Thr Gly Asp
   435 440 445
   Gly Ser Ile Gln Met Asn Ile Gln Glu Leu Ser Thr Ala Leu Gln Tyr
       450                 455                 460
   Glu Leu Pro Val Leu Val Val Asn Leu Asn Asn Arg Tyr Leu Gly Met
   465                 470                 475                 480
   Val Lys Gln Trp Gln Asp Met Ile Tyr Ser Gly Arg His Ser Gln Ser
                   485                 490                 495
   Tyr Met Gln Ser Leu Pro Asp Phe Val Arg Leu Ala Glu Ala Tyr Gly
               500                 505                 510
   His Val Gly Ile Gln Ile Ser His Pro His Glu Leu Glu Ser Lys Leu
           515                 520                 525
   Ser Glu Ala Leu Glu Gln Val Arg Asn Asn Arg Leu Val Phe Val Asp
       530                 535                 540
   Val Thr Val Asp Gly Ser Glu His Val Tyr Pro Met Gln Ile Arg Gly
   545                 550                 555                 560
   Gly Gly Met Asp Glu Met Trp Leu Ser Lys Thr Glu Arg Thr
                   565                 570
<210> 29
   <211> 1917
   <212> DNA
   <213> Zea mays
<220>
   <221> misc_feature
   <223> Maize acetohydroxyacid synthase large subunit 2 (ZmAHASL2) codi ng sequence, S621N allele.
<400> 29
   atggccaccg ccgccgccgc gtctaccgcg ctcactggcg ccactaccgc tgcgcccaag      60
   gcgaggcgcc gggcgcacct cctggccacc cgccgcgccc tcgccgcgcc catcaggtgc     120
   tcagcggcgt cacccgccat gccgatggct cccccggcca ccccgctccg gccgtggggc     180
   cccaccgatc cccgcaaggg cgccgacatc ctcgtcgagt ccctcgagcg ctgcggcgtc     240
   cgcgacgtct tcgcctaccc cggcggcgcg tccatggaga tccaccaggc actcacccgc     300
   tcccccgtca tcgccaacca cctcttccgc cacgagcaag ggaggccttt tgcggcctcc     360
   ggctacgcgc gctcctcggg ccgcgtcggc gtctgcatcg ccacctccgg ccccggcgcc     420
   accaaccttg tctccgcgct cgccgacgcg ctgctcgatt ccgtccccat ggtcgccatc     480
   acgggacagg tgccgcgacg catgattggc accgacgcct tccaggagac gcccatcgtc     540
   gaggtcaccc gctccatcac caagcacaac tacctggtcc tcgacgtcga cgacatcccc     600
   cgcgtcgtgc aggaggcttt cttcctcgcc tcctctggtc gaccggggcc ggtgcttgtc     660
   gacat cccca  aggacat cca  gcagcagat g  gcggt gcct g  t ct gggacaa  gcccat gagt   720
   ctgcctgggt acattgcgcg ccttcccaag ccccctgcga ctgagttgct tgagcaggtg     780
   ctgcgtcttg ttggtgaatc ccggcgccct gttctttatg ttggcggtgg ctgcgcagca     840
   t ct ggt gagg  agt t gcgacg  ct t t gt ggag  ct gact ggaa  t cccggt cac  aact act ct t   900
   atgggcctcg gcaacttccc cagcgacgac ccactgtctc tgcgcatgct aggtatgcat     960
   ggcacggt gt  at gcaaat t a  t gcagt ggat  aaggccgat c  t gt t gct t gc  act t ggt gt g  1020
   cggtttgatg atcgtgtgac agggaagatt gaggcttttg caagcagggc taagattgtg    1080
   cacgttgata ttgatccggc tgagattggc aagaacaagc agccacatgt gtccatctgt    1140
   gcagat gt t a  agct t gct t t  gcagggcat g  aat gct ct t c  t t gaaggaag  cacat caaag  1200
   aagagctttg actttggctc atggaacgat gagttggatc agcagaagag ggaattcccc    1260
   cttgggtata aaacatctaa tgaggagatc cagccacaat atgctattca ggttcttgat    1320
   gagctgacga aaggcgaggc catcatcggc acaggtgttg ggcagcacca gatgtgggcg    1380
   gcacagtact acacttacaa gcggccaagg cagtggttgt cttcagctgg tcttggggct    1440
   atgggatttg gtttgccggc tgctgctggt gcttctgtgg ccaacccagg tgttactgtt    1500
   gt t gacat cg  at ggagat gg  t agct t t ct c  at gaacgt t c  aggagct agc  t at gat ccga  1560
   at t gagaacc  t cccggt gaa  ggt ct t t gt g  ct aaacaacc  agcacct ggg  gat ggt ggt g  1620
   cagtgggagg acaggttcta taaggccaac agagcgcaca catacttggg aaacccagag    1680
   aat gaaagt g  agat at at cc  agat t t cgt g  acgat cgcca  aagggt t caa  cat t ccagcg  1740
   gtccgtgtga caaagaagaa cgaagtccgc gcagcgataa agaagatgct cgagactcca    1800
   gggccgtacc tcttggatat aatcgtccca caccaggagc atgtgttgcc tatgatccct    1860
   aat ggt gggg  ct t t caagga  t at gat cct g  gat ggt gat g  gcaggact gt  gt act ga     1917
<210> 30
   <211> 2013
   <212> DNA
   <213> Arabidopsis thaliana
<220>
   <221> misc_feature
   <223> Arabidopsis acetohydroxyacid synthase large subunit (At AHASL) codi ng sequence, S653N mutant allele.
<400> 30
   atggcggcgg caacaacaac aacaacaaca tcttcttcga tctccttctc caccaaacca      60
   t ct cct t cct   cct ccaaat c  accat t acca  at ct ccagat   t ct ccct ccc  at t ct ccct a    120
   aaccccaaca aatcatcctc ctcctcccgc cgccgcggta tcaaatccag ctctccctcc     180
   tccatctccg ccgtgctcaa cacaaccacc aatgtcacaa ccactccctc tccaaccaaa     240
   cctaccaaac ccgaaacatt catctcccga ttcgctccag atcaaccccg caaaggcgct     300
   gat at cct cg  t cgaagct t t   agaacgt caa  ggcgt agaaa  ccgt at t cgc  t t accct gga    360
   ggt gcat caa  t ggagat t ca  ccaagcct t a  acccgct ct t   cct caat ccg  t aacgt cct t    420
   cctcgtcacg aacaaggagg tgtattcgca gcagaaggat acgctcgatc ctcaggtaaa     480
   ccaggt at ct   gt at agccac  t t caggt ccc  ggagct acaa  at ct cgt t ag  cggat t agcc    540
   gatgcgttgt tagatagtgt tcctcttgta gcaatcacag gacaagtccc tcgtcgtatg 600
   attggtacag atgcgtttca agagactccg attgttgagg taacgcgttc gattacgaag 660
   cataactatc ttgtgatgga tgttgaagat atccctagga ttattgagga agctttcttt 720
   ttagctactt ctggtagacc tggacctgtt ttggttgatg ttcctaaaga tattcaacaa 780
   cagcttgcga ttcctaattg ggaacaggct atgagattac ctggttatat gtctaggatg 840
   cctaaacctc cggaagattc tcatttggag cagattgtta ggttgatttc tgagtctaag 900
   aagcctgtgt tgtatgttgg tggtggttgt ttgaattcta gcgatgaatt gggtaggttt 960
   gttgagctta cggggatccc tgttgcgagt acgttgatgg ggctgggatc ttatccttgt 1020
   gatgatgagt tgtcgttaca tatgcttgga atgcatggga ctgtgtatgc aaattacgct 1080
   gtggagcata gtgatttgtt gttggcgttt ggggtaaggt ttgatgatcg tgtcacgggt 1140
   aagcttgagg cttttgctag tagggctaag attgttcata ttgatattga ctcggctgag 1200
   attgggaaga ataagactcc tcatgtgtct gtgtgtggtg atgttaagct ggctttgcaa 1260
   gggatgaata aggttcttga gaaccgagcg gaggagctta agcttgattt tggagtttgg 1320
   aggaatgagt tgaacgtaca gaaacagaag tttccgttga gctttaagac gtttggggaa 1380
   gctattcctc cacagtatgc gattaaggtc cttgatgagt tgactgatgg aaaagccata 1440
   ataagtactg gtgtcgggca acatcaaatg tgggcggcgc agttctacaa ttacaagaaa 1500
   ccaaggcagt ggctatcatc aggaggcctt ggagctatgg gatttggact tcctgctgcg 1560
   attggagcgt ctgttgctaa ccctgatgcg atagttgtgg atattgacgg agatggaagc 1620
   tttataatga atgtgcaaga gctagccact attcgtgtag agaatcttcc agtgaaggta 1680
   cttttattaa acaaccagca tcttggcatg gttatgcaat gggaagatcg gttctacaaa 1740
   gctaaccgag ctcacacatt tctcggggat ccggctcagg aggacgagat attcccgaac 1800
   atgttgctgt ttgcagcagc ttgcgggatt ccagcggcga gggtgacaaa gaaagcagat 1860
   ctccgagaag ctattcagac aatgctggat acaccaggac cttacctgtt ggatgtgatt 1920
   tgtccgcacc aagaacatgt gttgccgatg atcccgaatg gtggcacttt caacgatgtc 1980
   ataacggaag gagatggccg gattaaatac tga 2013
<210> 31
   <211> 2013
   <212> DNA
   <213> Arabidopsis thaliana
<220>
   <221> misc_feature
   <223> Arabidopsis acetohydroxyacid synthase large subunit (At AHASL) coding sequence, wild type allele.
<400> 31
   atggcggcgg caacaacaac aacaacaaca tcttcttcga tctccttctc caccaaacca 60
   tctccttcct cctccaaatc accattacca atctccagat tctccctccc attctcccta 120
   aaccccaaca aatcatcctc ctcctcccgc cgccgcggta tcaaatccag ctctccctcc 180
   tccatctccg ccgtgctcaa cacaaccacc aatgtcacaa ccactccctc tccaaccaaa     240
   cctaccaaac ccgaaacatt catctcccga ttcgctccag atcaaccccg caaaggcgct     300
   gatatcctcg tcgaagcttt agaacgtcaa ggcgtagaaa ccgtattcgc ttaccctgga     360
   ggtgcatcaa tggagattca ccaagcctta acccgctctt cctcaatccg taacgtcctt     420
   cctcgtcacg aacaaggagg tgtattcgca gcagaaggat acgctcgatc ctcaggtaaa     480
   ccaggtatct gtatagccac ttcaggtccc ggagctacaa atctcgttag cggattagcc     540
   gatgcgttgt tagatagtgt tcctcttgta gcaatcacag gacaagtccc tcgtcgtatg     600
   attggtacag atgcgtttca agagactccg attgttgagg taacgcgttc gattacgaag     660
   cataactatc ttgtgatgga tgttgaagat atccctagga ttattgagga agctttcttt     720
   ttagctactt ctggtagacc tggacctgtt ttggttgatg ttcctaaaga tattcaacaa     780
   cagcttgcga ttcctaattg ggaacaggct atgagattac ctggttatat gtctaggatg     840
   cctaaacctc cggaagattc tcatttggag cagattgtta ggttgatttc tgagtctaag     900
   aagcctgtgt tgtatgttgg tggtggttgt ttgaattcta gcgatgaatt gggtaggttt     960
   gttgagctta cggggatccc tgttgcgagt acgttgatgg ggctgggatc ttatccttgt    1020
   gatgatgagt tgtcgttaca tatgcttgga atgcatggga ctgtgtatgc aaattacgct    1080
   gtggagcata gtgatttgtt gttggcgttt ggggtaaggt ttgatgatcg tgtcacgggt    1140
   aagcttgagg cttttgctag tagggctaag attgttcata ttgatattga ctcggctgag    1200
   attgggaaga ataagactcc tcatgtgtct gtgtgtggtg atgttaagct ggctttgcaa    1260
   gggatgaata aggttcttga gaaccgagcg gaggagctta agcttgattt tggagtttgg    1320
   aggaatgagt tgaacgtaca gaaacagaag tttccgttga gctttaagac gtttggggaa    1380
   gctattcctc cacagtatgc gattaaggtc cttgatgagt tgactgatgg aaaagccata    1440
   ataagtactg gtgtcgggca acatcaaatg tgggcggcgc agttctacaa ttacaagaaa    1500
   ccaaggcagt ggctatcatc aggaggcctt ggagctatgg gatttggact tcctgctgcg    1560
   attggagcgt ctgttgctaa ccctgatgcg atagttgtgg atattgacgg agatggaagc    1620
   tttataatga atgtgcaaga gctagccact attcgtgtag agaatcttcc agtgaaggta    1680
   cttttattaa acaaccagca tcttggcatg gttatgcaat gggaagatcg gttctacaaa    1740
   gctaaccgag ctcacacatt tctcggggat ccggctcagg aggacgagat attcccgaac    1800
   atgttgctgt ttgcagcagc ttgcgggatt ccagcggcga gggtgacaaa gaaagcagat    1860
   ctccgagaag ctattcagac aatgctggat acaccaggac cttacctgtt ggatgtgatt    1920
   tgtccgcacc aagaacatgt gttgccgatg atcccgagtg gtggcacttt caacgatgtc    1980
   ataacggaag gagatggccg gattaaatac tga                                 2013
<210> 32
   <211> 1917
   <212> DNA
   <213> Zea mays
<220>
   <221> misc_feature
   <223> Maize acetohydroxyacid synthase large subunit 2 (ZmAHASL2) codi ng sequence, wild type allele.
<210> 33
   <211> 3234
   <212> DNA
   <213> Arabidopsis thaliana
<220>
   <221> misc_feature
   <223> Arabidopsis acetohydr oxyacid synthase large subunit (At AHASL) genomic fragment, S653N allele.
<400> 33
   atggcggcgg caacaacaac aacaacaaca tcttcttcga tctccttctc caccaaacca 60
   tctccttcct cctccaaatc accattacca atctccagat tctccctccc attctcccta 120
   aaccccaaca aatcatcctc ctcctcccgc cgccgcggta tcaaatccag ctctccctcc 180
   tccatctccg ccgtgctcaa cacaaccacc aatgtcacaa ccactccctc tccaaccaaa 240
   cctaccaaac ccgaaacatt catctcccga ttcgctccag atcaaccccg caaaggcgct 300
   gatatcctcg tcgaagcttt agaacgtcaa ggcgtagaaa ccgtattcgc ttaccctgga 360
   ggtgcatcaa tggagattca ccaagcctta acccgctctt cctcaatccg taacgtcctt 420
   cctcgtcacg aacaaggagg tgtattcgca gcagaaggat acgctcgatc ctcaggtaaa 480
   ccaggtatct gtatagccac ttcaggtccc ggagctacaa atctcgttag cggattagcc 540
   gatgcgttgt tagatagtgt tcctcttgta gcaatcacag gacaagtccc tcgtcgtatg 600
   attggtacag atgcgtttca agagactccg attgttgagg taacgcgttc gattacgaag 660
   cataactatc ttgtgatgga tgttgaagat atccctagga ttattgagga agctttcttt 720
   ttagctactt ctggtagacc tggacctgtt ttggttgatg ttcctaaaga tattcaacaa 780
   cagcttgcga ttcctaattg ggaacaggct atgagattac ctggttatat gtctaggatg 840
   cctaaacctc cggaagattc tcatttggag cagattgtta ggttgatttc tgagtctaag 900
   aagcctgtgt tgtatgttgg tggtggttgt ttgaattcta gcgatgaatt gggtaggttt 960
   gttgagctta cggggatccc tgttgcgagt acgttgatgg ggctgggatc ttatccttgt 1020
   gatgatgagt tgtcgttaca tatgcttgga atgcatggga ctgtgtatgc aaattacgct 1080
   gtggagcata gtgatttgtt gttggcgttt ggggtaaggt ttgatgatcg tgtcacgggt 1140
   aagcttgagg cttttgctag tagggctaag attgttcata ttgatattga ctcggctgag 1200
   attgggaaga ataagactcc tcatgtgtct gtgtgtggtg atgttaagct ggctttgcaa 1260
   gggatgaata aggttcttga gaaccgagcg gaggagctta agcttgattt tggagtttgg 1320
   aggaatgagt tgaacgtaca gaaacagaag tttccgttga gctttaagac gtttggggaa 1380
   gctattcctc cacagtatgc gattaaggtc cttgatgagt tgactgatgg aaaagccata 1440
   ataagtactg gtgtcgggca acatcaaatg tgggcggcgc agttctacaa ttacaagaaa 1500
   ccaaggcagt ggctatcatc aggaggcctt ggagctatgg gatttggact tcctgctgcg 1560
   attggagcgt ctgttgctaa ccctgatgcg atagttgtgg atattgacgg agatggaagc 1620
   tttataatga atgtgcaaga gctagccact attcgtgtag agaatcttcc agtgaaggta    1680
   cttttattaa acaaccagca tcttggcatg gttatgcaat gggaagatcg gttctacaaa    1740
   gctaaccgag ctcacacatt tctcggggat ccggctcagg aggacgagat attcccgaac    1800
   atgttgctgt ttgcagcagc ttgcgggatt ccagcggcga gggtgacaaa gaaagcagat    1860
   ctccgagaag ctattcagac aatgctggat acaccaggac cttacctgtt ggatgtgatt    1920
   tgtccgcacc aagaacatgt gttgccgatg atcccgaatg gtggcacttt caacgatgtc    1980
   ataacggaag gagatggccg gattaaatac tgagagatga aaccggtgat tatcagaacc    2040
   ttttatggtc tttgtatgca tatggtaaaa aaacttagtt tgcaatttcc tgtttgtttt    2100
   ggtaatttga gtttctttta gttgttgatc tgcctgcttt ttggtttacg tcagactact    2160
   actgctgttg ttgtttggtt tcctttcttt cattttataa ataaataatc cggttcggtt    2220
   tactccttgt gactggctca gtttggttat tgcgaaatgc gaatggtaaa ttgagtaatt    2280
   gaaattcgtt attagggttc taagctgttt taacagtcac tgggttaata tctctcgaat    2340
   cttgcatgga aaatgctctt accattggtt tttaattgaa atgtgctcat atgggccgtg    2400
   gtttccaaat taaataaaac tacgatgtca tcgagaagta aaatcaactg tgtccacatt    2460
   atcagttttg tgtatacgat gaaatagggt aattcaaaat ctagcttgat atgccttttg    2520
   gttcatttta accttctgta aacatttttt cagattttga acaagtaaat ccaaaaaaaa    2580
   aaaaaaaaaa tctcaactca acactaaatt attttaatgt ataaaagatg cttaaaacat    2640
   ttggcttaaa agaaagaagc taaaaacata gagaactctt gtaaattgaa gtatgaaaat    2700
   atactgaatt gggtattata tgaatttttc tgatttagga ttcacatgat ccaaaaagga    2760
   aatccagaag cactaatcag acattggaag taggaatatt tcaaaaagtt tttttttttt    2820
   aagtaagtga caaaagcttt taaaaaatag aaaagaaact agtattaaag ttgtaaattt    2880
   aataaacaaa agaaattttt tatatttttt catttctttt tccagcatga ggttatgatg    2940
   gcaggatgtg gatttcattt ttttcctttt gatagccttt taattgatct attataattg    3000
   acgaaaaaat attagttaat tatagatata ttttaggtag tattagcaat ttacacttcc    3060
   aaaagactat gtaagttgta aatatgatgc gttgatctct tcatcattca atggttagtc    3120
   aaaaaaataa aagcttaact agtaaactaa agtagtcaaa aattgtactt tagttttaaa    3180
   tattacatga ataatccaaa acgacattta tgtgaaacaa aaacaatatc taga          3234
<210> 34
   <211> 5717
   <212> DNA
   <213> Arabidopsis thaliana
<220>
   <221> misc_feature
   <223> Arabidopsis acetohydroxyacid synthase large subunit (At AHASL) genomic fragment, S653N allele.
<400> 34

## Description

### FIELD OF THE INVENTION

This invention relates generally to compositions and methods for increasing tolerance of plants to acetohydroxyacid synthase-inhibiting herbicides.

### BACKGROUND OF THE INVENTION

Acetohydroxyacid synthase (AHAS; EC 4.1.3.18, also known as acetolactate synthase or ALS), is the first enzyme that catalyzes the biochemical synthesis of the branched chain amino acids valine, leucine and isoleucine (Singh (1999) "Biosynthesis of valine, leucine and isoleucine," in Plant Amino Acids, Singh, B.K., ed., Marcel Dekker Inc. New York, New York, pp. 227-247). AHAS is the site of action of four structurally diverse herbicide families including the sulfonylureas (Tan et al. (2005) Pest Manag. Sci. 61:246-57; Mallory-Smith and Retzinger (2003) Weed Technology 17:620-626; LaRossa and Falco (1984) Trends Biotechnol. 2:158-161), the imidazolinones (Shaner et al. (1984) Plant Physiol. 76:545-546), the triazolopyrimidines (Subramanian and Gerwick (1989) "Inhibition of acetolactate synthase by triazolopyrimidines," in Biocatalysis in Agricultural Biotechnology, Whitaker, J.R. and Sonnet, P.E.. eds., ACS Symposium Series, American Chemical Society, Washington, D.C., pp. 277-288), and the pyrimidinyloxybenzoates (Subramanian et al. (1990) Plant Physiol. 94: 239-244). Imidazolinone and sulfonylurea herbicides are widely used in modern agriculture due to their effectiveness at very low application rates and relative non-toxicity in animals. By inhibiting AHAS activity, these families of herbicides prevent further growth and development of susceptible plants including many weed species. Several examples of commercially available imidazolinone herbicides are PURSUIT® (imazethapyr), SCEPTER® (imazaquin) and ARSENAL® (imazapyr). Examples of sulfonylurea herbicides are chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfuron, flazasulfuron, imazosulfuron, pyrazosulfuron ethyl and halosulfuron.

Due to their high effectiveness and low-toxicity, imidazolinone herbicides are favored for application by spraying over the top of a wide area of vegetation. The ability to spray a herbicide over the top of a wide range of vegetation decreases the costs associated with plant establishment and maintenance, and decreases the need for site preparation prior to use of such chemicals. Spraying over the top of a desired tolerant species also results in the ability to achieve maximum yield potential of the desired species due to the absence of competitive species. However, the ability to use such spray-over techniques is dependent upon the presence of imidazolinone-resistant species of the desired vegetation in the spray over area.

Among the major agricultural crops, some leguminous species such as soybean are naturally resistant to imidazolinone herbicides due to their ability to rapidly metabolize the herbicide compounds (Shaner and Robinson (1985) Weed Sci. 33:469-471). Other crops such as corn (Newhouse et al. (1992) Plant Physiol. 100:882-886) and rice (Barrett et al. (1989) Crop Safeners for Herbicides, Academic Press, New York, pp. 195-220) are somewhat susceptible to imidazolinone herbicides. The differential sensitivity to the imidazolinone herbicides is dependent on the chemical nature of the particular herbicide and differential metabolism of the compound from a toxic to a non-toxic form in each plant (Shaner et al. (1984) Plant Physiol. 76:545-546; Brown et al. (1987) Pestic. Biochem. Physiol. 27:24-29). Other plant physiological differences such as absorption and translocation also play an important role in sensitivity (Shaner and Robinson (1985) Weed Sci. 33:469-471).

Plants resistant to imidazolinones, sulfonylureas, triazolopyrimidines, and pyrimidinyloxybenzoates have been successfully produced using seed, microspore, pollen, and callus mutagenesis in *Zea mays, Arabidopsis thaliana, Brassica napus (i.e.,* canola) *Glycine max, Nicotiana tabacum,* sugarbeet (*Beta vulgaris*) and *Oryza sativa* (Sebastian et al. (1989) Crop Sci. 29:1403-1408; Swanson et al. 1989 Theor. Appl. Genet. 78:525-530; Newhouse et al. (1991) Theor. Appl. Genet. 83:65-70; Sathasivan et al. (1991) Plant Physiol. 97:1044-1050; Mourand et al. (1993) J. Heredity 84:91-96; Wright and Penner (1998) Theor. Appl. Genet. 96:612-620; U.S. Patent No. 5,545,822). In all cases, a single, partially dominant nuclear gene conferred resistance. Four imidazolinone resistant wheat plants were also previously isolated following seed mutagenesis of *Triticum aestivum* L. cv. Fidel (Newhouse et al. (1992) Plant Physiol. 100:882-886). Inheritance studies confirmed that a single, partially dominant gene conferred resistance. Based on allelic studies, the authors concluded that the mutations in the four identified lines were located at the same locus. One of the Fidel cultivar resistance genes was designated FS-4 (Newhouse et al. (1992) Plant Physiol. 100:882-886).

Computer-based modeling of the three dimensional conformation of the AHAS-inhibitor complex predicts several amino acids in the proposed inhibitor binding pocket as sites where induced mutations would likely confer selective resistance to imidazolinones (Ott et al. (1996) J. Mol. Biol. 263:359-368). Tobacco plants produced with some of these rationally designed mutations in the proposed binding sites of the AHAS enzyme have in fact exhibited specific resistance to a single class of herbicides (Ott et al. (1996) J. Mol. Biol. 263:359-368).

Plant resistance to imidazolinone herbicides has also been reported in a number of patents. U.S. Patent Nos. 4,761,373, 5,331,107, 5,304,732, 6,211,438, 6,211,439 and 6,222,100 generally describe the use of an altered AHAS gene to elicit herbicide resistance in plants, and specifically discloses certain imidazolinone resistant corn lines. U.S. Patent No. 5,013,659 discloses plants exhibiting herbicide resistance due to mutations in at least one amino acid in one or more conserved regions. The mutations described therein encode either cross-resistance for imidazolinones and sulfonylureas or sulfonylurea-specific resistance, but imidazolinone-specific resistance is not described. U.S. Patent No. 5,731,180 and U.S. Patent No. 5,767,361 discuss an isolated gene having a single amino acid substitution in a wild-type monocot AHAS amino acid sequence that results in imidazolinone-specific resistance. In addition, rice plants that are resistant to herbicides that interfere with AHAS have been developed by mutation breeding and tissue culture selection. *See*, U.S. Patent Nos. 5,545,822, 5,736,629, 5,773,703, 5,773,704, 5,952,553 and 6,274,796. Herbicide-resistant sunflower plants comprising mutant AHAS large subunit polypeptides are described in WO 2007/005581.

In plants, as in all other organisms examined, the AHAS enzyme is comprised of two subunits: a large subunit (catalytic role) and a small subunit (regulatory role) (Duggleby and Pang (2000) J. Biochem. Mol. Biol. 33:1-36). The AHAS large subunit (also referred to herein as AHASL) may be encoded by a single gene as in the case of *Arabidopsis*, and sugar beet or by multiple gene family members as in maize, canola, and cotton. Specific, single-nucleotide substitutions in the large subunit confer upon the enzyme a degree of insensitivity to one or more classes of herbicides (Chang and Duggleby (1998) Biochem J. 333:765-777).

For example, bread wheat, *Triticum aestivum* L., contains three homoeologous acetohydroxyacid synthase large subunit genes. Each of the genes exhibit significant expression based on herbicide response and biochemical data from mutants in each of the three genes (Ascenzi et al. (2003) International Society of Plant Molecular Biologists Congress, Barcelona, Spain, Ref. No. S10-17). The coding sequences of all three genes share extensive homology at the nucleotide level (WO 03/014357). Through sequencing the AHASL genes from several varieties of *Triticum aestivum*, the molecular basis of herbicide tolerance in most IMI-tolerant (imidazolinone-tolerant) lines was found to be the mutation S653(*At*)N, indicating a serine to asparagine substitution at a position equivalent to the serine at amino acid 653 in *Arabidopsis thaliana* (WO 03/014357). This mutation is due to a single nucleotide polymorphism (SNP) in the DNA sequence encoding the AHASL protein.

Multiple AHASL genes are also known to occur in dicotyledonous plants species. Recently, Kolkman et al. ((2004) Theor. Appl. Genet. 109: 1147-1159) reported the identification, cloning, and sequencing for three AHASL genes (AHASL1, AHASL2, and AHASL3) from herbicide-resistant and wild type genotypes of sunflower (*Helianthus annuus* L.). Kolkman *et al.* reported that the herbicide-resistance was due either to the Pro197Leu (using the *Arabidopsis* AHASL amino acid position nomenclature) substitution or the Ala205Val substitution in the AHASL1 protein and that each of these substitutions provided resistance to both imidazolinone and sulfonylurea herbicides.

A number of single mutations in the AHAS large subunit are known that result in tolerance or resistance to herbicides (Duggleby et al. (2000) Journal of Biochem and Mol. Bio. 33:1-36; Jander et al. (2003) Plant Physiology 131:139-146). For example, an alanine to valine substitution at position 122 of *Arabidopsis* AHASL (or an alanine to threonine substitution at corresponding position 100 of Cocklebur AHASL) confers resistance to imidazolinone and sulfonylureas. A methionine to glutamic acid or isoleucine substitution at position 124 of *Arabidopsis* AHASL confers resistance to imidazolinones and sulfonylureas. A proline to serine substitution at position 197 of *Arabidopsis* AHASL (or a proline to alanine, glutamic acid, leucine, glutamine, arginine, valine, tryptophan, or tyrosine substitution at corresponding position 192 of yeast AHASL) confers resistance to imidazolinones, sulfonylureas, and triazolopyrimidine. An arginine to alanine or glutamic acid substitution at position 199 of *Arabidopsis* AHASL confers imidazolinone resistance. An alanine to valine substitution at position 205 of *Arabidopsis* AHASL (or an alanine to cysteine, aspartic acid, glutamic acid, arginine, threonine, tryptophan or tyrosine substitution at corresponding position 200 of yeast AHASL) confers imidazolinones and sulfonylureas resistance. A substitution of almost any amino acid for the tryptophan at position 574 of *Arabidopsis* AHASL, corresponding to position 586 of yeast AHASL, confers resistance to imidazolinones, sulfonylureas, triazolopyrimidine, and pyrimidyl oxybenzoates. A serine to phenylalanine, asparagine, or threonine substitution at position 653 of *Arabidopsis* AHASL confers resistance to imidazolinones and pyrimidyl oxybenzoates.

U.S. Patent Nos. 5,853,973; 5,928,937; and 6,576,455 disclose structure-based modeling methods for making AHAS variants which include amino acid substitutions at specific positions that differ from the positions described above. In Mourad et al. (1992) Planta 188;491-497, it has shown that mutant lines resistant to sulfonylureas are cross-resistant to triazolopyrimidine, and mutant lines resistant to imidazolinones are cross-resistant to pyrimidyl oxybenzoates.

U.S. Patent No. 5,859,348 discloses a double mutant sugar beet AHAS large subunit having an alanine to threonine substitution at amino acid 113 and a proline to serine substitution at amino acid 188. Sugar beet plants containing the double mutant AHAS protein are described as being both imidazolinone and sulfonylurea resistant.

Mourad et al. (1994) Mol. Gen. Genet. 242:178-184, discloses an *Arabidopsis* AHAS double mutant designated *csr1-4*. The *csr1-4* mutant AHAS contained a C to T nucleotide substitution at position 589 (corresponding to a proline to serine substitution at amino acid 197 of *Arabidopsis* AHASL) and a G to A nucleotide substitution at position 1958 (corresponding to a serine to threonine substitution at amino acid 653 of *Arabidopsis* AHASL).

Lee et al. (1988) EMBO Journal 7:1241-1248, discloses a tobacco AHAS double mutant designated S4-Hra, which includes a Pro-Ala substitution at amino acid 196 (corresponding to the amino acid 197 of *Arabidopsis* AHASL) and a Trp-Leu substitution at amino acid 573 (corresponding to amino acid 574 of *Arabidopsis* AHASL). Transgenic lines carrying the double mutant gene show resistance to sulfonylurea herbicide.

U.S. Patent No. 7,119,256 discloses a double mutant rice AHAS large subunit having a tryptophan to leucine substitution at amino acid 548 and a serine to isoleucine substitution at amino acid 627. Transgenic rice plants expressing a polynucleotide encoding this double mutant AHAS protein were reported to have increased resistance to the pyrimidinyl carboxy herbicide, bispyribac-sodium.

Given their high effectiveness and low-toxicity, imidazolinone herbicides are favored for agricultural use. However, the ability to use imidazolinone herbicides in a particular crop production system depends upon the availability of imidazolinone-resistant varieties of the crop plant of interest. To produce such imidazolinone-resistant varieties, there remains a need for crop plants comprising mutant AHAS polypeptides which confer demonstrated improved tolerance to imidazolinones and/or other AHAS-inhibiting herbicides when compared to crop plants with existing AHAS mutants.

Although some AHAS mutants have been characterized, there remains a need for mutant AHAS polypeptides which confer, when expressed in a crop plant of interest, demonstrated improved herbicide tolerance to one or more classes of AHAS-inhibiting herbicides when compared to existing AHAS mutants in crop plants.

### SUMMARY OF THE INVENTION

This invention relates to new mutant AHAS polypeptides that demonstrate tolerance to a herbicide, in particular, an imidazolinone herbicide, or sulfonylurea herbicide, or a mixture thereof. In preferred embodiments, the herbicide tolerance conferred by the mutants of the invention is improved and/or enhanced relative to that obtained using known AHAS mutants. The mutants of the invention comprise at least two amino acid substitutions in the AHAS large subunit polypeptide, namely a valine, threonine, glutamine, cysteine or methionine substitution at a position corresponding to position 122 of SEQ ID NO:1 or position 90 of SEQ ID NO:2 and a glycine or tryptophan substitution at a position corresponding to position 653 of SEQ ID NO:1 or position 621 of SEQ ID NO:2.

In one embodiment, the invention provides an isolated polynucleotide encoding an AHAS large subunit double mutant polypeptide selected from the group consisting of a polypeptide having a valine, threonine, glutamine, cysteine, or methionine at a position corresponding to position 122 of SEQ ID NO:1 or position 90 of SEQ ID NO:2 and a glycine, or tryptophan at a position corresponding to position 653 of SEQ ID NO:1 or position 621 of SEQ ID NO:2.

In another embodiment, the invention provides an isolated polynucleotide encoding an AHAS large subunit triple mutant polypeptide selected from the group consisting of a polypeptide having a valine, threonine, glutamine, cysteine, or methionine at a position corresponding to position 122 of SEQ ID NO:1 or position 90 of SEQ ID NO:2, an alanine, glutamic acid, serine, phenylalanine, threonine, aspartic acid, cysteine, or asparagine at a position corresponding to position 199 of SEQ ID NO: 1 or position 167 of SEQ ID NO:2 and a glycine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a glycine, or tryptophan at a position corresponding to position 653 of SEQ ID NO:1 or position 621 of SEQ ID NO:2.

The invention also relates to AHASL polypeptides comprising the double and triple mutants described above, expression vectors comprising the polynucleotides encoding the AHASL double and triple mutants described above, cells comprising the polynucleotides encoding the AHASL double and triple mutants described above, transgenic plants comprising the polynucleotides and polypeptides described above and methods of making and using transgenic plants comprising the polynucleotides encoding the AHASL double and triple mutants described above.

The invention further relates to transgenic and non-transgenic plants comprising one or more polynucleotides comprising two or more mutations. In one embodiment, the plants of the invention comprise a first polynucleotide encoding a first AHASL single mutant polypeptide and a second polynucleotide encoding a second AHASL single mutant polypeptide, or an AHASL encoding polynucleotide comprising two mutations that result in the amino acid mutations of said first and second AHASL single mutant polypeptides, wherein said first and second AHASL single mutant polypeptides are selected from the group consisting of: a first polypeptide having a valine, threonine, glutamine, cysteine, or methionine at a position corresponding to position 122 of SEQ ID NO:1 or position 90 of SEQ ID NO:2 and a second polypeptide having a glycine, or tryptophan at a position corresponding to position 653 of SEQ ID NO:1 or position 621 of SEQ ID NO:2.

In another embodiment, the invention provides transgenic and non-transgenic plants comprising a first polynucleotide encoding a first AHASL single mutant polypeptide, a second polynucleotide encoding a second AHASL single mutant polypeptide, and a third polynucleotide encoding a third AHASL single mutant polypeptide; or an AHASL encoding polynucleotide comprising three mutations, wherein the three nucleotide mutations result in the amino acid mutations corresponding to the mutations of said first, second and third AHASL single mutant polypeptides; or an AHASL encoding polynucleotide comprising a single mutation and an AHASL encoding polynucleotide comprising a double mutations, wherein the nucleotide mutations result in the amino acid mutations corresponding to the amino acid mutations of said first, second and third AHASL single mutant polypeptides, wherein said first, second, and third AHASL single mutant polypeptides are selected from the group consisting of: a first polypeptide having a valine, threonine, glutamine, cysteine, or methionine at a position corresponding to position 122 of SEQ ID NO:1 or position 90 of SEQ ID NO:2, a second polypeptide having an alanine, glutamic acid, serine, phenylalanine, threonine, aspartic acid, cysteine, or asparagine at a position corresponding to position 199 of SEQ ID NO:1 or position 167 of SEQ ID NO:2 and a third polypeptide having a glycine, or tryptophan at a position corresponding to position 653 of SEQ ID NO:1 or position 621 of SEQ ID NO:2; a first polypeptide having a valine, threonine, glutamine, cysteine, or methionine at a position corresponding to position 122 of SEQ ID NO:1 or position 90 of SEQ ID NO:2, a second polypeptide having a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a third polypeptide having a glycine, or tryptophan at a position corresponding to position 653 of SEQ ID NO:1 or position 621 of SEQ ID NO:2.

The present invention provides the use of the transgenic and non-transgenic plants of the invention in a method for controlling weeds. Such plants comprise increased herbicide resistance relative to a wild-type plant. The method comprises applying an effective amount of an AHAS-inhibiting herbicide to the weeds and to the plant of the invention.

### BRIEF DESCRIPTION OF THE DRAWINGS

Figure 1 sets forth the full length sequence of the *Arabidopsis* AHAS large subunit protein (amino acid sequence SEQ ID NO:1; nucleic acid sequence SEQ ID NO:31) with putative translation showing positions of mutations indicated in bold and underlined. DNA numbering is on the left and amino acid numbering on the right.
Figure 2 sets forth the sequence of the maize AHAS large subunit protein (amino acid sequence SEQ ID NO:2; nucleic acid sequence SEQ ID NO:32) with amino acids at positions of claimed mutations indicated in bold and underlined. DNA numbering is on the left and amino acid numbering on the right.

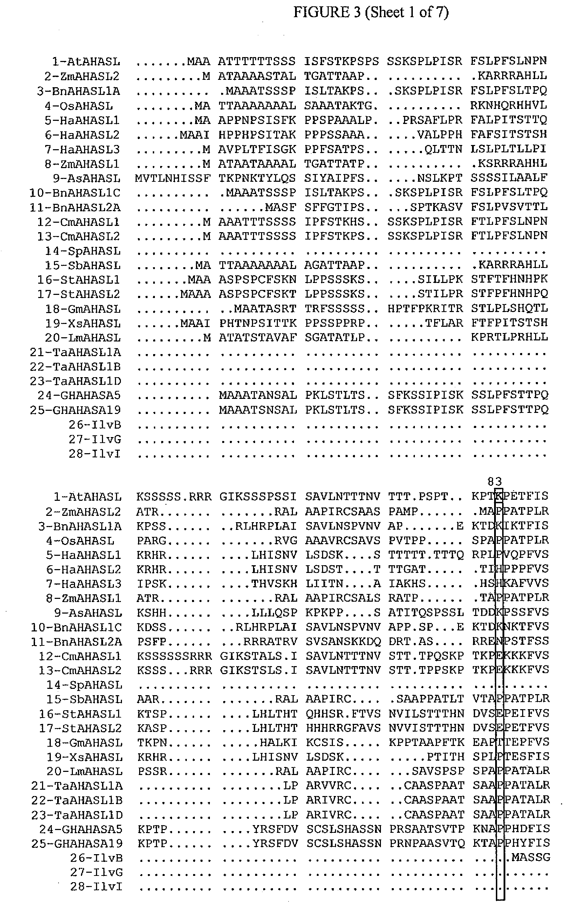

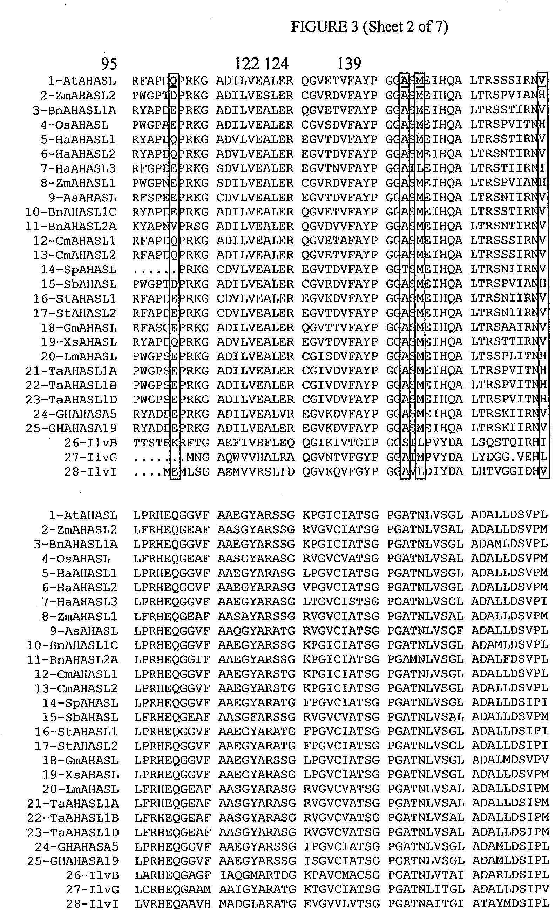

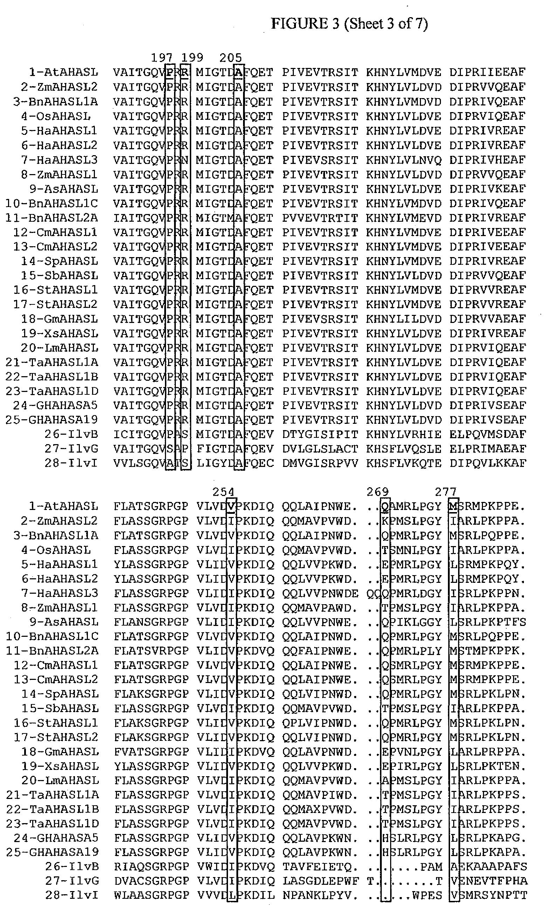

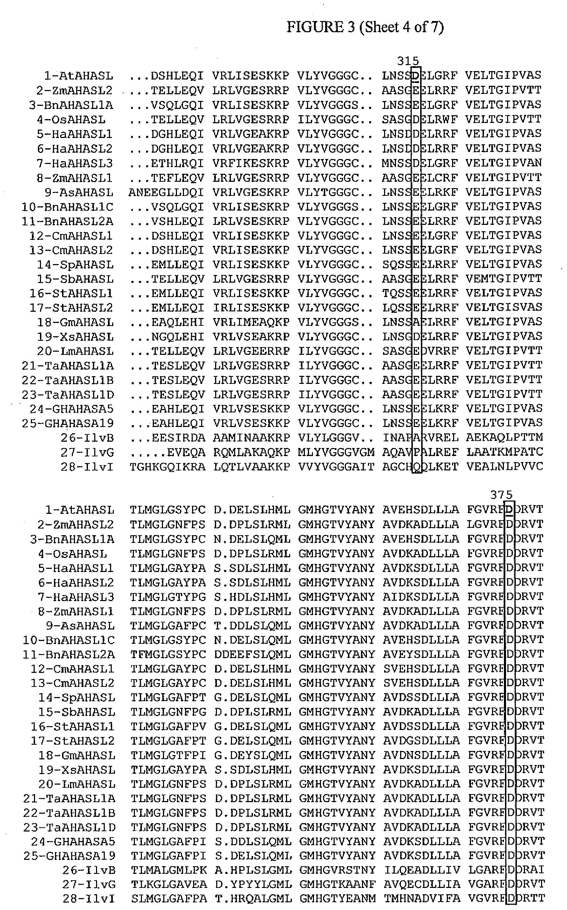

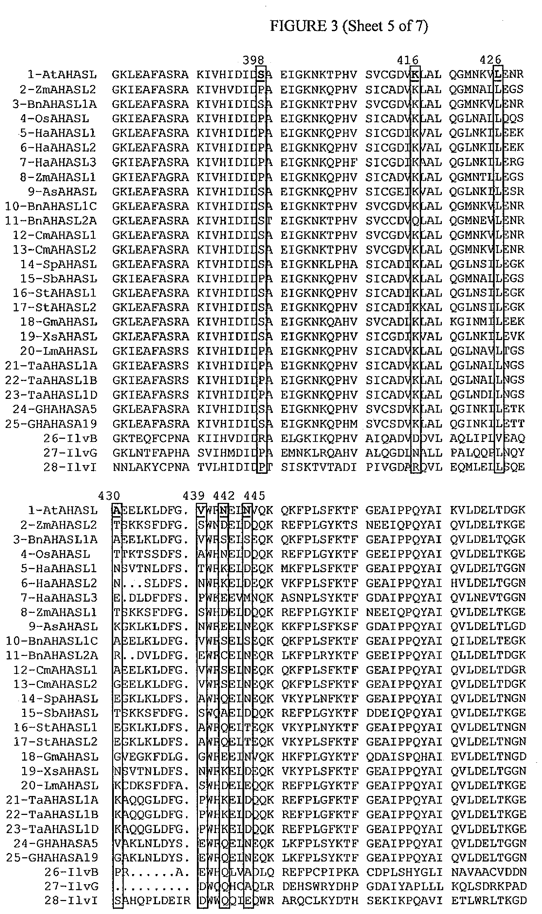

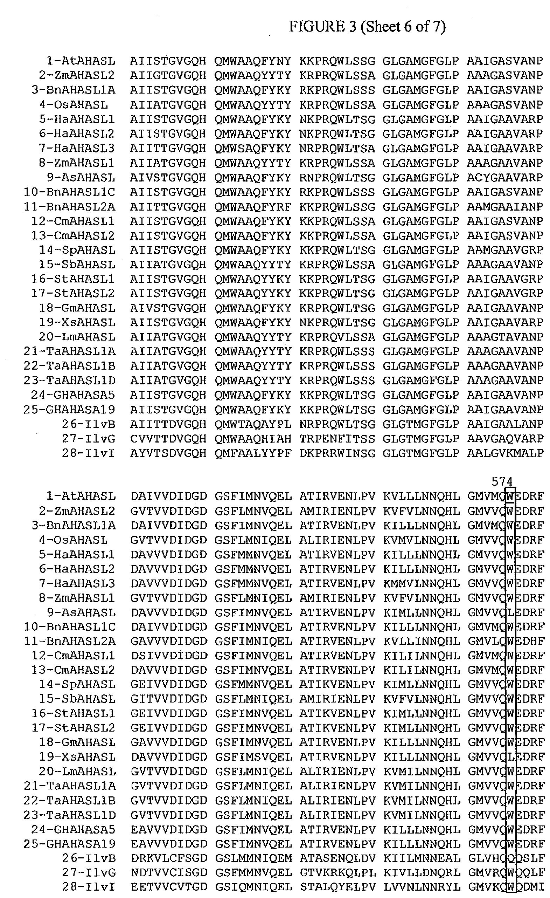

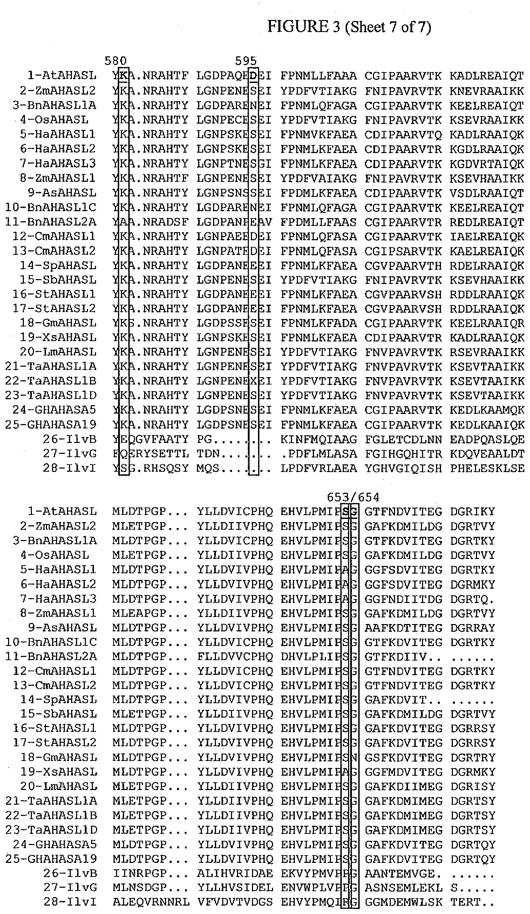

Figure 3 is an alignment of the positions of correspondence of the *Arabidopsis* AHAS large subunit protein (AtAHASL, SEQ ID NO:1) with the AHAS large subunit protein of a number of species where the double and triple mutations of the invention may be made showing the position of substitutions which correspond to the positions of substitution in SEQ ID NO:1: *Amaranthus* sp. (AsAHASL SEQ ID NO:9), *Brassica napus* (BnAHASL1A SEQ ID NO:3, BnAHASL1C SEQ ID NO:10, BnAHASL2A SEQ ID NO:11), *Camelina microcarpa* (CmAHASL1 SEQ ID NO:12, CmAHASL2 SEQ ID NO:13), *Solanum tuberosum* (StAHASL1 SEQ ID NO:16, StAHASL2 SEQ ID NO:17), *Oryza sativa* (OsAHASL SEQ ID NO:4), *Lolium multiflorum* (LmAHASL SEQ ID NO:20), *Solanum ptychanthum* (SpAHASL SEQ ID NO:14), *Sorghum bicolor* (SbAHASL SEQ ID NO:15), *Glycine max* (GmAHASL SEQ ID NO:18), *Helianthus annuus* (HaAHASL1 SEQ ID NO:5, HaAHASL2 SEQ ID NO:6, HaAHASL3 SEQ ID NO:7), Triticum aestivum (TaAHASL1A SEQ ID NO:21, TaAHASL1B SEQ ID NO:22, TaAHASL1D SEQ ID NO:23), *Xanthium* sp. (XsAHASL SEQ ID NO:19), *Zea mays* (ZmAHASL1 SEQ ID NO:8, ZmAHASL2 SEQ ID NO:2), *Gossypium hirsutum* (GhAHASA5 SEQ ID NO:24, GhAHASA19 SEQ ID NO:25), and *E*.*coli* (ilvB SEQ ID NO:26, ilvG SEQ ID NO:27, ilvI SEQ ID NO:28).
Figure 4 is a map of the AE base vector used for construction of *Arabidopsis* AHASL mutants AE2-AE8 in *E. coli,* with relative positions of mutations in *Arabidopsis* AHASL indicated.
Figure 5 is a vector map of plant transformation base vector AP used for construction of vectors AP2-AP5, which differ only by the mutations indicated in Table 1.

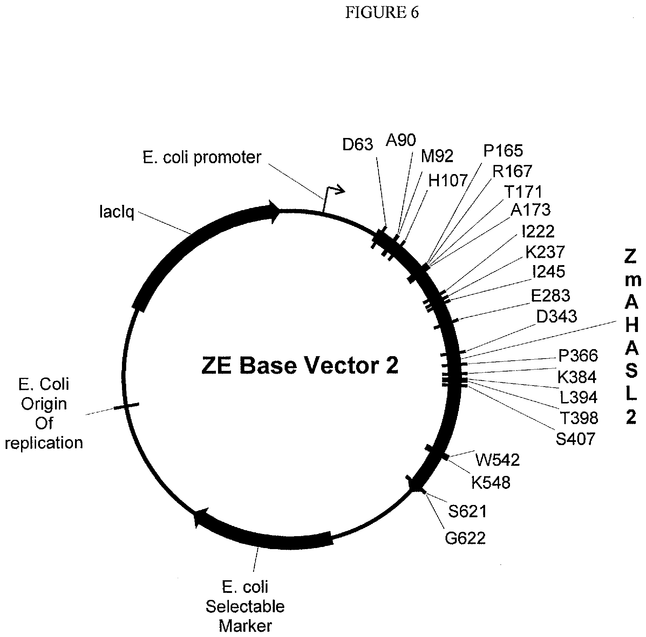

Figure 6 is a map of base vector ZE used to study maize AHASL mutants ZE2, ZE5, ZE6, and ZE7 in *E. coli,* with relative positions of mutations indicated.
Figure 7 is a map of plant transformation vector ZP used as a base vector for construction of vectors ZP2-ZP10.
Figure 8 is a table showing the concordant amino acid positions of AHASL genes derived from different species.

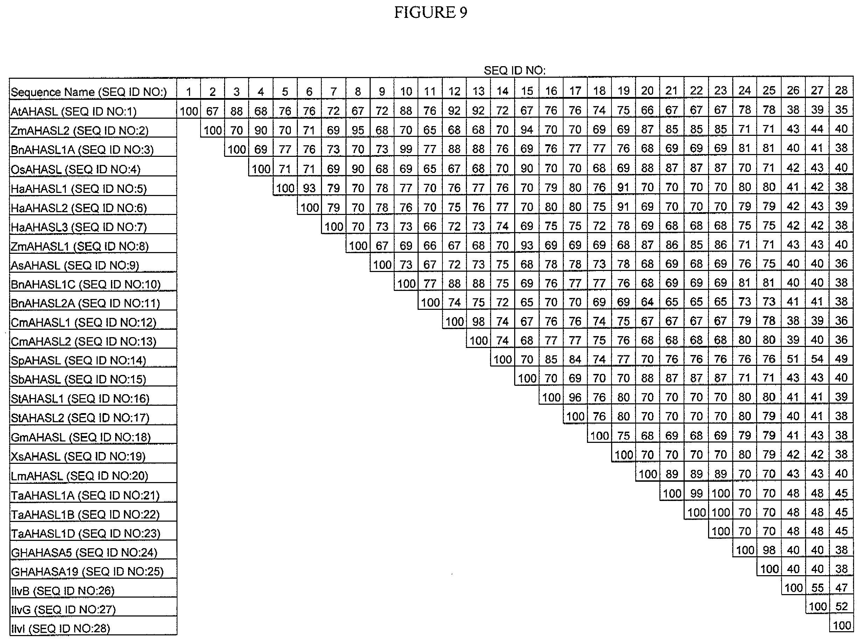

Figure 9 is a table showing the protein identity percentage of the AHASL genes derived from different species. The analysis was performed in Vector NTI software suite (gap opening penalty = 10, gap extension penalty = 0.05, gap separation penalty = 8, blosum 62MT2 matrix).
Figure 10 sets forth the results of a vertical plate growth assay of seeds from several lines of *Arabidopsis* plated on media with 37.5 micromolar of imazethapyr. The seeds used were: 1) wild type ecotype Columbia 2; 2) the csrl-2 mutant (homozygous for the AtAHASL S653N mutation in the genomic copy of the AHAS large subunit gene); 3) Columbia 2 transformed with AP1; 4) Columbia 2 transformed with AP7; and 5) Columbia 2 transformed with AP2.
Figure 11 is a vector map of plant transformation base vector AUP used for construction of vectors AUP2 and AUP, which differ only by the mutations indicated in Table 3.
Figure 12 is a vector map of plant transformation vector BAP1, which comprises the coding sequence for an AtAHASL with the S653N mutation.

### DETAILED DESCRIPTION OF THE INVENTION

The invention provides polynucleotides encoding AHASL with at least two mutations, for example double and triple mutants, that demonstrate tolerance to herbicides, in particular, to imidazolinone herbicides and optionally, to sulfonylurea, triazolopyrimidine sulfoanilide, and/or pyrimidyl oxybenzoate herbicides. The AHASL mutants of the invention may be used to create transgenic plants that demonstrate levels of herbicide resistance sufficient to confer commercial levels of herbicide tolerance when present on only one parent of a hybrid cross or on one genome of a polyploid plant. The polynucleotides of the invention may also be used as selectable markers for transformation of linked genes encoding other traits, as set forth in U.S. Patent No. 6,025,541.

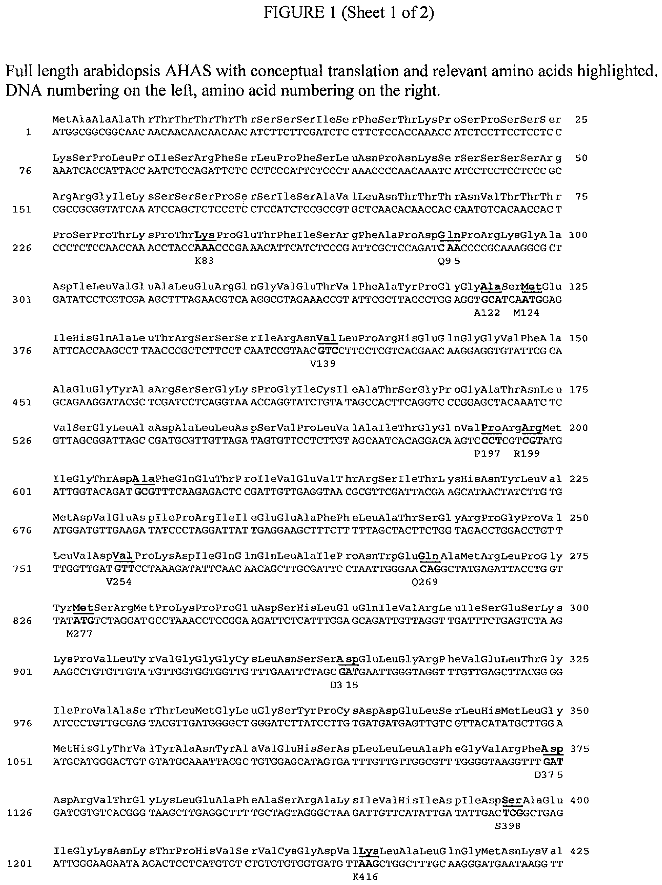

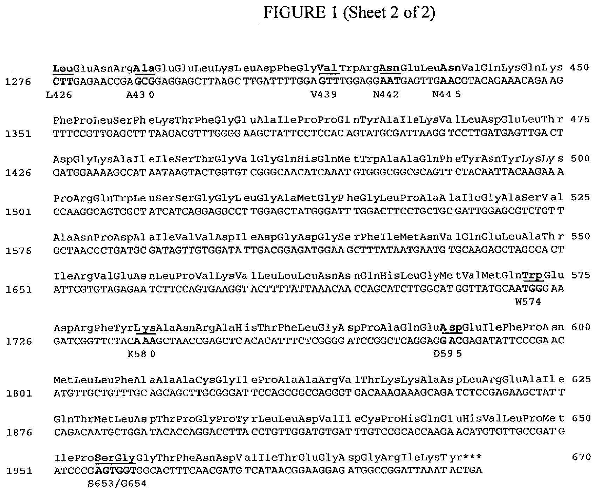

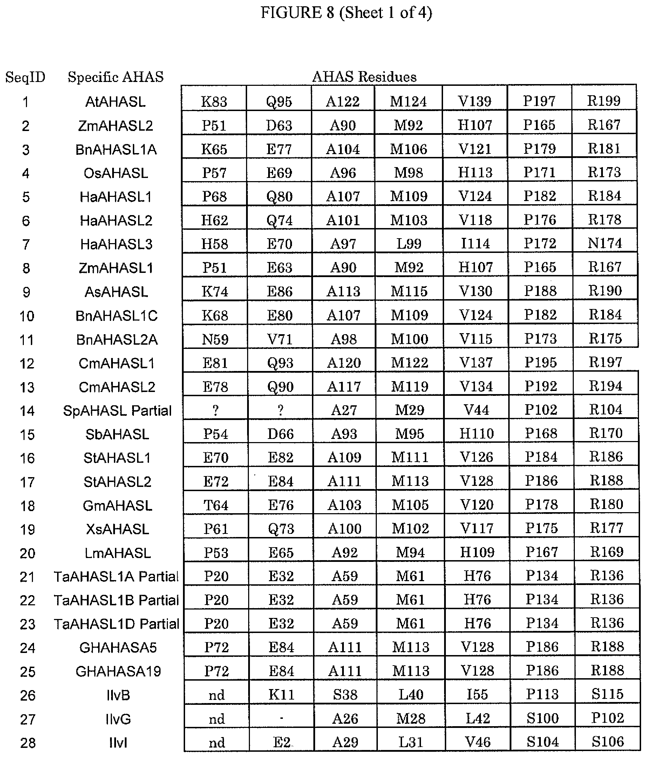

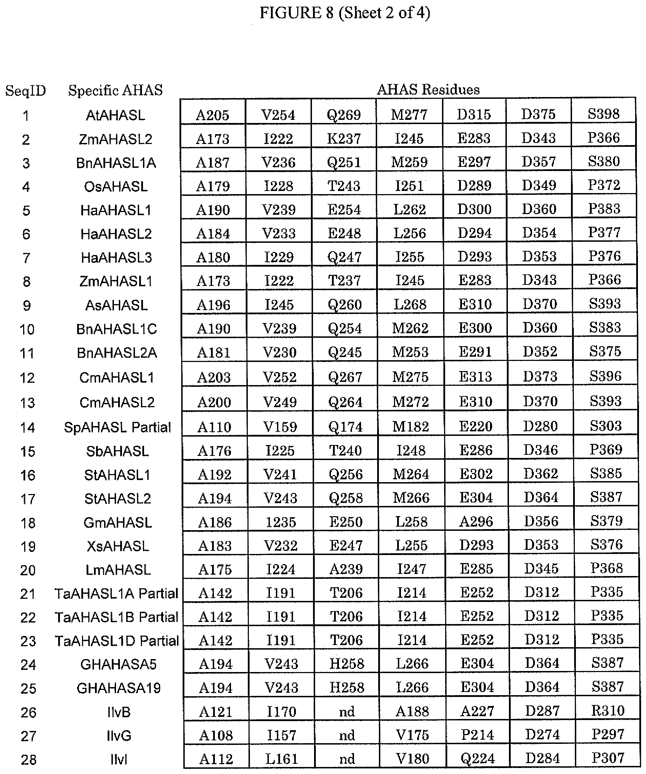

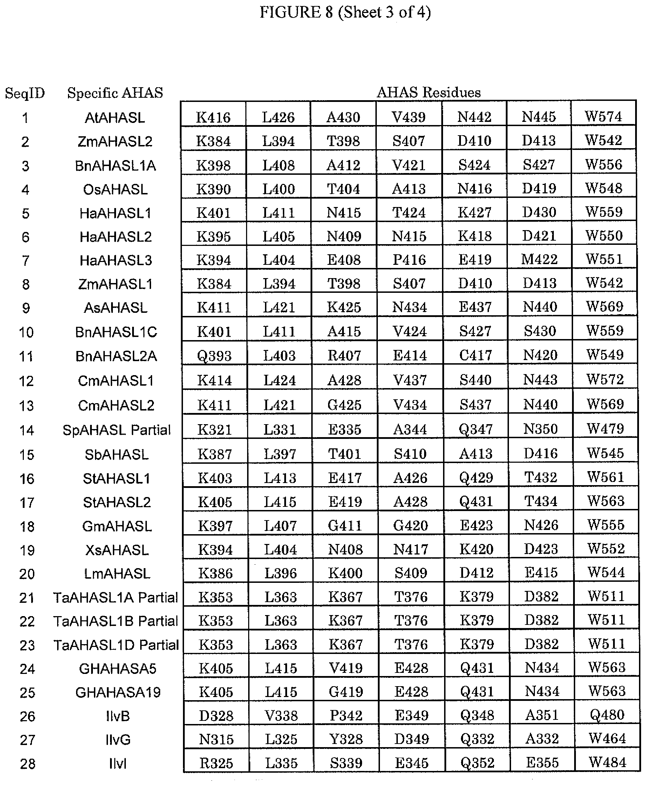

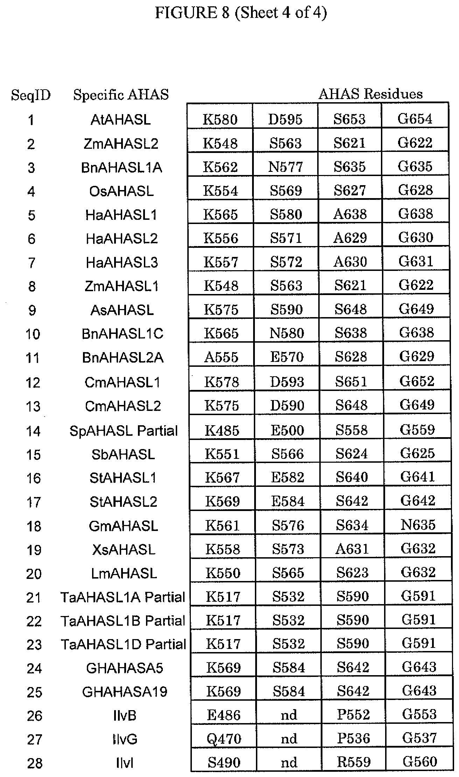

Although the AHASL proteins of various species differ in length by a few amino acids, the relative positions of residues subject to modification in accordance with the present invention are conserved (Figure 8). Accordingly, the mutations described herein are expressed in terms of positions corresponding to the amino acid residue numbers of the *Arabidopsis* AHASL polypeptide (SEQ ID NO:1, Figure 1, Figure 8) unless noted otherwise or apparent from the context. For example, residue 122 of the *Arabidopsis* AHASL corresponds to residue 90 of maize AHASL, residue 104 of *Brassica napus* AHASL 1A, residue 107 of *B. napus* AHASL 1C, residue 96 of *O*. *sativa* AHASL, residue 113 of *Amaranthus* AHASL, residue 26 of *Escherichia coli* ilvG, residue 117 of *Saccharomyces cerevisiae* AHASL, residue 113 of sugar beet, residue 111 of cotton, residue 120 of *Camelina microcarpa* AHASL 1, residue 117 of *Camelina microcarpa* AHASL2, residue 109 of *Solanum tuberosum* AHASL1, residue 111 of *Solanum tuberosum* AHASL2, residue 92 of *Lolium multiflorum,* residue 27 of *Solanum ptychanthum,* residue 93 of *Sorghum bicolor*, residue 103 of *Glycine max,* residue 107 of *Helianthus annuus* AHASL1, residue 101 of *Helianthus annuus* AHASL2, residue 97 of *Helianthus annuus* AHASL3, residue 59 of *Triticum aestivum*, and residue 100 of *Xanthium* sp. These correspondences are well known to those of skill in the art. Based on such correspondence, the corresponding positions in AHAS large subunit sequences not specifically disclosed herein could be readily determined by the skilled artisan. Specific exemplary regions of correspondence relevant to the present invention are set forth in Figure 3.

In a preferred embodiment, the invention provides an isolated polynucleotide encoding an *Arabidopsis* AHASL double mutant selected from the group consisting of a polypeptide having a valine, threonine, glutamine, cysteine, or methionine at a position corresponding to position 122 of SEQ ID NO:1 or position 90 of SEQ ID NO:2 and a glycine, or tryptophan at a position corresponding to position 653 of SEQ ID NO:1 or position 621 of SEQ ID NO:2.

In another preferred embodiment, the invention provides an isolated polynucleotide encoding an *Arabidopsis* AHAS large subunit triple mutant polypeptide selected from the group consisting of: a polypeptide having a valine, threonine, glutamine, cysteine, or methionine at a position corresponding to position 122 of SEQ ID NO:1 or position 90 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and a glycine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a glycine, or tryptophan at a position corresponding to position 653 of SEQ ID NO:1 or position 621 of SEQ ID NO:2.

Other preferred embodiments include AHASL double and triple mutants from other species, wherein the double and triple mutations occur at positions corresponding to those of the specific *Arabidopsis* and maize mutants described above and in table shown in Figure 8. For example, corresponding double and triple mutants of AHASL from microorganisms such as *E. coli, S. cerevisiae, Salmonella, Synechocystis;* and from plants such as wheat, rye, oat, triticale, rice, barley, sorghum, millet, sugar beet, sugarcane, soybean, peanut, cotton, rapeseed, canola, *Brassica* species, manihot, melon, squash, pepper, sunflower, tagetes, solanaceous plants, potato, sweet potato, tobacco, eggplant, tomato, *Vicia* species, pea, alfalfa, coffee, cacao are also within the scope of the present invention. Such double and triple mutants can be made using known methods, for example, *in vitro* using site-directed mutagenesis, or *in vivo* using targeted mutagenesis or similar techniques, as described in U.S. Patent Nos. 5,565,350; 5,731,181; 5756,325; 5,760,012; 5,795,972 and 5,871,984.

The polynucleotides of the invention are provided in expression cassettes for expression in the plant of interest. The cassette will include regulatory sequences operably linked to an AHASL polynucleotide sequence of the invention. The term "regulatory element" as used herein refers to a polynucleotide that is capable of regulating the transcription of an operably linked polynucleotide. It includes, but not limited to, promoters, enhancers, introns, 5' UTRs, and 3' UTRs. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

Such an expression cassette is provided with a plurality of restriction sites for insertion of the AHASL polynucleotide sequence to be under the transcriptional regulation of the regulatory regions. The expression cassette may additionally contain selectable marker genes.

The expression cassette will include in the 5'-3' direction of transcription, a transcriptional and translational initiation region (i.e., a promoter), an AHASL polynucleotide sequence of the invention, and a transcriptional and translational termination region (i.e., termination region) functional in plants. The promoter may be native or analogous, or foreign or heterologous, to the plant host and/or to the AHASL polynucleotide sequence of the invention. Additionally, the promoter may be the natural sequence or alternatively a synthetic sequence. Where the promoter is "foreign" or "heterologous" to the plant host, it is intended that the promoter is not found in the native plant into which the promoter is introduced. Where the promoter is "foreign" or "heterologous" to the AHASL polynucleotide sequence of the invention, it is intended that the promoter is not the native or naturally occurring promoter for the operably linked AHASL polynucleotide sequence of the invention. As used herein, a chimeric gene comprises a coding sequence operably linked to a transcription initiation region that is heterologous to the coding sequence.

While it may be preferable to express the AHASL polynucleotides of the invention using heterologous promoters, the native promoter sequences may be used. Such constructs would change expression levels of the AHASL protein in the plant or plant cell. Thus, the phenotype of the plant or plant cell is altered.

The termination region may be native with the transcriptional initiation region, may be native with the operably linked AHASL sequence of interest, may be native with the plant host, or may be derived from another source (i.e., foreign or heterologous to the promoter, the AHASL polynucleotide sequence of interest, the plant host, or any combination thereof). Convenient termination regions are available from the Ti-plasmid of *A. tumefaciens*, such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed plant. That is, the genes can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

Nucleotide sequences for enhancing gene expression can also be used in the plant expression vectors. These include the introns of the maize AdhI, intronl gene (Callis et al. Genes and Development 1:1183-1200, 1987), and leader sequences, (W-sequence) from the Tobacco Mosaic virus (TMV), Maize Chlorotic Mottle Virus and Alfalfa Mosaic Virus (Gallie et al. Nucleic Acid Res. 15:8693-8711, 1987 and Skuzeski et al. Plant Mol. Biol. 15:65-79, 1990). The first intron from the shrunken-1 locus of maize, has been shown to increase expression of genes in chimeric gene constructs. U.S. Pat. Nos. 5,424,412 and 5,593,874 disclose the use of specific introns in gene expression constructs, and Gallie et al. (Plant Physiol. 106:929-939, 1994) also have shown that introns are useful for regulating gene expression on a tissue specific basis. To further enhance or to optimize AHAS large subunit gene expression, the plant expression vectors of the invention may also contain DNA sequences containing matrix attachment regions (MARs). Plant cells transformed with such modified expression systems, then, may exhibit overexpression or constitutive expression of a nucleotide sequence of the invention.

The expression cassettes may additionally contain 5' leader sequences in the expression cassette construct. Such leader sequences can act to enhance translation. Translation leaders are known in the art and include: picornavirus leaders, for example, EMCV leader (Encephalomyocarditis 5' noncoding region) (Elroy-Stein et al. (1989) Proc. Natl. Acad. Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus) (Virology 154:9-20), and human immunoglobulin heavy-chain binding protein (BiP) (Macejak et al. (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol. 84:965-968. Other methods known to enhance translation can also be utilized, for example, introns, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and trans versions, may be involved.

A number of promoters can be used in the practice of the invention. The promoters can be selected based on the desired outcome. The nucleic acids can be combined with constitutive, tissue-preferred, or other promoters for expression in plants.

Such constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

Tissue-preferred promoters can be utilized to target enhanced AHASL expression within a particular plant tissue. Such tissue-preferred promoters include, but are not limited to, leaf-preferred promoters, root-preferred promoters, seed-preferred promoters, and stem-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3): 1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression.

In one embodiment, the nucleic acids of interest are targeted to the chloroplast for expression. In this manner, where the nucleic acid of interest is not directly inserted into the chloroplast, the expression cassette will additionally contain a chloroplast-targeting sequence comprising a nucleotide sequence that encodes a chloroplast transit peptide to direct the gene product of interest to the chloroplasts. Such transit peptides are known in the art. With respect to chloroplast-targeting sequences, "operably linked" means that the nucleic acid sequence encoding a transit peptide (*i.e.,* the chloroplast-targeting sequence) is linked to the AHASL polynucleotide of the invention such that the two sequences are contiguous and in the same reading frame. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481. While the AHASL proteins of the invention include a native chloroplast transit peptide, any chloroplast transit peptide known in the art can be fused to the amino acid sequence of a mature AHASL protein of the invention by operably linking a chloroplast-targeting sequence to the 5'-end of a nucleotide sequence encoding a mature AHASL protein of the invention.

Chloroplast targeting sequences are known in the art and include the chloroplast small subunit of ribulose-1,5-bisphosphate carboxylase (Rubisco) (de Castro Silva Filho et al. (1996) Plant Mol. Biol. 30:769-780; Schnell et al. (1991) J. Biol. Chem. 266(5):3335-3342); 5- (enolpyruvyl)shikimate-3-phosphate synthase (EPSPS) (Archer et al. (1990) J. Bioenerg. Biomemb. 22(6):789-810); tryptophan synthase (Zhao et al. (1995) J. Biol. Chem. 270(11):6081- 6087); plastocyanin (Lawrence et al. (1997) J. Biol. Chem. 272(33):20357-20363); chorismate synthase (Schmidt et al. (1993) J. Biol. Chem. 268(36):27447-27457); and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

In particular, the present invention describes using polynucleotides encoding AHASL mutant polypeptides comprising at least two mutations to engineer plants which are herbicide tolerant. This strategy has herein been demonstrated using *Arabidopsis* AHASL mutants in *Arabidopsis thaliana* and maize AHASL2 mutants in corn, but its application is not restricted to these genes or to these plants. In preferred embodiments, the herbicide is imidazolinone and/or sulfonylurea. In other preferred embodiments, the herbicide tolerance is improved and/or enhanced compared to wild-type plants and to known AHAS mutants.

The invention also provides a method of producing a transgenic crop plant containing AHASL mutant coding nucleic acid comprising at least two mutations, wherein expression of the nucleic acid(s) in the plant results in herbicide tolerance as compared to wild-type plants or to known AHAS mutant type plants comprising: (a) introducing into a plant cell an expression vector comprising nucleic acid encoding an AHASL mutant with at least two mutations, and (b) generating from the plant cell a transgenic plant which is herbicide tolerant. The plant cell includes, but is not limited to, a protoplast, gamete producing cell, and a cell that regenerates into a whole plant. As used herein, the term "transgenic" refers to any plant, plant cell, callus, plant tissue, or plant part that contains all or part of at least one recombinant polynucleotide. In many cases, all or part of the recombinant polynucleotide is stably integrated into a chromosome or stable extra-chromosomal element, so that it is passed on to successive generations.

In another embodiment, the invention relates to using the mutant AHASL polypeptides of the invention as selectable markers. The invention provides a method of identifying or selecting a transformed plant cell, plant tissue, plant or part thereof comprising a) providing a transformed plant cell, plant tissue, plant or part thereof, wherein said transformed plant cell, plant tissue, plant or part thereof comprises an isolated nucleic acid encoding an AHAS large subunit double mutant polypeptide of the invention as described above, wherein the polypeptide is used as a selection marker, and wherein said transformed plant cell, plant tissue, plant or part thereof may optionally comprise a further isolated nucleic acid of interest; b) contacting the transformed plant cell, plant tissue, plant or part thereof with at least one AHAS inhibitor or AHAS inhibiting compound; c) determining whether the plant cell, plant tissue, plant or part thereof is affected by the inhibitor or inhibiting compound; and d) identifying or selecting the transformed plant cell, plant tissue, plant or part thereof.

The invention is also embodied in purified AHASL proteins that contain the double and triple mutations described herein, which are useful in molecular modeling studies to design further improvements to herbicide tolerance. Methods of protein purification are well known, and can be readily accomplished using commercially available products or specially designed methods, as set forth for example, in Protein Biotechnology, Walsh and Headon (Wiley, 1994).

The invention further provides non-transgenic and transgenic herbicide-tolerant plants comprising one polynucleotide encoding an AHASL double mutant polypeptide, or two polynucleotides encoding AHASL single mutant polypeptides. Non-transgenic plants generated therefrom can be produced by cross-pollinating a first plant with a second plant and allowing the pollen acceptor plant (can be either the first or second plant) to produce seed from this cross pollination. Seeds and progeny plants generated thereof can have the double mutations crossed onto one single allele or two alleles. The pollen-acceptor plant can be either the first or second plant. The first plant comprises a first polynucleotide encoding a first AHASL single mutant polypeptide. The second plant comprises a second polynucleotide encoding a second AHASL single mutant polypeptide. The first and second AHASL single mutant polypeptides comprise a different single amino acid substitution relative to a wild-type AHASL polypeptide. Seeds or progeny plants arising therefrom which comprise one polynucleotide encoding the AHASL double mutant polypeptide or two polynucleotides encoding the two AHASL single mutant polypeptides can be selected. The selected progeny plants display an unexpectedly higher level of tolerance to an AHAS-inhibiting herbicide, for example an imidazolinone herbicide or sulfonylurea herbicide, than is predicted from the combination of the two AHASL single mutant polypeptides in a single plant. The progeny plants display a synergy with respect to herbicide tolerance, whereby the level of herbicide tolerance in the progeny plants comprising the first and second mutations from the parent plants is greater than the herbicide tolerance of a plant comprising two copies of the first polynucleotide or two copies of the second polynucleotide.

When the first and second plants are homozygous for the first and second polynucleotides, respectively, each of the resulting progeny plants comprises one copy of each of the first and second polynucleotides and the selection step can be omitted. When at least one of the first and second plants is heterozygous, progeny plants comprising both polynucleotides can be selected, for example, by analyzing the DNA of progeny plants to identify progeny plants comprising both the first and second polynucleotides or by testing the progeny plants for increased herbicide tolerance. The progeny plants that comprise both the first and second polynucleotides display a level of herbicide tolerance that is greater than the herbicide tolerance of a plant comprising two copies of the first polypeptide or two copies of the second polypeptide.

In one embodiment, the plants of the invention comprise a first polynucleotide encoding a first AHASL single mutant polypeptide and a second polynucleotide encoding a second AHASL single mutant polypeptide, or an AHASL encoding polynucleotide comprising two nucleotide mutations that result in the amino acid mutations corresponding to the amino acid mutations of said first and said second AHASL single mutant polypeptides, wherein said first and said second AHASL single mutant polypeptides are selected from the group consisting of: a first polypeptide having a valine, threonine, glutamine, cysteine, or methionine at a position corresponding to position 122 of SEQ ID NO:1 or position 90 of SEQ ID NO:2 and a second polypeptide having a glycine, or tryptophan at a position corresponding to position 653 of SEQ ID NO:1 or position 621 of SEQ ID NO:2. Non-transgenic plants comprising the double mutations of AHASL polynucleotides can be produced by methods other than the cross pollination described above, such as, for example but not limited to, targeted *in vivo* mutagenesis as described in Kochevenko et al. (Plant Phys. 132:174-184, 2003). The double mutations can be localized on a single allele, or two alleles of a plant genome.

Another embodiment of the invention relates to a transgenic plant transformed with an expression vector comprising an isolated polynucleotide, wherein the isolated polynucleotide encodes an acetohydroxyacid synthase large subunit (AHASL) double mutant polypeptide selected from the group consisting of: a polypeptide having a valine, threonine, glutamine, cysteine, or methionine at a position corresponding to position 122 of SEQ ID NO:1 or position 90 of SEQ ID NO:2 and a glycine, or tryptophan at a position corresponding to position 653 of SEQ ID NO:1 or position 621 of SEQ ID NO:2.

The invention further provides non-transgenic and transgenic herbicide-tolerant plants comprising one polynucleotide encoding an AHASL triple mutant polypeptide, or one or more AHASL encoding polynucleotides comprising three mutations. For the production of a nontransgenic plant with one or more polynucleotides comprising three mutations, a progeny plant comprising one or two polynucleotides comprising said first and said second mutations described above is cross pollinated with third plant that comprises a third polynucleotide encoding a third AHASL single mutant polypeptide. The third AHASL single mutant polypeptide comprises a different single amino acid substitution relative to a wild-type AHASL polypeptide than either the first or second AHASL single mutant polypeptides. Seeds or progeny plants that comprise one or more polynucleotides comprising the three mutations are selected as described above. The selected progeny plants comprise a level of herbicide tolerance that is greater than the additive effect of combining the three AHASL single mutant polypeptides in a single plant. Nontransgenic plants comprising the triple or multiple mutations of AHASL polynucleotides can be produced by methods other than the cross pollination described above, such as, for example but not limited to, targeted *in vivo* mutagenesis as described above. The multiple mutations can be localized on a single allele, or multiple alleles of a plant genome.

In one embodiment, plants of the invention comprise a first polynucleotide encoding a first AHASL single mutant polypeptide, a second polynucleotide encoding a second AHASL single mutant polypeptide, and a third polynucleotide encoding a third AHASL single mutant polypeptide. In another embodiment, plants of the invention comprise an AHASL encoding polynucleotide comprising three mutations, wherein the three nucleotide mutations result in the amino acid mutations corresponding to the mutations of said first, said second and said third AHASL single mutant polypeptides. In yet another embodiment, plants of the invention comprise an AHASL encoding polynucleotide comprising a single mutation and a polynucleotide comprising a double mutations, wherein the nucleotide mutations result in the amino acid mutations corresponding to the mutations of aforementioned first, second and third AHASL single mutant polypeptides, wherein said first, second, and third AHASL single mutant polypeptides are selected from the group consisting of: a polypeptide having a valine, threonine, glutamine, cysteine, or methionine at a position corresponding to position 122 of SEQ ID NO:1 or position 90 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and a glycine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 or position 165 of SEQ ID NO:2 and a glycine, or tryptophan at a position corresponding to position 653 of SEQ ID NO:1 or position 621 of SEQ ID NO:2.

Alternatively, plants comprising one or more polynucleotides encoding AHASL single mutant polypeptides are produced by transforming a plant with two or more of such polynucleotides or transforming a first plant with a first polynucleotide encoding a first AHASL single mutant polypeptide and cross pollinating the first plant with a second plant comprising a second polynucleotide encoding a second AHASL single mutant polypeptide. The second plant comprises a second polynucleotide comprising second AHASL single mutant polypeptide that is endogenous or was introduced via transformation. The first and second AHASL single mutant polypeptides comprise a different single amino acid substitution relative to a wild-type AHASL polypeptide. As necessary, seeds or progeny plants comprising both the first and second polynucleotides are selected as described above.

Yet another embodiment of the invention relates to a transgenic plant transformed with an expression vector comprising an isolated polynucleotide, wherein the isolated polynucleotide encodes an acetohydroxyacid synthase large subunit (AHASL) triple mutant polypeptide selected from the group consisting of: a polypeptide having a valine, threonine, glutamine, cysteine, or methionine at a position corresponding to position 122 of SEQ ID NO:1 1 or position 90 of SEQ ID NO:2, an alanine, glutamic acid, serine, phenylalanine, threonine, aspartic acid, cysteine, or asparagine at a position corresponding to position 199 of SEQ ID NO:1 or position 167 of SEQ ID NO:2 and a glycine, or tryptophan at a position corresponding to position 653 of SEQ ID NO:1 or position 621 of SEQ ID NO:2; a polypeptide having a valine, threonine, glutamine, cysteine, or methionine at a position corresponding to position 122 of SEQ ID NO:1 1 or position 90 of SEQ ID NO:2, a serine, alanine, glutamic acid, leucine, glutamine, arginine, valine, tryptophan, tyrosine, or isoleucine at a position corresponding to position 197 of SEQ ID NO:1 1 or position 165 of SEQ ID NO:2 and a glycine, or tryptophan at a position corresponding to position 653 of SEQ ID NO:1 or position 621 of SEQ ID NO:2.

The present invention provides herbicide-tolerant or herbicide-resistant plants comprising a herbicide-tolerant or herbicide-resistant AHASL protein including, but not limited to, AHASL single mutant polypeptides and AHASL double and triple mutant polypeptides that are encoded by the polynucleotides of the present invention. By a "herbicide-tolerant" or "herbicide-resistant" plant, it is intended that a plant that is tolerant or resistant to at least one herbicide at a level that would normally kill, or inhibit the growth of, a normal or wild-type plant. By "herbicide-tolerant AHASL protein" or "herbicide-resistant AHASL protein", it is intended that such an AHASL protein displays higher AHAS activity, relative to the AHAS activity of a wild-type AHASL protein, when in the presence of at least one herbicide that is known to interfere with AHAS activity and at a concentration or level of the herbicide that is known to inhibit the AHAS activity of the wild-type AHASL protein. Furthermore, the AHAS activity of such a herbicide-tolerant or herbicide-resistant AHASL protein may be referred to herein as "herbicide-tolerant" or "herbicide-resistant" AHAS activity.

For the present invention, the terms "herbicide-tolerant" and "herbicide-resistant" are used interchangeable and are intended to have an equivalent meaning and an equivalent scope. Similarly, the terms "herbicide-tolerance" and "herbicide-resistance" are used interchangeable and are intended to have an equivalent meaning and an equivalent scope. Likewise, the terms "imidazolinone-resistant" and "imidazolinone-resistance" are used interchangeable and are intended to be of an equivalent meaning and an equivalent scope as the terms "imidazolinone-tolerant" and "imidazolinone-tolerance", respectively.

The invention encompasses herbicide-resistant AHASL polynucleotides and herbicide-resistant AHASL proteins. By "herbicide-resistant AHASL polynucleotide" is intended a polynucleotide that encodes a protein comprising herbicide-resistant AHAS activity. By "herbicide-resistant AHASL protein" is intended a protein or polypeptide that comprises herbicide-resistant AHAS activity.

Further, it is recognized that a herbicide-tolerant or herbicide-resistant AHASL protein can be introduced into a plant by transforming a plant or ancestor thereof with a nucleotide sequence encoding a herbicide-tolerant or herbicide-resistant AHASL protein. Such herbicide-tolerant or herbicide-resistant AHASL proteins are encoded by the herbicide-tolerant or herbicide-resistant AHASL polynucleotides. Alternatively, a herbicide-tolerant or herbicide-resistant AHASL protein such as, for example, an AHASL single mutation polypeptide as disclosed herein, may occur in a plant as a result of a naturally occurring or induced mutation in an endogenous AHASL gene in the genome of a plant or progenitor thereof.

The present invention provides plants, plant tissues, plant cells, and host cells with increased resistance or tolerance to at least one herbicide, particularly an imidazolinone or sulfonylurea herbicide. The preferred amount or concentration of the herbicide is an "effective amount" or "effective concentration." By "effective amount" and "effective concentration" is intended an amount and concentration, respectively, that is sufficient to kill or inhibit the growth of a similar, wild-type, plant, plant tissue, plant cell, or host cell, but that said amount does not kill or inhibit as severely the growth of the herbicide-resistant plants, plant tissues, plant cells, and host cells of the present invention. Typically, the effective amount of a herbicide is an amount that is routinely used in agricultural production systems to kill weeds of interest. Such an amount is known to those of ordinary skill in the art.

By "similar, wild-type, plant, plant tissue, plant cell or host cell" is intended a plant, plant tissue, plant cell, or host cell, respectively, that lacks the herbicide-resistance characteristics and/or particular polynucleotide of the invention that are disclosed herein. The use of the term "wild-type" is not, therefore, intended to imply that a plant, plant tissue, plant cell, or other host cell lacks recombinant DNA in its genome, and/or does not possess herbicide-resistant characteristics that are different from those disclosed herein.

As used herein unless clearly indicated otherwise, the term "plant" intended to mean a plant at any developmental stage, as well as any part or parts of a plant that may be attached to or separate from a whole intact plant. Such parts of a plant include, but are not limited to, organs, tissues, and cells of a plant. Examples of particular plant parts include a stem, a leaf, a root, an inflorescence, a flower, a floret, a fruit, a pedicle, a peduncle, a stamen, an anther, a stigma, a style, an ovary, a petal, a sepal, a carpel, a root tip, a root cap, a root hair, a leaf hair, a seed hair, a pollen grain, a microspore, a cotyledon, a hypocotyl, an epicotyl, xylem, phloem, parenchyma, endosperm, a companion cell, a guard cell, and any other known organs, tissues, and cells of a plant. Furthermore, it is recognized that a seed is a plant.

The plants of the present invention include both non-transgenic plants and transgenic plants. By "non-transgenic plant" is intended to mean a plant lacking recombinant DNA in its genome. By "transgenic plant" is intended to mean a plant comprising recombinant DNA in its genome. Such a transgenic plant can be produced by introducing recombinant DNA into the genome of the plant. When such recombinant DNA is incorporated into the genome of the transgenic plant, progeny of the plant can also comprise the recombinant DNA. A progeny plant that comprises at least a portion of the recombinant DNA of at least one progenitor transgenic plant is also a transgenic plant.

In certain embodiments, the present invention involves herbicide-resistant plants that are produced by mutation breeding. Such plants comprise a polynucleotide encoding an AHAS large subunit single mutant polypeptide and are tolerant to one or more AHAS-inhibiting herbicides. Such methods can involve, for example, exposing the plants or seeds to a mutagen, particularly a chemical mutagen such as, for example, ethyl methanesulfonate (EMS) and selecting for plants that have enhanced tolerance to at least one AHAS-inhibiting herbicide, particularly an imidazolinone herbicide or sulfonylurea herbicide. However, the present invention is not limited to herbicide-tolerant plants that are produced by a mutagenesis method involving the chemical mutagen EMS. Any mutagenesis method known in the art may be used to produce the herbicide-resistant plants of the present invention. Such mutagenesis methods can involve, for example, the use of any one or more of the following mutagens: radiation, such as X-rays, Gamma rays (e.g., cobalt 60 or cesium 137), neutrons, (e.g., product of nuclear fission by uranium 235 in an atomic reactor), Beta radiation (e.g., emitted from radioisotopes such as phosphorus 32 or carbon 14), and ultraviolet radiation (preferably from 2500 to 2900 nm), and chemical mutagens such as base analogues (e.g., 5-bromo-uracil), related compounds (e.g., 8- ethoxy caffeine), antibiotics (e.g., streptonigrin), alkylating agents (e.g., sulfur mustards, nitrogen mustards, epoxides, ethylenamines, sulfates, sulfonates, sulfones, lactones), azide, hydroxylamine, nitrous acid, or acridines. Herbicide-resistant plants can also be produced by using tissue culture methods to select for plant cells comprising herbicide-resistance mutations and then regenerating herbicide-resistant plants therefrom. See, for example, U.S. Patent Nos. 5,773,702 and 5,859,348. Further details of mutation breeding can be found in "Principals of Cultivar Development" Fehr, 1993 Macmillan Publishing Company.

Further, methods for enhancing the tolerance or resistance of a plant, plant tissue, plant cell, or other host cell to at least one herbicide that interferes with the activity of the AHAS enzyme are described herein. Preferably, such a herbicide is an imidazolinone herbicide, a sulfonylurea herbicide, a triazolopyrimidine herbicide, a pyrimidinyloxybenzoate herbicide, a sulfonylamino-carbonyltriazolinone herbicide, or mixture thereof. More preferably, such a herbicide is an imidazolinone herbicide, a sulfonylurea herbicide, or mixture thereof. For the present invention, the imidazolinone herbicides include, but are not limited to, PURSUIT® (imazethapyr), CADRE® (imazapic), RAPTOR® (imazamox), SCEPTER® (imazaquin), ASSERT® (imazethabenz), ARSENAL® (imazapyr), a derivative of any of the aforementioned herbicides, and a mixture of two or more of the aforementioned herbicides, for example, imazapyr/imazamox (ODYSSEY®). More specifically, the imidazolinone herbicide can be selected from, but is not limited to, 2-(4-isopropyl-4-methyl-5-oxo-2-imidiazolin-2-yl)-nicotinic acid, 2-(4-isopropyl)-4-methyl-5-oxo-2-imidazolin-2-yl)-3-quinolinecarboxylic acid, 5-ethyl-2-(4-isopropyl-4-methyl-5-oxo-2-imidazolin-2-yl)-nicotinic acid, 2-(4-isopropyl-4-methyl-5-oxo-2- imidazolin-2-yl)-5-(methoxymethyl)-nicotinic acid, 2-(4-isopropyl-4-methyl-5-oxo-2-imidazolin-2-yl)-5-methylnicotinic acid, and a mixture of methyl 6-(4-isopropyl-4-methyl-5-oxo-2-imidazolin-2-yl)-m-toluate and methyl 2-(4-isopropyl-4-methyl-5-oxo-2-imidazolin-2-yl)-p-toluate. The use of 5-ethyl-2-(4-isopropyl-4-methyl-5-oxo-2-imidazolin-2-yl)-nicotinic acid and 2-(4-isopropyl-4-methyl-5-oxo-2-imidazolin-2-yl)-5-(methoxymethyl)-nicotinic acid is preferred. The use of 2-(4-isopropyl-4-methyl-5-oxo-2-imidazolin-2-yl)-5-(methoxymethyl)-nicotinic acid is particularly preferred.

For the present invention, the sulfonylurea herbicides include, but are not limited to, chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfuron, flazasulfuron, imazasulfuron, pyrazosulfuron ethyl, halosulfuron, azimsulfuron, cyclosulfuron, ethoxysulfuron, flazasulfuron, flupyrsulfuron methyl, foramsulfuron, iodosulfuron, oxasulfuron, mesosulfuron, prosulfuron, sulfosulfuron, trifloxysulfuron, tritosulfuron, a derivative of any of the aforementioned herbicides, and a mixture of two or more of the aforementioned herbicides. The triazolopyrimidine herbicides of the invention include, but are not limited to, cloransulam, diclosulam, florasulam, flumetsulam, metosulam, and penoxsulam. The pyrimidinyloxybenzoate (or pyrimidinyl carboxy) herbicides of the invention include, but are not limited to, bispyribac, pyrithiobac, pyriminobac, pyribenzoxim and pyriftalid. The sulfonylamino-carbonyltriazolinone herbicides include, but are not limited to, flucarbazone and propoxycarbazone.

It is recognized that pyrimidinyloxybenzoate herbicides are closely related to the pyrimidinylthiobenzoate herbicides and are generalized under the heading of the latter name by the Weed Science Society of America. Accordingly, the herbicides of the present invention further include pyrimidinylthiobenzoate herbicides, including, but not limited to, the pyrimidinyloxybenzoate herbicides described above.

Methods for enhancing AHAS activity in a plant may comprise transforming a plant with a polynucleotide construct comprising a promoter operably linked to an AHASL nucleotide sequence of the invention. The methods involve introducing a polynucleotide construct of the invention into at least one plant cell and regenerating a transformed plant therefrom. The methods involve the use of a promoter that is capable of driving gene expression in a plant cell. Preferably, such a promoter is a constitutive promoter or a tissue-preferred promoter. The methods find use in enhancing or increasing the resistance of a plant to at least one herbicide that interferes with the catalytic activity of the AHAS enzyme, particularly an imidazolinone herbicide.

The present invention provides expression cassettes for expressing the polynucleotides of the invention in plants, plant tissues, plant cells, and other host cells. The expression cassettes comprise a promoter expressible in the plant, plant tissue, plant cell, or other host cells of interest operably linked to a polynucleotide of the invention that comprises a nucleotide sequence encoding either a full-length (*i.e*. including the chloroplast transit peptide) or mature AHASL protein (*i.e.* without the chloroplast transit peptide). If expression is desired in the plastids or chloroplasts of plants or plant cells, the expression cassette may also comprise an operably linked chloroplast-targeting sequence that encodes a chloroplast transit peptide.

The expression cassettes of the invention find use in a method for enhancing the herbicide tolerance of a plant or a host cell. The method involves transforming the plant or host cell with an expression cassette of the invention, wherein the expression cassette comprises a promoter that is expressible in the plant or host cell of interest and the promoter is operably linked to a polynucleotide of the invention that comprises a nucleotide sequence encoding an imidazolinone-resistant AHASL protein of the invention.

The use of the term "polynucleotide constructs" herein is not intended to limit the present invention to polynucleotide constructs comprising DNA. Those of ordinary skill in the art will recognize that polynucleotide constructs, particularly polynucleotides and oligonucleotides, comprised of ribonucleotides and combinations of ribonucleotides and deoxyribonucleotides may also be employed in the methods disclosed herein. Thus, the polynucleotide constructs of the present invention encompass all polynucleotide constructs that can be employed in the methods of the present invention for transforming plants including, but not limited to, those comprised of deoxyribonucleotides, ribonucleotides, and combinations thereof. Such deoxyribonucleotides and ribonucleotides include both naturally occurring molecules and synthetic analogues. The polynucleotide constructs of the invention also encompass all forms of polynucleotide constructs including, but not limited to, single-stranded forms, double-stranded forms, hairpins, stem-and-loop structures, and the like. Furthermore, it is understood by those of ordinary skill in the art that each nucleotide sequences disclosed herein also encompasses the complement of that exemplified nucleotide sequence.

Further, it is recognized that, for expression of a polynucleotide of the invention in a host cell of interest, the polynucleotide is typically operably linked to a promoter that is capable of driving gene expression in the host cell of interest. The methods of the invention for expressing the polynucleotides in host cells do not depend on particular promoter. The methods encompass the use of any promoter that is known in the art and that is capable of driving gene expression in the host cell of interest.

The present invention encompasses AHASL polynucleotide molecules and fragments and variants thereof. Polynucleotide molecules that are fragments of these nucleotide sequences are also encompassed by the present invention. By "fragment" is intended a portion of the nucleotide sequence encoding an AHASL protein of the invention. Preferably, a fragment of an AHASL nucleotide sequence of the invention encodes a biologically active portion of an AHASL protein. A biologically active portion of an AHASL protein can be prepared by isolating a portion of one of the AHASL nucleotide sequences of the invention, expressing the encoded portion of the AHASL protein (e.g., by recombinant expression *in vitro*), and assessing the activity of the encoded portion of the AHASL protein. Polynucleotide molecules that are fragments of an AHASL nucleotide sequence and encode biologically active portions of AHASL proteins comprise at least about 500, 750, 1000, 1250, 1500, 1600, 1700, 1800, 1900, or 2000 nucleotides, or up to the number of nucleotides present in a full-length nucleotide sequence disclosed herein (for example, 2013 nucleotides for SEQ ID NO:30) depending upon the intended use.

A fragment of an AHASL nucleotide sequence that encodes a biologically active portion of an AHASL protein of the invention will encode at least about 200, 300, 400, 500, 550, 650, or 650 contiguous amino acids, or up to the total number of amino acids present in a full-length AHASL protein of the invention (for example, 670 amino acids for SEQ ID NO:1).

\ Polynucleotide molecules comprising nucleotide sequences that are variants of the nucleotide sequences disclosed herein are also encompassed by the present invention. "Variants" of the AHASL nucleotide sequences of the invention include those sequences that encode the mutant AHASL polypeptides disclosed herein but that differ conservatively because of the degeneracy of the genetic code. These naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the AHASL protein disclosed in the present invention as discussed below. Generally, polynucleotide sequence variants of the invention will have at least about 80%, 85%, 90%, 91%, 92%, 93%, 94%, 95%, 96%, 97%, 98%, or 99% identity to a particular nucleotide sequence disclosed herein. A variant AHASL polynucleotide sequence will encode an AHASL mutant polypeptide, respectively, that has an amino acid sequence having at least about 80%, 85%, 90%, 91%, 92%, 93%, 94%, 95%, 96%, 97%, 98%, or 99% identity to the amino acid sequence of an AHASL polypeptide disclosed herein.

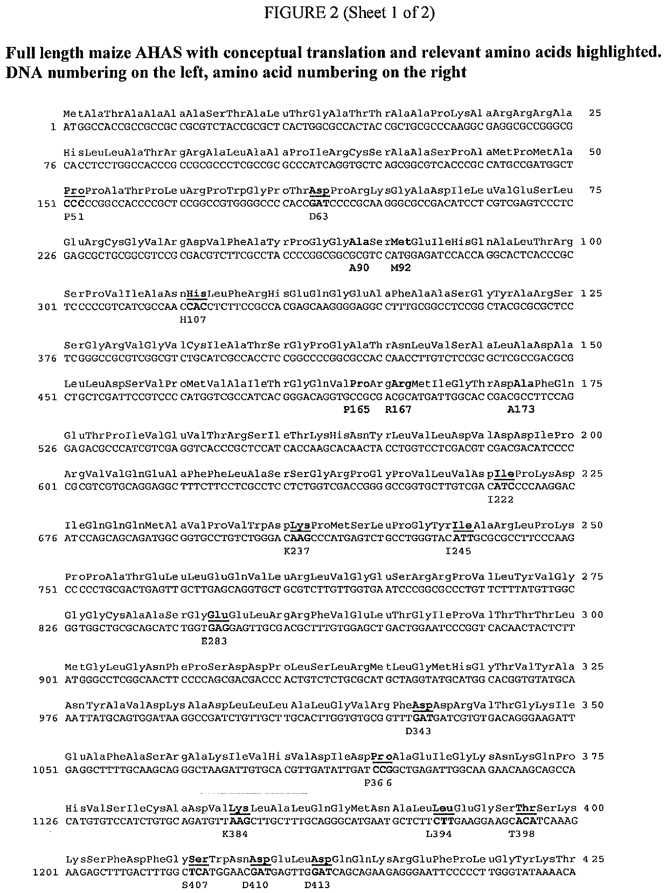

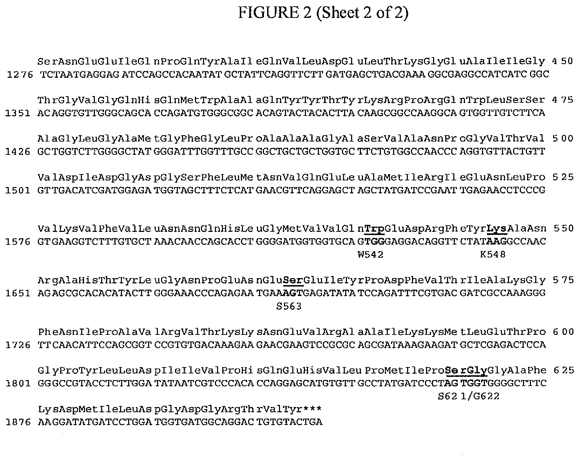

In addition, the skilled artisan will further appreciate that changes can be introduced by mutation into the polynucleotides sequences of the invention thereby leading to changes in the amino acid sequence of the encoded AHASL double and triple mutant polypeptides without altering the biological activity of the double and triple mutant polypeptides. Thus, an isolated polynucleotide molecule encoding an AHASL double and triple mutant polypeptide having a sequence that differs from the double and triple mutant sequences set forth in Figures 1 and 2 can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention.

For example, preferably, conservative amino acid substitutions may be made at one or more predicted, preferably nonessential amino acid residues. A "nonessential" amino acid residue is a residue that can be altered from the wild-type sequence of an AHASL protein (*e.g.*, the sequence of SEQ ID NO:1) without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g*., tyrosine, phenylalanine, tryptophan, histidine). Such substitutions would not be made for conserved amino acid residues, or for amino acid residues residing within a conserved motif.

The proteins of the invention may be altered in various ways including amino acid substitutions, deletions, truncations, and insertions. Methods for such manipulations are generally known in the art. For example, amino acid sequence variants of the AHASL proteins can be prepared by mutations in the DNA. Methods for mutagenesis and nucleotide sequence alterations are well known in the art. See, for example, Kunkel (1985) Proc. Natl. Acad. Sci. USA 82:488-492; Kunkel et al. (1987) Methods in Enzymol. 154:367-3 82; U.S. Patent No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Conservative substitutions, such as exchanging one amino acid with another having similar properties, may be preferable.

It is recognized that the polynucleotide molecules and polypeptides of the invention encompass polynucleotide molecules and polypeptides comprising a nucleotide or an amino acid sequence that is sufficiently identical to the double or triple nucleotide sequences set forth in Figures 1 and 2, or to the amino acid sequences set forth in Figures 1 and 2. The term "sufficiently identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity. For example, amino acid or nucleotide sequences that contain a common structural domain having at least about 80% identity, preferably 85% identity, more preferably 90%, 95%, or 98% identity are defined herein as sufficiently identical.

To determine the percent identity of two amino acid sequences or of two nucleic acids, the sequences are aligned for optimal comparison purposes. The percent identity between the two sequences is a function of the number of identical positions shared by the sequences (*i.e.,* percent identity = number of identical positions/total number of positions (*e.g*., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A preferred, nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the NBLAST and XBLAST programs of Altschul et al. (1990) J. Mol. Biol. 215:403. BLAST nucleotide searches can be performed with the NBLAST program, score = 100, wordlength = 12, to obtain nucleotide sequences homologous to the polynucleotide molecules of the invention. BLAST protein searches can be performed with the XBLAST program, score = 50, wordlength = 3, to obtain amino acid sequences homologous to protein molecules of the invention. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. *See*, Altschul *et al.* (1997) *supra*. When utilizing BLAST, Gapped BLAST, and PSI-Blast programs, the default parameters of the respective programs (e.g., XBLAST and NBLAST) can be used. *See* http://www.ncbi.nlm.nih.gov. Another preferred, non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package. When utilizing the ALIGN program for comparing amino acid sequences, a PAM120 weight residue table, a gap length penalty of 12, and a gap penalty of 4 can be used. Alignment may also be performed manually by inspection.

Unless otherwise stated, sequence identity/similarity values provided herein refer to the value obtained using the full-length sequences of the invention and using multiple alignment by mean of the algorithm Clustal W (Nucleic Acid Research, 22(22):4673-4680, 1994) using the program AlignX included in the software package Vector NTI Suite Version 9 (Invitrogen, 1600 Faraday Ave., Carlsbad, CA 92008) using the default parameters; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by AlignX in the software package Vector NTI Suite Version 9.

The deletions, insertions, and substitutions of the protein sequences encompassed herein are not expected to produce radical changes in the characteristics of the protein. However, when it is difficult to predict the exact effect of the substitution, deletion, or insertion in advance of doing so, one skilled in the art will appreciate that the effect will be evaluated by routine screening assays. That is, the activity can be evaluated by AHAS activity assays. *See,* for example, Singh et al. (1988) Anal. Biochem. 171:173-179.

As disclosed herein, the polynucleotides of the invention find use in enhancing the herbicide tolerance of plants that comprise in their genomes a gene encoding a herbicide-tolerant AHASL protein. Such a gene may be an endogenous gene or a transgene. Additionally, in certain embodiments, the polynucleotides of the present invention can be stacked with any combination of polynucleotide sequences of interest in order to create plants with a desired phenotype. For example, the polynucleotides of the present invention may be stacked with any other polynucleotides encoding polypeptides having pesticidal and/or insecticidal activity, such as, for example, the *Bacillus thuringiensis* toxin proteins (described in U.S. Patent Nos. 5,366,892; 5,747,450; 5,737,514; 5,723,756; 5,593,881; and Geiser et al. (1986) Gene 48:109). The combinations generated can also include multiple copies of any one of the polynucleotides of interest.

While the polynucleotides of the invention find use as selectable marker genes for plant transformation, the expression cassettes of the invention can include another selectable marker gene for the selection of transformed cells. Selectable marker genes, including those of the present invention, are utilized for the selection of transformed cells or tissues. Marker genes include, but are not limited to, genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). See generally, Yarranton (1992) Curr. Opin. Biotech. 3:506-511; Christopherson et al. (1992) Proc. Natl. Acad. Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschle et al. (1989) Proc. Natl. Acad. Sci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 (Springer-Verlag, Berlin); Gill et al. (1988) Nature 334:721-724.

The above list of selectable marker genes is not meant to be limiting. Any selectable marker gene can be used in the present invention.

The isolated polynucleotide molecules comprising nucleotide sequence that encode the AHASL proteins of the invention can be used in vectors to transform plants so that the plants created have enhanced resistant to herbicides, particularly an imidazolinone herbicide or sulfonylurea herbicide. The isolated AHASL polynucleotide molecules of the invention can be used in vectors alone or in combination with a nucleotide sequence encoding the small subunit of the AHAS (AHASS) enzyme in conferring herbicide resistance in plants. *See,* U.S. Patent No. 6,348,643.

The invention also relates to a plant expression vector comprising a promoter that drives expression in a plant operably linked to an isolated polynucleotide molecule of the invention. The isolated polynucleotide molecule comprises a nucleotide sequence encoding an AHASL protein of the invention, or a functional fragment and variant thereof. The plant expression vector of the invention does not depend on a particular promoter, only that such a promoter is capable of driving gene expression in a plant cell. Preferred promoters include constitutive promoters and tissue-preferred promoters.

The transformation vectors of the invention can be used to produce plants transformed with a gene of interest. The transformation vector will comprise a selectable marker gene of the invention and a gene of interest to be introduced and typically expressed in the transformed plant. Such a selectable marker gene comprises a polynucleotide of the invention that encodes an AHASL double or triple mutant polypeptide, wherein the polynucleotide is operably linked to a promoter that drives expression in a host cell. For use in plants and plant cells, the transformation vector comprises a selectable marker gene comprising a polynucleotide of the invention that encodes an AHASL double or triple mutant polypeptide operably linked to a promoter that drives expression in a plant cell.

The genes of interest of the invention vary depending on the desired outcome. For example, various changes in phenotype can be of interest including modifying the fatty acid composition in a plant, altering the amino acid content of a plant, altering a plant's insect and/or pathogen defense mechanisms, and the like. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in a change in -phenotype of the transformed plant.

In one embodiment of the invention, the genes of interest include insect resistance genes such as, for example, *Bacillus thuringiensis* toxin protein genes (U.S. Patent Nos. 5,366,892; 5,747,450; 5,736,514; 5,723,756; 5,593,881; and Geiser et al. (1986) Gene 48:109).

The AHASL proteins or polypeptides of the invention can be purified from, for example, sunflower plants and can be used in compositions. Also, an isolated polynucleotide molecule encoding an AHASL protein of the invention can be used to express an AHASL protein of the invention in a microbe such as *E. coli* or a yeast. The expressed AHASL protein can be purified from extracts of *E*. *coli* or yeast by any method known to those of ordinary skill in the art.

The polynucleotides of the invention find use in methods for enhancing the resistance of herbicide-tolerant plants. In one embodiment of the invention, the herbicide-tolerant plants that comprise a polynucleotide of the invention that encodes an AHASL double or triple mutant polypeptide. The invention further provides herbicide-tolerant plants that comprise two or more polynucleotides encoding AHASL single mutant polypeptides. Polynucleotides encoding herbicide-tolerant AHASL proteins and herbicide-tolerant plants comprising an endogenous gene that encodes a herbicide-tolerant AHASL protein include the polynucleotides and plants of the present invention and those that are known in the art. *See,* for example, U.S. Patent Nos. 5,013,659, 5,731,180, 5,767,361, 5,545,822, 5,736,629, 5,773,703, 5,773,704, 5,952,553 and 6,274,796. Such methods for enhancing the resistance of herbicide-tolerant plants comprise transforming a herbicide-tolerant plant with at least one polynucleotide construct comprising a promoter that drives expression in a plant cell that is operably linked to a polynucleotide of the invention.

Numerous plant transformation vectors and methods for transforming plants are available. *See*, for example, An, G. et al. (1986) Plant Physiol., 81:301-305; Fry, J., et al. (1987) Plant Cell Rep. 6:321-325; Block, M. (1988) Theor. Appl Genet.76:767-774*;* Hinchee, et al. (1990) Stadler. Genet. Symp.203212.203-212; Cousins, et al. (1991) Aust. J. Plant Physiol. 18:481-494; Chee, P. P. and Slightom, J. L. (1992) Gene.118:255-260; Christou, et al. (1992) Trends. Biotechnol. 10:239-246; D'Halluin, et al. (1992) Bio/Technol. 10:309-314; Dhir, et al. (1992) Plant Physiol. 99:81-88; Casas et al. (1993) Proc. Nat. Acad Sci. USA 90:11212-11216; Christou, P. (1993) In Vitro Cell. Dev. Biol.-Plant; 29P:119-124; Davies, et al. (1993) Plant Cell Rep. 12:180-183; Dong, J. A. and Mchughen, A. (1993) Plant Sci. 91:139-148; Franklin, C. I. and Trieu, T. N. (1993) Plant. Physiol. 102:167; Golovkin, et al. (1993) Plant Sci. 90:41-52; Guo Chin Sci. Bull. 38:2072-2078; Asano, et al. (1994) Plant Cell Rep. 13; Ayeres N. M. and Park, W. D. (1994) Crit. Rev. Plant. Sci. 13:219-239; Barcelo, et al. (1994) Plant. J. 5:583-592; Becker, et al. (1994) Plant. J. 5:299-307; Borkowska et al. (1994) Acta. Physiol Plant. 16:225-230; Christou, P. (1994) Agro. Food. Ind. Hi Tech. 5: 17-27; Eapen et al. (1994) Plant Cell Rep. 13:582-586; Hartman, et al. (1994) Bio-Technology 12: 919923; Ritala, et al. (1994) Plant. Mol. Biol. 24:317-325; and Wan, Y. C. and Lemaux, P. G. (1994) Plant Physiol. 104:3748.

The methods of the invention involve introducing a polynucleotide construct into a plant. By "introducing" is intended presenting to the plant the polynucleotide construct in such a manner that the construct gains access to the interior of a cell of the plant. The methods of the invention do not depend on a particular method for introducing a polynucleotide construct to a plant, only that the polynucleotide construct gains access to the interior of at least one cell of the plant. Methods for introducing polynucleotide constructs into plants are known in the art including, but not limited to, stable transformation methods, transient transformation methods, and virus-mediated methods.

By "stable transformation" is intended that the polynucleotide construct introduced into a plant integrates into the genome of the plant and is capable of being inherited by progeny thereof. By "transient transformation" is intended that a polynucleotide construct introduced into a plant does not integrate into the genome of the plant.

For the transformation of plants and plant cells, the nucleotide sequences of the invention are inserted using standard techniques into any vector known in the art that is suitable for expression of the nucleotide sequences in a plant or plant cell. The selection of the vector depends on the preferred transformation technique and the target plant species to be transformed. In an embodiment of the invention, an AHASL nucleotide sequence is operably linked to a plant promoter that is known for high-level expression in a plant cell, and this construct is then introduced into a plant that is susceptible to an imidazolinone or sulfonylurea herbicide and a transformed plant is regenerated. The transformed plant is tolerant to exposure to a level of an imidazolinone or sulfonylurea herbicide that would kill or significantly injure an untransformed plant. This method can be applied to any plant species; however, it is most beneficial when applied to crop plants.

Methodologies for constructing plant expression cassettes and introducing foreign nucleic acids into plants are generally known in the art and have been previously described. For example, foreign DNA can be introduced into plants, using tumor-inducing (Ti) plasmid vectors. Agrobacterium based transformation techniques are well known in the art. The Agrobacterium strain (e.g., *Agrobacterium tumefaciens* or *Agrobacterium rhizogenes*) comprises a plasmid (Ti or Ri plasmid) and a T-DNA element which is transferred to the plant following infection with Agrobacterium. The T-DNA (transferred DNA) is integrated into the genome of the plant cell. The T-DNA may be localized on the Ri- or Ti-plasmid or is separately comprised in a so-called binary vector. Methods for the Agrobacterium-mediated transformation are described, for example, in Horsch RB et al. (1985) Science 225:1229f. The Agrobacterium-mediated transformation can be used in both dicotyledonous plants and monocotyledonous plants. The transformation of plants by *Agrobacteria* is described in White FF, Vectors for Gene Transfer in Higher Plants; Vol. 1, Engineering and Utilization, edited by S.D. Kung and R. Wu, Academic Press, 1993, pp. 15 - 38; Jenes B et al. (1993) Techniques for Gene Transfer, in: Transgenic Plants, Vol. 1, Engineering and Utilization, edited by S.D. Kung and R. Wu, Academic Press, pp. 128-143; Potrykus (1991) Annu Rev Plant Physiol Plant Molec Biol 42:205- 225. Other methods utilized for foreign DNA delivery involve the use of PEG mediated protoplast transformation, electroporation, microinjection whiskers, and biolistics or microprojectile bombardment for direct DNA uptake. Such methods are known in the art. (U.S. Pat. No. 5,405,765 to Vasil et al.; Bilang et al. (1991) Gene 100: 247-250; Scheid et al. (1991) Mol. Gen. Genet., 228: 104-112; Guerche et al. (1987) Plant Science 52: 111-116; Neuhause et al. (1987) Theor. Appl Genet. 75: 30-36; Klein et al. (1987) Nature 327: 70-73; Howell et al. (1980) Science 208:1265; Horsch et al. (1985) Science 227: 1229-1231; DeBlock et al. (1989) Plant Physiology 91: 694-701; Methods for Plant Molecular Biology (Weissbach and Weissbach, eds.) Academic Press, Inc. (1988) and Methods in Plant Molecular Biology (Schuler and Zielinski, eds.) Academic Press, Inc. (1989). The method of transformation depends upon the plant cell to be transformed, stability of vectors used, expression level of gene products and other parameters.

Other suitable methods of introducing nucleotide sequences into plant cells and subsequent insertion into the plant genome include microinjection as Crossway et al. (1986) Biotechniques 4:320-334, electroporation as described by Riggs et al. (1986) Proc. Natl. Acad. Sci. USA 83:5602-5606, *Agrobacterium*-mediated transformation as described by Townsend *et al.* U.S. Patent No. 5,563,055, Zhao *et al.* U.S. Patent No. 5,981,840, direct gene transfer as described by Paszkowski et al. (1984) EMBO J. 3:2717-2722, and ballistic particle acceleration as described in, for example, Sanford et al. U.S. Patent No. 4,945,050; Tomes et al. U.S. Patent No. 5,879,918; Tomes et al. U.S. Patent No. 5,886,244; Bidney et al. U.S. Patent No. 5,932,782; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); McCabe et al. (1988) Biotechnology 6:923-926); and Lec1 transformation (WO 00/28058). *Also see,* Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); Tomes, U.S. Patent No. 5,240,855; Buising et al., U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin) (maize); Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; Bowen et al., U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*).

The polynucleotides of the invention may be introduced into plants by contacting plants with a virus or viral nucleic acids. Generally, such methods involve incorporating a polynucleotide construct of the invention within a viral DNA or RNA molecule. It is recognized that the AHASL protein of the invention may be initially synthesized as part of a viral polyprotein, which later may be processed by proteolysis *in vivo* or *in vitro* to produce the desired recombinant protein. Further, it is recognized that promoters that may be used according to the invention also encompass promoters utilized for transcription by viral RNA polymerases. Methods for introducing polynucleotide constructs into plants and expressing a protein encoded therein, involving viral DNA or RNA molecules, are known in the art. See, for example, U.S. Patent Nos. 5,889,191, 5,889,190, 5,866,785, 5,589,367 and 5,316,931.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a polynucleotide construct of the invention, for example, an expression cassette of the invention, stably incorporated into their genome.

The present invention may be used for transformation of any plant species, including, but not limited to, monocots and dicots. Examples of plant species of interest include, but are not limited to, corn or maize (*Zea mays*), *Brassica sp.* (e.g., *B. napus, B. rapa, B. juncea*), particularly those *Brassica* species useful as sources of seed oil, alfalfa (*Medicago sativa*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet *(Eleusine coracana))*, sunflower (*Helianthus annuus),* safflower (*Carthamus tinctorius),* wheat (*Triticum aestivum*, *T*. *Turgidum ssp*. *durum),* soybean (*Glycine tinctorius)*, wheat (*Triticum aestivum, T. Turgidum ssp. durum),* soybean (*Glycine max),* tobacco (*Nicotiana tabacum),* potato *(Solanum tuberosum),* peanuts (*Arachis hypogaea),* cotton (*Gossypium barbadense, Gossypium hirsutum),* sweet potato *(Ipomoea batatus),* cassava *(Manihot esculenta),* coffee (*Coffea spp.),* coconut (*Cocos nucifera),* pineapple *(Ananas comosus),* citrus trees (*Citrus spp.),* cocoa (*Theobroma cacao),* tea (*Camellia sinensis),* banana (*Musa* spp.), avocado (*Persea americana),* fig (*Ficus casica),* guava *(Psidium guajava),* mango (*Mangifera indica),* olive (*Olea europaea),* papaya (*Carica papaya),* cashew (*Anacardium occidentale),* macadamia (*Macadamia integrifolia),* almond *(Prunus amygdalus),* sugar beets (*Beta vulgaris),* sugarcane (*Saccharum* spp.), oats, barley, vegetables, ornamentals, and conifers. Preferably, plants of the present invention are crop plants (for example, sunflower, *Brassica sp.*, cotton, sugar beet, soybean, peanut, alfalfa, safflower, tobacco, corn, rice, wheat, rye, barley triticale, sorghum, millet, etc.).

The plants of the invention are herbicide-resistant plants and thus, find use in methods for controlling weeds that involve the application of a herbicide. Thus, a method for controlling weeds in the vicinity of a herbicide-resistant plant of the invention is described herein. The method comprises applying an effective amount of a herbicide to the weeds and to the herbicide-resistant plant, wherein the plant has increased resistance to at least one AHASinhibiting herbicide, particularly an imidazolinone or sulfonylurea herbicide, when compared to a wild-type plant. In such a method for controlling weeds, the herbicide-resistant plants of the invention are preferably crop plants, including, but not limited to, sunflower, alfalfa, *Brassica sp.,* soybean, cotton, safflower, peanut, tobacco, tomato, potato, wheat, rice, maize, sorghum, barley, rye, millet, and sorghum.

By providing plants having increased resistance to herbicides, particularly imidazolinone and sulfonylurea herbicides, a wide variety of formulations can be employed for protecting plants from weeds, so as to enhance plant growth and reduce competition for nutrients. A herbicide can be used by itself for pre-emergence, post-emergence, pre-planting and at planting control of weeds in areas surrounding the plants described herein or an imidazolinone herbicide formulation can be used that contains other additives. The herbicide can also be used as a seed treatment. Additives found in an imidazolinone or sulfonylurea herbicide formulation include other herbicides, detergents, adjuvants, spreading agents, sticking agents, stabilizing agents, or the like. The herbicide formulation can be a wet or dry preparation and can include, but is not limited to, flowable powders, emulsifiable concentrates and liquid concentrates. The herbicide and herbicide formulations can be applied in accordance with conventional methods, for example, by spraying, irritation, dusting, or the like.

The present invention provides non-transgenic and transgenic plants and seeds with increased tolerance to at least one herbicide, particularly an AHAS-inhibiting herbicide, more particularly imidazolinone and sulfonylurea herbicides, most particularly imidazolinone herbicides. In preferred embodiment of the invention, the plants and seeds of the invention display a higher level of herbicide tolerance that similar plants that comprise only one AHASL single mutant polypeptide. Such plants and seeds of the invention find use in improved methods for controlling weeds that allow for the application of a herbicide to the weeds and to the herbicide-resistant plant at an effective amount that comprises a higher herbicidal concentration or rate than can be used with similar plants that comprise only one AHASL single mutant polypeptide. Accordingly, such improved methods provide superior weed control when compared to existing methods involving plants comprising only one AHASL single mutant polypeptide and the application of a lower herbicidal concentration or rate.

The present invention provides herbicide-resistant plants comprising polynucleotides encoding AHASL double or triple mutant polypeptides and herbicide-resistant plants comprising two or more polynucleotides encoding AHASL single mutant polypeptides. These herbicide-resistant plants of the present invention find use in methods for producing herbicide-resistant plants through conventional plant breeding involving sexual reproduction. The methods comprise crossing a first plant that is a herbicide-resistant plant of the invention to a second plant that is not resistant to the herbicide. The second plant can be any plant that is capable of producing viable progeny plants (i.e., seeds) when crossed with the first plant. Typically, but not necessarily, the first and second plants are of the same species. The methods can optionally involve selecting for progeny plants that comprise the polynucleotide encoding the AHASL mutant polypeptide or the two or more polynucleotides encoding AHASL single mutant polypeptides of the first plant. These methods can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant.

The herbicide-resistant plants of the invention that comprise polynucleotides encoding AHASL double or triple mutant polypeptides and herbicide-resistant plants comprising two or more polynucleotides encoding AHASL single mutant polypeptides also find use in methods for increasing the herbicide-resistance of a plant through conventional plant breeding involving sexual reproduction. The methods comprise crossing a first plant that is a herbicide-resistant plant of the invention to a second plant that may or may not be resistant to the same herbicide or herbicides as the first plant or may be resistant to different herbicide or herbicides than the first plant. The second plant can be any plant that is capable of producing viable progeny plants (i.e., seeds) when crossed with the first plant. Typically, but not necessarily, the first and second plants are of the same species. The methods can optionally involve selecting for progeny plants that comprise the polynucleotide encoding the AHASL mutant polypeptide or the two or more polynucleotides encoding AHASL single mutant polypeptides of the first plant and the herbicide resistance characteristics of the second plant. The progeny plants produced by this method have increased resistance to a herbicide when compared to either the first or second plant or both. When the first and second plants are resistant to different herbicides, the progeny plants will have the combined herbicide tolerance characteristics of the first and second plants. These methods can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant.

The present invention also provides plants, plant organs, plant tissues, plant cells, seeds, and non-human host cells that are transformed with the at least one polynucleotide molecule, expression cassette, or transformation vector of the invention. Such transformed plants, plant organs, plant tissues, plant cells, seeds, and non-human host cells have enhanced tolerance or resistance to at least one herbicide, at levels of the herbicide that kill or inhibit the growth of an untransformed plant, plant tissue, plant cell, or non-human host cell, respectively. Preferably, the transformed plants, plant tissues, plant cells, and seeds of the invention are *Arabidopsis thaliana* and crop plants.

The present invention provides methods that involve the use of at least one AHASinhibiting herbicide selected from the group consisting of imidazolinone herbicides, sulfonylurea herbicides, triazolopyrimidine herbicides, pyrimidinyloxybenzoate herbicides, sulfonylaminocarbonyltriazolinone herbicides, and mixtures thereof. In these methods, the AHAS-inhibiting herbicide can be applied by any method known in the art including, but not limited to, seed treatment, soil treatment, and foliar treatment.

Prior to application, the AHAS-inhibiting herbicide can be converted into the customary formulations, for example solutions, emulsions, suspensions, dusts, powders, pastes and granules. The use form depends on the particular intended purpose; in each case, it should ensure a fine and even distribution of the compound according to the invention.

The formulations are prepared in a known manner (see e.g. for review US 3,060,084, EP-A 707 445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al. Weed Control Handbook, 8th Ed., Blackwell Scientific Publications, Oxford, 1989 and Mollet, H., Grubemann, A., Formulation technology, Wiley VCH Verlag GmbH, Weinheim (Germany), 2001, 2. D. A. Knowles, Chemistry and Technology of Agrochemical Formulations, Kluwer Academic Publishers, Dordrecht, 1998 (ISBN 0-7514-0443-8), for example by extending the active compound with auxiliaries suitable for the formulation of agrochemicals, such as solvents and/or carriers, if desired emulsifiers, surfactants and dispersants, preservatives, antifoaming agents, anti-freezing agents, for seed treatment formulation also optionally colorants and/or binders and/or gelling agents.

Examples of suitable solvents are water, aromatic solvents (for example Solvesso products, xylene), paraffins (for example mineral oil fractions), alcohols (for example methanol, butanol, pentanol, benzyl alcohol), ketones (for example cyclohexanone, gamma-butyrolactone), pyrrolidones (NMP, NOP), acetates (glycol diacetate), glycols, fatty acid dimethylamides, fatty acids and fatty acid esters. In principle, solvent mixtures may also be used.

Examples of suitable carriers are ground natural minerals (for example kaolins, clays, talc, chalk) and ground synthetic minerals (for example highly disperse silica, silicates).

Suitable emulsifiers are nonionic and anionic emulsifiers (for example polyoxyethylene fatty alcohol ethers, alkylsulfonates and arylsulfonates).

Examples of dispersants are lignin-sulfite waste liquors and methylcellulose.

Suitable surfactants used are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol ethylene oxide condensates, ethoxylated castor oil, polyoxyethylene alkyl ethers, ethoxylated polyoxypropylene, lauryl alcohol polyglycol ether acetal, sorbitol esters, lignosulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, highly polar solvents, for example dimethyl sulfoxide, N-methylpyrrolidone or water.

Also anti-freezing agents such as glycerin, ethylene glycol, propylene glycol and bactericides such as can be added to the formulation.

Suitable antifoaming agents are for example antifoaming agents based on silicon or magnesium stearate.

Suitable preservatives are, for example, dichlorophenol and benzylalcoholhemiformaldehyde.

Seed Treatment formulations may additionally comprise binders and optionally colorants.

Binders can be added to improve the adhesion of the active materials on the seeds after treatment. Suitable binders are block copolymers EO/PO surfactants but also polyvinylalcohols, polyvinylpyrrolidones, polyacrylates, polymethacrylates, polybutenes, polyisobutylenes, polystyrene, polyethyleneamines, polyethyleneamides, polyethyleneimines (Lupasol®, Polymin®), polyethers, polyurethans, polyvinylacetate, tylose and copolymers derived from these polymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

An example of a suitable gelling agent is carrageen (Satiagel®).

Powders, materials for spreading, and dustable products can be prepared by mixing or concomitantly grinding the active substances with a solid carrier.

Granules, for example coated granules, impregnated granules and homogeneous granules, can be prepared by binding the active compounds to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the AHAS-inhibiting herbicide. In this case, the AHAS-inhibiting herbicides are employed in a purity of from 90% to 100% by weight, preferably 95% to 100% by weight (according to NMR spectrum). For seed treatment purposes, respective formulations can be diluted 2-10 fold leading to concentrations in the ready to use preparations of 0.01 to 60% by weight active compound by weight, preferably 0.1 to 40% by weight.

The AHAS-inhibiting herbicide can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the AHAS-inhibiting herbicide according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. However, it is also possible to prepare concentrates composed of active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, preferably from 0.01 to 1% per weight.

The AHAS-inhibiting herbicide may also be used successfully in the ultra-low-volume process (ULV), it being possible to apply formulations comprising over 95% by weight of active compound, or even to apply the active compound without additives.

The following are examples of formulations:
1. Products for dilution with water for foliar applications. For seed treatment purposes, such products may be applied to the seed diluted or undiluted.
   A) Water-soluble concentrates (SL, LS)
      Ten parts by weight of the AHAS-inhibiting herbicide are dissolved in 90 parts by weight of water or a water-soluble solvent. As an alternative, wetters or other auxiliaries are added. The AHAS-inhibiting herbicide dissolves upon dilution with water, whereby a formulation with 10 % (w/w) of AHAS-inhibiting herbicide is obtained.
   B) Dispersible concentrates (DC)
      Twenty parts by weight of the AHAS-inhibiting herbicide are dissolved in 70 parts by weight of cyclohexanone with addition of 10 parts by weight of a dispersant, for example polyvinylpyrrolidone. Dilution with water gives a dispersion, whereby a formulation with 20% (w/w) of AHAS-inhibiting herbicide is obtained.
   C) Emulsifiable concentrates (EC)
      Fifteen parts by weight of the AHAS-inhibiting herbicide are dissolved in 7 parts by weight of xylene with addition of calcium dodecylbenzenesulfonate and castor oil ethoxylate (in each case 5 parts by weight). Dilution with water gives an emulsion, whereby a formulation with 15% (w/w) of AHAS-inhibiting herbicide is obtained.
   D) Emulsions (EW, EO, ES)
      Twenty-five parts by weight of the AHAS-inhibiting herbicide are dissolved in 35 parts by weight of xylene with addition of calcium dodecylbenzenesulfonate and castor oil ethoxylate (in each case 5 parts by weight). This mixture is introduced into 30 parts by weight of water by means of an emulsifier machine (e.g. Ultraturrax) and made into a homogeneous emulsion. Dilution with water gives an emulsion, whereby a formulation with 25% (w/w) of AHAS-inhibiting herbicide is obtained.
   E) Suspensions (SC, OD, FS)
      In an agitated ball mill, 20 parts by weight of the AHAS-inhibiting herbicide are comminuted with addition of 10 parts by weight of dispersants, wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained.
   F) Water-dispersible granules and water-soluble granules (WG, SG)
      Fifty parts by weight of the AHAS-inhibiting herbicide are ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the AHAS-inhibiting herbicide, whereby a formulation with 50% (w/w) of AHAS-inhibiting herbicide is obtained.
   G) Water-dispersible powders and water-soluble powders (WP, SP, SS, WS)
      Seventy-five parts by weight of the AHAS-inhibiting herbicide are ground in a rotor-stator mill with addition of 25 parts by weight of dispersants, wetters and silica gel. Dilution with water gives a stable dispersion or solution of the AHAS-inhibiting herbicide, whereby a formulation with 75% (w/w) of AHAS-inhibiting herbicide is obtained.
   H) Gel-Formulation (GF)
      In an agitated ball mill, 20 parts by weight of the AHAS-inhibiting herbicide are comminuted with addition of 10 parts by weight of dispersants, 1 part by weight of a gelling agent wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained. This gel formulation is suitable for us as a seed treatment.
2. Products to be applied undiluted for foliar applications. For seed treatment purposes, such products may be applied to the seed diluted.
   A) Dustable powders (DP, DS)
      Five parts by weight of the AHAS-inhibiting herbicide are ground finely and mixed intimately with 95 parts by weight of finely divided kaolin. This gives a dustable product having 5% (w/w) of AHAS-inhibiting herbicide.
   B) Granules (GR, FG, GG, MG)
      One-half part by weight of the AHAS-inhibiting herbicide is ground finely and associated with 95.5 parts by weight of carriers, whereby a formulation with 0.5% (w/w) of AHAS-inhibiting herbicide is obtained. Current methods are extrusion, spray-drying or the fluidized bed. This gives granules to be applied undiluted for foliar use.

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, or either directly on the seeds.

In a preferred embodiment a FS formulation is used for seed treatment. Typically, an FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.
For seed treatment, seeds of the herbicide resistant plants according of the present invention are treated with herbicides, preferably herbicides selected from the group consisting of AHAS-inhibiting herbicides such as amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid, pyrithiobac, and mixtures thereof, or with a formulation comprising a AHAS-inhibiting herbicide.

The term seed treatment comprises all suitable seed treatment techniques known in the art, such as seed dressing, seed coating, seed dusting, seed soaking, and seed pelleting.

Further described herein is a method of treating soil by the application, in particular into the seed drill: either of a granular formulation containing the AHAS-inhibiting herbicide as a composition/formulation (e.g. a granular formulation, with optionally one or more solid or liquid, agriculturally acceptable carriers and/or optionally with one or more agriculturally acceptable surfactants. This method is advantageously employed, for example, in seedbeds of cereals, maize, cotton, and sunflower.

The present invention also comprises seeds coated with or containing with a seed treatment formulation comprising at least one AHAS-inhibiting herbicide selected from the group consisting of amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid and pyrithiobac.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

The seed treatment application with the AHAS-inhibiting herbicide or with a formulation comprising the AHAS-inhibiting herbicide is carried out by spraying or dusting the seeds before sowing of the plants and before emergence of the plants.

In the treatment of seeds, the corresponding formulations are applied by treating the seeds with an effective amount of the AHAS-inhibiting herbicide or a formulation comprising the AHAS-inhibiting herbicide. Herein, the application rates are generally from 0.1 g to 10 kg of the a.i. (or of the mixture of a.i. or of the formulation) per 100 kg of seed, preferably from 1 g to 5 kg per 100 kg of seed, in particular from 1 g to 2.5 kg per 100 kg of seed. For specific crops such as lettuce the rate can be higher.

A method for combating undesired vegetation or controlling weeds may comprise contacting the seeds of the resistant plants according to the present invention before sowing and/or after pregermination with an AHAS-inhibiting herbicide. The method can further comprise sowing the seeds, for example, in soil in a field or in a potting medium in greenhouse. The method finds particular use in combating undesired vegetation or controlling weeds in the immediate vicinity of the seed.

The control of undesired vegetation is understood as meaning the killing of weeds and/or otherwise retarding or inhibiting the normal growth of the weeds. Weeds, in the broadest sense, are understood as meaning all those plants which grow in locations where they are undesired.

The weeds as described herein include, for example, dicotyledonous and monocotyledonous weeds. Dicotyledonous weeds include, but are not limited to, weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, and Taraxacum. Monocotyledonous weeds include, but are not limited to, weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, and Apera.

In addition, the weeds as described herein can include, for example, crop plants that are growing in an undesired location. For example, a volunteer maize plant that is in a field that predominantly comprises soybean plants can be considered a weed, if the maize plant is undesired in the field of soybean plants.

The articles "a" and "an" are used herein to refer to one or more than one (i.e., to at least one) of the grammatical object of the article. By way of example, "an element" means one or more elements.

As used herein, the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation.

### Example 1

### Vectors containing Arabidopsis AHASL mutant genes

The entire XbaI fragment of *Arabidopsis thaliana* genomic DNA that contains the entire AHAS large subunit gene with some additional DNA, inclusive of the XbaI sites at the 5' and 3' ends is set forth in SEQ ID NO:34 (AtAHASL). Bases 2484 to 4496 of SEQ ID NO:3-4 encompass the coding sequence of the *Arabidopsis thaliana* AHAS large subunit gene serine 653 to threonine mutant allele, inclusive of the stop codon shown in SEQ ID NO:30. A smaller genomic fragment of the *Arabidopsis thaliana* AHAS large subunit gene serine 653 to threonine mutant allele shown in SEQ ID NO:33, encompassed in bases 2484 to 5717 of SEQ ID NO:34, includes the coding sequence and the 3' end, up to and including the 3' end XbaI site, with the first two bases of the NcoI site found at the start codon of AtAHASL left off for clarity.

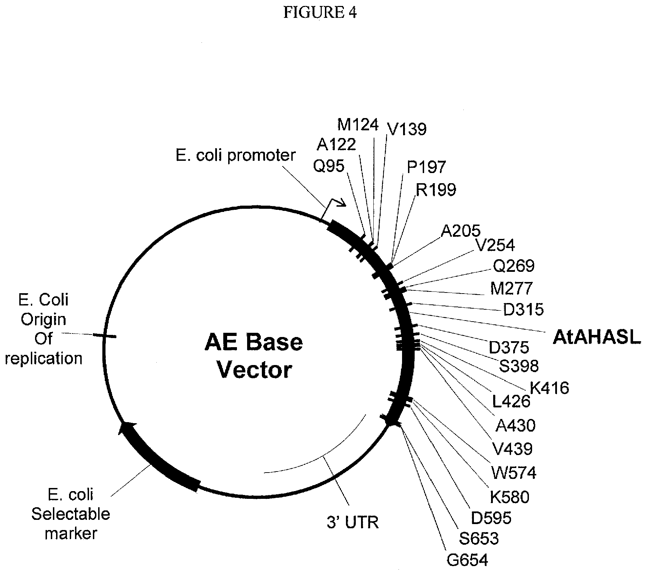

The DNA fragment of SEQ ID NO: 33 encoding the full-length *Arabidopsis* AHASL single mutation S653N and 3' untranslated region was cloned into pKK233-2 to yield the vector designated AE1 for expression and testing in *E coli.* (pKK233-2, bacterial expression vector, Pharmacia, GenBank Accession No. X70478). Vectors AE2 through AE9 were generated from AE1 by mutagenesis and standard cloning procedures. Figure 4 shows the map of the AE1 base vector, with positions of mutations indicated.

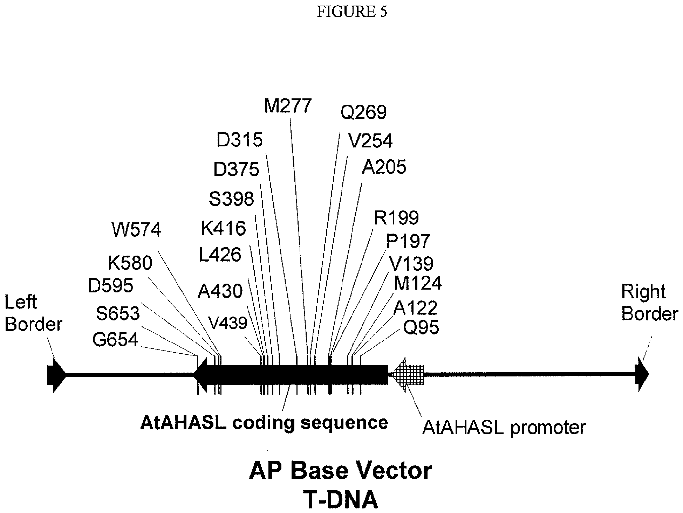

Vector AP1 (Figure 5) is a plant transformation vector that includes a genomic fragment of *Arabidopsis thaliana* DNA that includes the AtAHASL gene with the single S653N mutation (SEQ ID NO:34). The DNA fragment as shown in SEQ ID NO: 34 was cloned into AP1 in the reverse-complement orientation. Vectors AP2-AP7 were generated from AP1 and the AE plasmids using standard cloning procedures and differ only by mutations as indicated in Table 1. For convenience in cloning, different fragments were used to generate AP6 and AP7, compared to AP2-AP5. Thus, AP6 and AP7 are 47 base pairs shorter than AP1-AP5. This difference is in the plasmid backbone and not the *Arabidopsis thaliana* genomic fragment.

Vectors AE10 through AE24 were made as follows. The wild type *Arabidopsis thaliana* AHAS large subunit gene was amplified under mutagenic conditions using the Genemorph II random mutagenesis kit (Stratagene, La Jolla, California), resulting in randomly mutagenized amplified DNA fragments of this gene. This mutant DNA was then cloned back into AE7, replacing the wild type *A. thaliana* large subunit gene (between the unique SacII and AgeI sites on AE7) with the mutagenized forms. This DNA was transformed into *E coli* strain TOP10 and selected on LB agar medium in such a fashion as to have a large number of unique transformants, each with independent, mutagenized AHAS genes. These colonies were scraped together and plasmid DNA was prepared from this entire primary library. This DNA was transformed into AHAS minus *E coli* and again selected on LB agar media with carbenicillin. Plasmid positive colonies from this step were replica plated using velveteen onto minimal agar medium without branched chain amino acids and containing 30-micromolar imazethapyr. Those colonies that grew on this selective media possessed a functional *A. thaliana* AHAS mutant gene that was also imidazolinone tolerant.

The DNA sequence of the *A. thaliana* AHAS large subunit gene was determined for each of the growth positive colonies. No effort was made to determine the sequence of the *A. thaliana* AHAS large subunit genes that did not confer growth on the selective media. Because the AHAS function and imidazolinone tolerance screen was on a secondary library, replicates of the same mutations were found, as determined by DNA sequence analysis. Only one clone of each was advanced for testing on increasing imidazolinone concentrations and inclusion in Table 1.

**Table 1**

| *E. coli* Plasmid* | *Arabidopsis* Transformation Vector* | Mutations* | *E. coli* Imazethapyr Tolerance Score** | Transgenic Plant Tolerance: X-fold improvement over AP1 ^{@}(approximate) |
|---|---|---|---|---|
| AE1 | AP1 | S653N | + | NA |
| AE2 | AP2 | A122T & S653N | ++ | 16 |
| AE3 | AP3 | P197S & S653N | + | 2 |
| AE4 | AP8 | A122T, R199A, & S653N | NA | 16 |
| AE5 | AP4 | R199A, & S653N | +++ | 1.5 |
| AE6 | AP5 | A122T, P197S, & S653N | NA | 8 |
| AE7 | | Wild type | - (IN) | NA |
| AE8 | AP6 | A122T and R199A | +++ | 2 |
| AE9 | AP7 | A122T and P197S | NA | 8 |
| AE10 | | A122T, S57R and S398L | +++ | NA |
| AE11 | | A122T and V139I | ++ | NA |
| AE12 | | A122T and Q269H | + | NA |
| AE13 | | A122T and K416M | ++ | NA |
| AE14 | | A122T and L426I | +++ | NA |
| AE15 | | A122T and A430V | +++ | NA |
| AE16 | | A122T and N442I | ++ | NA |
| AE17 | | A122T and N445I | ++ | NA |
| AE18 | | A122T and N445D | +++ | NA |
| AE19 | | A122T and K580E | +++ | NA |
| AE20 | | A122T, V439G, D595G, and S653N | + | NA |
| AE21 | | P197S and D375N | +++ | NA |
| AE22 | | D375N | untested^{†} | NA |
| AE23 | | D375N, K83R, V254I, M277I, and D315Y | + | NA |
| AE24 | | Q95L, K416E, and S653N | + | NA |

| | | | | |
|---|---|---|---|---|
| * List of vectors for expression of AtAHASL2 in *E. coli* (AE plasmids) and for plant transformation plasmids (AP plasmids). Mutations in each vector are indicated relative to SEQ ID NO: 1. ** A simple single, double or triple plus system, +, ++, or +++ for respectively increasing colony size, was used to visually score the tolerance of the *Arabidopsis* AHAS function in AHAS minus *E. coli* containing the AE plasmids in the presence of the AHAS inhibitor imazethapyr. A "-", indicates there was no growth, meaning the mutation combination caused an inactive protein or there was no tolerance for imazethapyr at the selected rate. IN means inactive protein, while NT means not imidazolinone tolerant. NA means no data available (not tested). ^{†} Not tested compared to S653N, fact of tolerance determined by screening protocol. ^{@} For transgenic plants comprising the AP1 vector, 18.75 µM imazethapyr was the highest concentration which allowed good growth of the plants in the microtiter format plates. This concentration was used as the basis for determining X-fold improvement over AP1. | | | | |

### Example 2

### Vectors containing Zea mays AHASL mutant genes

The *Zea mays* AHASL2 gene (SEQ ID NO: 29) was cloned into the bacterial expression vector pTrcHis A (Invitrogen Corporation, Carlsbad, CA), fused to the vector tag and translational start site, beginning with base 160 of SEQ ID NO: 29. Mutagenesis and subcloning procedures were utilized to create vectors ZE2, ZE5, ZE6, and ZE7 using ZE1 as a base vector. Subcloning procedures were used to make ZE3 from ZE1, which is the maize AHASL2 gene fused to the vector tag and translational start site of pTrcHis A, beginning with base 121 of SEQ ID NO: 29. Since no functional difference was noted in *E. coli* between ZE1 or ZE3, standard mutagenesis and subcloning procedures were utilized to create vectors ZE4 and ZE8 through ZE22 using ZE3 as a base vector.

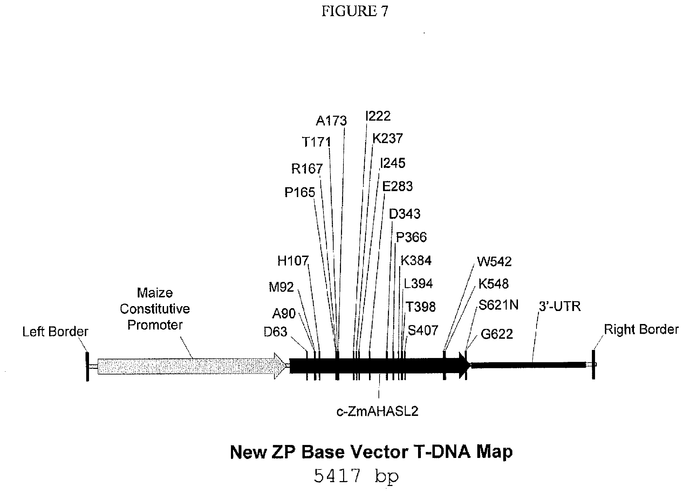

A plant transformation vector with an expression cassette comprising the maize ubiquitin promoter in combination with a polynucleotide encoding the maize AHASL2 S653N mutant was prepared using standard methods and designated ZP1 (Figure 7). To produce plant transformation vectors for expression of the other AHASL mutants, standard cloning techniques were used to replace polynucleotide segments of ZP1 with polynucleotide fragments of the ZE vectors encoding the mutations.

Vectors ZE23 through ZE38 were made as follows. Vector ZE3 was subjected to saturating site directed mutagenesis using the QuikChange® Multi Site Directed Mutagenesis Kit (Stratagene, La Jolla, California) following the "General Guidelines for Creating Engineered Mutant Clone™ Collections" appendix protocol. Mutagenic oligonucleotides that would generate all possible codons at the critical sites of the maize AHAS large subunit were used in various combinations to create a collection of mutants with substitutions at residues A90, M92, P165, R167, S621, and G622. The mutant collection plasmids were transformed into AHAS deficient *E. coli* and plated on LB agar medium supplemented with 100 ug per liter of carbenicillin. Colonies from this were picked into M9 salts at 1x concentration (for an isotonic buffer) in microtiter plates and then replica plated on minimal agar medium without branched chain amino acids and containing 150 micromolar imazethapyr. Those colonies that grew on this selective media possessed a functional maize AHAS mutant gene that was also imidazolinone tolerant.

The DNA sequence of the maize AHAS large subunit gene was determined for each of the growth positive colonies. No effort was made to determine the sequence of the maize AHAS large subunit genes that did not confer growth on the selective media.

**Table 2**

| *E. coli* Plasmid* | Maize Transformation Vector* | Mutations* | *E. coli* Imidazolinone Tolerance Score** | Maize Whole Plant Tolerance Score^{@} |
|---|---|---|---|---|
| ZE1 | - | S621N | + | NA |
| ZE2 | - | wild type | - (NT) | NA |
| ZE3 | - | wild type | - (NT) | NA |
| ZE4 | ZP1 | S621N | + | + |
| ZE5 | - | W542L, S621N | - (IN) | NA |
| ZE6 | ZP4 | P165S, S621N | + | + |
| ZE7 | - | W542L | - (NT) | NA |
| ZE8 | - | M92E, S621N | - (IN) | NA |
| ZE9 | ZP5 | R167S, S621N | +++ | +++ |
| ZE10 | ZP2 | A90T, S621N | +++ | +++ |
| ZE11 | ZP3 | A90T, R167S, S621N | +++ | +++ |
| ZE12 | ZP9 | M92I, S621N | +++ | +++ |
| ZE13 | - | R167A, S621N | NA | NA |
| ZE14 | - | A173V, S621N | ++ | NA |
| ZE15 | ZP8 | A90T, M92I | +++ | +++ |
| ZE16 | ZP10 | A90T, M92E | NA | NA |
| ZE17 | ZP6 | A90T, R167A | +++ | +++ |
| ZE18 | | P165S | - (NT) | NA |
| ZE19 | - | P165S, R167A | - (NT) | NA |
| ZE20 | - | T171I, S621N | + | NA |
| ZE21 | ZP7 | A90T | ++ | +++ |
| ZE22 | - | A90T, P165S | +++ | NA |
| ZE23 | ZP11 | A90Q | +++ | +++ |
| ZE24 | ZP12 | A90Q, M92L | ++ | +++ |
| ZE25 | - | A90Q, M92I | +++ | NA |
| ZE26 | - | A90C | ++ | NA |
| ZE27 | - | A90M, M92I | + | NA |
| ZE28 | - | P165E, R167F | - (NT) | NA |
| ZE29 | - | P165V, R167A | - (NT) | NA |
| ZE30 | - | P165E, R167T | + | + |
| ZE31 | - | P165I, R167D | - (IN) | NA |
| ZE32 | - | P165I, R167E | + | NA |
| ZE3 | - | M92I, P165E, R167A | - (NT) | NA |
| ZE34 | - | A90M, P165R, R167C | - (IN) | NA |
| ZE35 | - | M92N, S621G | +++ | NA |
| ZE36 | - | P165S, R167N, S621V, G622D | +++ | NA |
| ZE37 | - | S621W | +++ | NA |
| ZE38 | - | P165S, R167C, W542M | +++ | NA |

| | | | | |
|---|---|---|---|---|
| * List of vectors for expression of ZmAHASL2 in E. coli (ZE plasmids) and for plant transformation plasmids (ZP plasmids). Mutations in each vector are indicated relative to SEQ ID NO: 2. ** A simple single, double or triple plus system, +, ++, or +++ for respectively increasing colony size, was used to visually score the tolerance of the maize AHAS function in AHAS minus E. coli containing the ZE plasmids in the presence of the AHAS inhibitor imazethapyr. A "-", indicates there was no growth, meaning the mutation combination caused an inactive protein or there was no tolerance for imazethapyr at the selected rate. IN means inactive protein, while NT means not imidazolinone tolerant. NA means no data available (not tested). ^{@} The maize whole plant tolerance scores are based on combined results from tests conducted in the greenhouse and at multiple field sites over several growing seasons. The scoring system for the maize whole plant tolerance was the same as described above for the *E. coli* imidazolinone tolerance. Note that all ZP constructs with +++ scores are tolerant to more than three thousand grams imazamox per hectare, which represents the highest tested spray rate. | | | | |

### Example 3

### E. coli Complementation Assay

*E. coli* strain JMC1 (genotype [*ilvB1201 ilvHI2202 rbs-221 ara thi delta(pro-lac) recA56 sr1C300::Tn10*]*, DE(hsdR)::Cat*) is a knockout for all copies of *ilvG* of the native *E. coli* AHASL gene. This strain can only grow on minimal growth medium lacking leucine, isoleucine, and valine if AHASL is complemented by an exogenous AHASL gene (see Singh, et al. (1992) Plant Physiol. 99, 812-816; Smith, et al. (1989) Proc. Natl. Acad. Sci. USA 86, 4179-4183). This *E. coli* complementation assay was used to screen for AHASL enzyme activity and herbicide tolerance encoded by the AE and ZE vectors in the absence and presence of the imidazolinone herbicide Pursuit^{®} (imazethapyr, BASF Corporation, Florham Park, NJ).

### Example 4

### Biochemical Characterization

Based on growth during complementation testing or simple activity tests, certain of the ZE series of vectors were used for AHAS biochemical assay inhibition testing in crude E. coli lysates. A 2-4 ml culture of LB containing 50 µg/ml carbenicillin (LB-carb) was inoculated with a single colony of JMC1 transformed with the ZE vector to be tested and incubated overnight at 37°C with shaking. The following morning, 0.5-1 ml of overnight culture was used to inoculate 20 ml of LB-carb, which was incubated at 37°C with shaking until the culture optical density (OD) at 600 nm was approximately 0.6 to 0.8 OD units. Isopropyl-1-thio-beta D-galactopyranoside (IPTG) was added to a concentration of 0.1 mM and the cultures incubated with shaking for 1-1.5 hours. The culture was centrifuged to pellet the cells and the supernatant discarded. The cell pellet was lysed with AHAS assay buffer (as in Singh et al. (1988) Anal. Biochem. 171:173-179) supplemented with 10 mg/ml lysozyme and subjected to brief sonication. The insoluble fraction was pelleted by centrifugation and the supernatant used in an assay for AHAS activity. At each concentration of imazethapyr inhibitor used, the activity was compared to an uninhibited control of the same ZE mutant. This results in a "percent of control" measurement.

### Example 5

### Plant Transformation

The AP vectors were transformed into *A. thaliana* ecotype Col-2. The T1 seeds were selected for transformation on plates with 100 nM Pursuit^{®} as the selective agent. T2 seeds from approximately twenty independent transformation events (lines) were plated on MS agar with increasing Pursuit^{®} concentrations, to score increases in tolerance compared to AP 1. The vectors were scored by comparison of the highest concentrations of Pursuit^{®} having uninhibited growth of seedlings by visual examination. The results of the *Arabidopsis* transformation experiments are shown in Table 1.

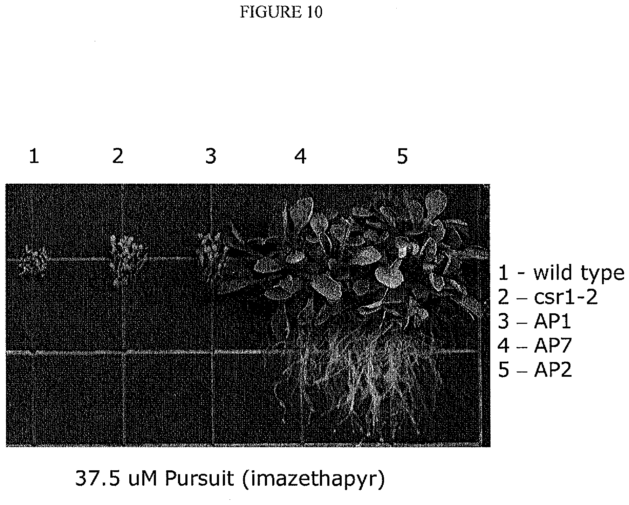

Seeds from several lines of *Arabidopsis* were tested by a vertical plate growth assay. A plate with standard Murashige and Skoog semisolid media containing 37.5 micromolar Pursuit (imazethapyr) was spotted with several seeds in 0.1% agarose. The plate was held vertically, so that the roots would grow along the agar surface. The seeds used were: 1) wild type ecotype Columbia 2; 2) the csrl-2 mutant (homozygous for the AtAHASL S653N mutation in the genomic copy of the AHAS large subunit gene); 3) Columbia 2 transformed with AP1; 4) Columbia 2 transformed with AP7; 5) Columbia 2 transformed with AP2. Note that numbers 2 and 3 are roughly equivalent in terms of probable tolerance, as the AP1 plants are transformed with a clone of the genomic XbaI fragment of csrl-2 (SEQ ID NO: 34). At this concentration of imazethapyr, the wild type seedlings failed to germinate, the single mutant plants (csrl-2 and AP1 transformants) barely germinated. AP7 and AP2 produced good tolerant growth, although the AP7 plants appear to have slightly less root growth. Note that all lines germinated and grew well on media without imazethapyr. The results of the vertical plate growth assay are depicted in Figure 10.

ZP constructs were introduced into maize immature embryos via *Agrobacterium*-mediated transformation. Transformed cells were selected on selection media supplemented with 0.75 µM Pursuit^{®} for 3-4 weeks. Transgenic plantlets were regenerated on plant regeneration media supplemented with 0.75 µM Pursuit^{®}. Transgenic plantlets were rooted in the presence of 0.5 µM Pursuit^{®}. Transgenic plants were subjected to TaqMan analysis for the presence of the transgene before being transplanted to potting mixture and grown to maturity in greenhouse. The results of the maize transformation experiments are shown in Table 2. Maize plants transformed with the ZP constructs were sprayed with varying rates of imazamox, in several field locations and in the greenhouse. The relative ratings of the ZP constructs' whole plant test data are summarized in Table 2.

### Example 6

### Expression of AtAHASL Mutant Genes in Soybean

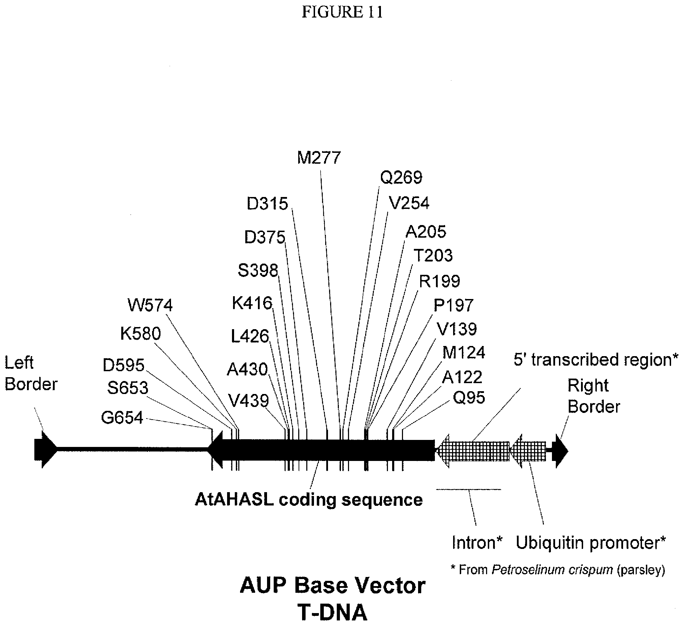

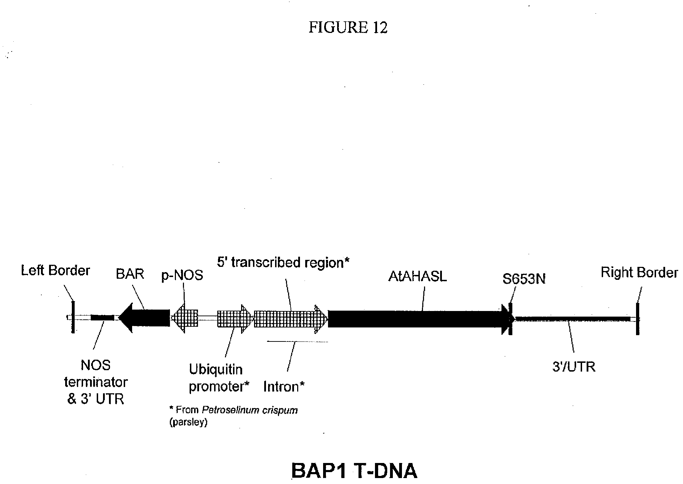

Vectors were prepared for expressing the AtAHASL genes in transformed soybean plants. Vectors AUP2 and AUP3 were made by cloning a polymerase chain reaction produce of the parsley ubiquitin promoter, amplified to incorporate sites for the Asp718 and NcoI restriction enzymes, digested and ligated into the same sites of AP2 and AP3 by standard cloning techniques (see, Figures 11 and 12). AUP2 encodes an AtAHASL protein with the A122T and S653N mutations, and AUP3 encodes an AtAHASL protein with the A122T and S653N mutations. Vector BAP1 was made by cloning the entire promoter, coding sequence and 3'-untranslated region sequence of AP1 into a standard dicot transformation backbone containing the BAR selectable marker expression cassette, by standard blunt-ended cloning techniques.

Constructs AP2, AUP2, and AUP3 were introduced into soybean's axillary meristem cells at the primary node of seedling explants via *Agrobacterium*-mediated transformation. After inoculation and co-cultivation with *Agrobacteria*, the explants were transferred to shoot induction media without selection for one week. The explants were subsequently transferred to a shoot induction medium with 5 µM imazapyr (Arsenal) for 3 weeks to select for transformed cells. Explants with healthy callus/shoot pads at the primary node were then transferred to shoot elongation medium containing 3 µM imazapyr until a shoot elongated or the explant died. Transgenic plantlets were rooted, transplanted to potting mixture, subjected to TaqMan analysis for the presence of the transgene, and then grown to maturity in greenhouse. Construct BAP1 was used to produce transformed soybean plants in a like manner, except that the selection agent was BASTA.

The transformed soybean plants were tested for herbicide tolerance in both greenhouse and field studies. For the field study, imazapyr was applied at a rate of 300 g ai/ha at the V3 stage. For the greenhouse study, imazapyr was applied at about the V2 stage. The results of these studies are summarized in Table 3.

**Table 3**

| Transformation Vector, Native *Arabidopsis* promoter | Transformation Vector, Parsley Ubiquitin promoter | Mutations | Maximum Greenhouse Tolerance (grams Imazapyr per hectare) | Field Tolerance (grams Imazapyr per hectare) |
|---|---|---|---|---|
| | BAP1* | S653N | 500 | NA |
| AP2 | AUP2 | A122T & S653N | AP2 - 1000, AUP2 - 1500 | AP2 - NA AUP2 - 300 |
| - | AUP3 | P197S & S653N | NA | 300** |

| | | | | |
|---|---|---|---|---|
| * BAR1 (figure 12) was transformed using the BAR gene for selection, as imazapyr selection in soybeans with the S653N mutation alone has not been optimized. ** Some injury compared to AUP2 | | | | |

### Example 7

### Transformant Selection

The polynucleotides generated by the invention may be used as selectable markers for plant transformation. The polynucleotides generated by the invention may be used as selectable markers to identify and/or select transformed plants which may comprise additional genes of interest. Plants or plant cells transformed with vectors containing the multiple mutant forms of the AHAS large subunit genes can be selected from non-transformed plants or plant cells by plating on minimal media, such as MS media, which incorporate AHAS inhibitors or AHAS inhibiting herbicides, such as imidazolinones. The transformed plants or tissues will be able to continue growing in the presence of these inhibitors, while the untransformed plants or tissues will die. In the case of transformed tissues, since the non-transformed tissues may receive branched chain amino acids from the transformed tissues, the actively growing tissues are removed from the slower growing or dying tissues and replated on selective media.

Whole plants may also be selected by planting the seeds and waiting for germination and seedling growth, followed by spraying the seedlings with AHAS inhibitors or AHAS inhibiting herbicides, such as imidazolinones. The transformed plants will survive while the untransformed plants will be killed.

### Example 8

### Field Trials with Transformed Maize

Field trials were conducted to assess whether or not maize plants transformed with one of the vectors comprising AHASL double and triple mutants displayed any gross physiological or reproductive affects with and without an imazamox application.

### Materials and Methods

### Source of Test Material

The genetically modified organism was produced by transforming corn inbred J553. F1 hybrid seed from 8 vector constructs were produced using TR5753 as an inbred male tester. The vector constructs are described in Table 4. Seed for the trial were produced in an isolated crossing block on the island of Kauai, Hawaii. USA during the 2006-2007 contraseason. Subsamples of each F1 hybrid produced were analyzed for the presence of the correct vector construct and absence of adventitious presence of other AHASL contructs.

Nontransformed commercial hybrids were purchased from Midwest USA corn seed companies and analyzed to confirm the absence of any adventitious presence of other AHASL contructs.

**Table 4**

| Construct | pZm UBI + I::c-ZmAHAS L2::t-ZmAHAS L2 Mutations (at Designation) |
|---|---|
| 1 | P197S |
| 2 | A122T, R199A |
| 3 | A122T |
| 4 | A122T, M124I |
| 5 | M124I, S653N |
| 6 | A122T, S653N |
| 7 | A122T, R199S, S653N |
| 8 | S653N |

### Trial Methodology

Trial design was a Split Plot in a Randomized Complete Block Design, with the main plot being an herbicide treatment, and the sub plot being an F1 hybrid entry. The herbicide treatments included 1) untreated and 2) imazamox applied at 150 g ai/ha. The F1 hybrid entries included 29 events from 8 vector constructs and 4 non-transformed commercial hybrids (3395IR from Pioneer Hi-Bred International, Inc, Johnston, IA, USA and 8342GLS/IT, 8546IT, and 8590IT from Garst Seed Co., Inc., Slater, IA. USA). Plot size was 2 rows; row width 2.5 feet; row length 20 feet. Each treatment combination had 4 replications. The trial was planted at three locations. These locations were: Ames, IA, ISA; Estherville, IA, USA; and York, NE, USA. All trials were planted during May 2007.

### Location conditions

The Ames, IA location was in a corn-after-corn rotation which may have had some impact on uniformity of emergence and early growth as significant amounts of corn residue were present at planting. No major influences on the crop due to weather, disease or insects were noted. The Estherville, IA site received heavy rain driven by 70 mph wind gusts and sustained winds of around 40 mph on July 16. Root lodging was observed in essentially every plot. No major influences on the crop due to disease or insects were noted. The York, NE site received above-normal rainfall in May, July and August and no major insect or disease issues were noted.

### Results and Discussion

Data analyses combined across three locations resulted in one vector construct with a significant yield decrease (p ≤ 0.05) when comparing yield with or without the imazamox herbicide application (Table 5). One vector construct had a significant yield increase when treated with the imazamox application. Other agronomic characteristics were also collected from the three trial locations, and no significant differences were detected within a construct when treated or untreated with imazamox for the traits plant height, ear height, stalk lodging and root lodging (data not shown).

The objective of the trial was to identify if an herbicide application of imazamox applied to vector constructs that have been optimized to provide improved herbicide tolerance to imazamox would result in gross, or obvious, physiological or reproductive affects, primarily yield. Only one vector construct (Construct 1, single mutant, P197L) had a significant (p ≤ 0.05) negative response for grain yield when treated with imazamox. The remaining seven vector constructs exhibited no adverse physiological or reproductive affects in the presence or absence of the herbicide imazamox. The results of these field trials demonstrate the excellent agronomic potential of maize plants transformed with a vector comprising either an AHASL double or triple mutant.

**Table 5**

| | | Yield (bu/A) | | | | | |
|---|---|---|---|---|---|---|---|
| Description | | Herbicide (H) | | Non-herbicide (NH) | | | |
| Construct | # Event | Mean | α= 0.05 | Mean | α= 0.05 | (H/NH)% | p-value |
| 1 | 4 | 160.68 | B | 175.58 | BC | 91.51 | 0 |
| 2 | 4 | 176.72 | AB | 177.05 | ABC | 99.81 | 0.94 |
| 3 | 4 | 180.59 | A | 179.02 | AB | 100.87 | 0.74 |
| 4 | 4 | 173.57 | AB | 163.79 | C | 105.97 | 0.11 |
| 5 | 4 | 173.87 | AB | 180.84 | AB | 96.14 | 0.10 |
| 6 | 4 | 188.02 | A | 178.65 | AB | 105.24 | 0.04 |
| 7 | 1 | 187.45 | AB | 189.03 | AB | 99.16 | 0.88 |
| 8 | 4 | 183.14 | A | 177.54 | ABC | 103.15 | 0.27 |
| 3395IR | | 168.03 | AB | 176.89 | ABC | 94.99 | 0.09 |
| 8590IT | | 181.92 | AB | 197.57 | A | 92.08 | 0.04 |
| G8546IT | | 174.94 | AB | 186.74 | AB | 93.68 | 0.40 |
| G8342GLS/IT | | 180.14 | AB | 177.53 | ABC | 101.47 | 0.84 |

All publications and patent applications mentioned in the specification are indicative of the level of those skilled in the art to which this invention pertains.

## Claims

1. A polynucleotide encoding an acetohydroxyacid synthase large subunit (AHASL) amino acid-substituted polypeptide having a valine, threonine, glutamine, cysteine or methionine substitution at a position corresponding to position 122 of SEQ ID NO:1 or position 90 of SEQ ID NO:2 and a glycine or tryptophan substitution at a position corresponding to position 653 of SEQ ID NO:1 or position 621 of SEQ ID NO:2.

2. The polynucleotide of claim 1 wherein said polynucleotide encodes an AHASL polypeptide having a third substitution selected from:
a) an alanine, glutamic acid, serine, phenylalanine, threonine, aspartic acid, cysteine or asparagine substitution at a position corresponding to position 199 of SEQ ID NO:1 or position 167 of SEQ ID NO:2; and
b) a serine, alanine, glutamic acid, leucine, glutamine, arginine, valine, tryptophan, tyrosine or isoleucine substitution at a position corresponding to position 197 of SEQ ID NO:1 or position 165 of SEQ ID NO:2.

3. The polynucleotide of claim 1 or 2, wherein the AHASL polypeptide has an amino acid sequence that is at least 80% identical to any one of the amino acid sequences set forth in SEQ ID NOs:1-25.

4. A polynucleotide encoding an acetohydroxyacid synthase large subunit (AHASL) amino acid-substituted polypeptide having a valine, threonine, glutamine, cysteine or methionine substitution at a position corresponding to position 122 of SEQ ID NO:1 or position 90 of SEQ ID NO:2 and a phenylalanine, asparagine, threonine, glycine, valine, isoleucine or tryptophan substitution at a position corresponding to position 653 of SEQ ID NO:1 or position 621 of SEQ ID NO:2;
wherein the AHASL polypeptide has an amino acid sequence that is at least 80% identical to any one of the amino acid sequences set forth in SEQ ID NOs:1-4 and 8-25.

5. The polynucleotide of claim 4, wherein the encoded AHASL polypeptide has a threonine substitution at a position corresponding corresponding to position 122 of SEQ ID NO:1 or position 90 of SEQ ID NO:2 and an asparagine substitution at a position corresponding to position 653 of SEQ ID NO:1 or position 621 of SEQ ID NO:2.

6. The polynucleotide of claim 4 or 5, wherein said polynucleotide encodes an AHASL polypeptide having a third substitution selected from:
a) an alanine, glutamic acid, serine, phenylalanine, threonine, aspartic acid, cysteine or asparagine substitution at a position corresponding to position 199 of SEQ ID NO:1 or position 167 of SEQ ID NO:2; and
b) a serine, alanine, glutamic acid, leucine, glutamine, arginine, valine, tryptophan, tyrosine or isoleucine substitution at a position corresponding to position 197 of SEQ ID NO:1 or position 165 of SEQ ID NO:2.

7. The polynucleotide of any one of claims 4-6, wherein the encoded AHASL polypeptide has an amino acid sequence that is at least 80% identical to any one of the amino acid sequences set forth in SEQ ID NOs:1-4, 8-20, 24 and 25.

8. The polynucleotide of any one of claims 1-7, wherein said polynucleotide is recombinant or mutagenized.

9. An expression vector comprising the polynucleotide of any one of claims 1-8 and capable of expressing said polynucleotide in a host cell.

10. A transgenic plant comprising the expression vector of claim 9.

11. The transgenic plant of claim 10, wherein the plant is a monocot.

12. The transgenic plant of claim 10, wherein the plant is a dicot.

13. The transgenic plant of claim 10, wherein the plant is selected from *Arabidopsis thaliana,* maize, rye, oat, triticale, rice, barley, sorghum, millet, sugarcane, soybean, sugar beet, peanut, cotton, rapeseed, canola, *Brassica* species, manihot, melon, squash, pepper, tagetes, solanaceous plants, potato, sweet potato, tobacco, eggplant, tomato, *Vicia* species, pea, alfalfa, coffee, cacao, tea, *Salix* species, oil palm, coconut, and perennial grass.

14. A plant seed produced by the transgenic plant of any one of claims 10-13, wherein the seed comprises the polynucleotide of any one of claims 1-8.

15. The transgenic plant of any one of claims 10-14, wherein expression of the polynucleotide in the plant results in tolerance to an herbicide selected from imidazolinones, sulfonylureas, triazolopyrimidines, and pyrimidinyloxybenzoates.

16. A purified AHASL protein encoded by the polynucleotide of any one of claims 1-8.

17. A plant comprising:
a) a first polynucleotide encoding a first AHASL polypeptide having a first amino acid substitution and a second polynucleotide encoding a second AHASL polypeptide having a second amino acid substitution, or
b) a polynucleotide encoding an AHASL polypeptide having both, said first and said second, amino acid substitutions;
said first amino acid substitution being a valine, threonine, glutamine, cysteine or methionine for the amino acid at the position corresponding to position 122 of SEQ ID NO:1 or position 90 of SEQ ID NO:2; and said second amino acid substitution being a glycine or tryptophan for the amino acid at the position corresponding to position 653 of SEQ ID NO:1 or position 621 of SEQ ID NO:2.

18. A non-transgenic plant comprising:
a) a first polynucleotide encoding a first AHASL polypeptide having a first amino acid substitution and a second polynucleotide encoding a second AHASL polypeptide having a second amino acid substitution, or
b) a polynucleotide encoding an AHASL polypeptide having both, said first and said second, amino acid substitutions;
said first amino acid substitution being a valine, threonine, glutamine, cysteine or methionine for the amino acid at the position corresponding to position 122 of SEQ ID NO: 1 or position 90 of SEQ ID NO:2; and said second amino acid substitution being a phenylalanine, asparagine, threonine, glycine, valine or tryptophan for the amino acid at the position corresponding to position 653 of SEQ ID NO:1 or position 621 of SEQ ID NO:2;
wherein said plant is selected from *Arabidopsis thaliana*, maize, wheat, rye, oat, triticale, rice, barley, sorghum, millet, sugar beet, sugarcane, soybean, peanut, cotton, rapeseed, canola, *Brassica* species, manihot, tagetes, solanaceous plants, potato, sweet potato, tobacco, eggplant, tomato, *Vicia* species, pea, alfalfa, coffee, cacao, tea, *Salix* species, oil palm, coconut, and perennial grass.

19. The plant of claim 18, wherein the first amino acid substitution is a threonine for the amino acid at the position corresponding to position 122 of SEQ ID NO: 1 or position 90 of SEQ ID NO:2, and the second substitution is an asparagine for the amino acid at the position corresponding to position 653 of SEQ ID NO:1 or position 621 of SEQ ID NO:2.

20. A plant comprising:
i) a first polynucleotide encoding a first AHASL polypeptide having a first amino acid substitution, a second polynucleotide encoding a second AHASL polypeptide having a second amino acid substitution and a third polynucleotide encoding a third AHASL polypeptide having a third amino acid substitution;
ii) a first polynucleotide encoding a first AHASL polypeptide having the first amino acid substitution and the second amino acid substitution, and a second polynucleotide encoding a second AHASL polypeptide having the third amino acid substitution;
iii) a first polynucleotide encoding a first AHASL polypeptide having the first amino acid substitution and the third amino acid substitution, and a second polynucleotide encoding a second AHASL polypeptide having the second amino acid substitution; or
iv) a polynucleotide encoding an AHASL polypeptide having the first, second and third amino acid substitutions;
wherein said first, second, and third amino acid substitutions are selected from:
a) the first amino acid substitution being a valine, threonine, glutamine, cysteine or methionine for the amino acid at the position corresponding to position 122 of SEQ ID NO:1 or position 90 of SEQ ID NO:2; the second amino acid substitution being an alanine, glutamic acid, serine, phenylalanine, threonine, aspartic acid, cysteine or asparagine for the amino acid at the position corresponding to position 199 of SEQ ID NO:1 or position 167 of SEQ ID NO:2; and the third amino acid substitution being a glycine or tryptophan for the amino acid at the position corresponding to position 653 of SEQ ID NO:1 or position 621 of SEQ ID NO:2; or
b) the first amino acid substitution being a valine, threonine, glutamine, cysteine or methionine for the amino acid at the position corresponding to position 122 of SEQ ID NO: 1 or position 90 of SEQ ID NO:2; the second amino acid substitution being a serine, alanine, glutamic acid, leucine, glutamine, arginine, valine, tryptophan, tyrosine or isoleucine for the amino acid at the position corresponding to position 197 of SEQ ID NO:1 or position 165 of SEQ ID NO:2; and the third amino acid substitution being a glycine or tryptophan for the amino acid at the position corresponding to position 653 of SEQ ID NO: 1 or position 621 of SEQ ID NO:2.

21. A non-transgenic plant comprising:
i) a first polynucleotide encoding a first AHASL polypeptide having a first amino acid substitution, and a second polynucleotide encoding a second AHASL polypeptide having a second amino acid substitution and a third amino acid substitution;
ii) a first polynucleotide encoding a first AHASL polypeptide having the first amino acid substitution and the second amino acid substitution, and a second polynucleotide encoding a second AHASL polypeptide having the third amino acid substitution;
iii) a first polynucleotide encoding a first AHASL polypeptide having the first amino acid substitution and the third amino acid substitution, and a second polynucleotide encoding a second AHASL polypeptide having the second amino acid substitution;
iv) a polynucleotide encoding an AHASL polypeptide having the first, second and third amino acid substitutions;
wherein said first, second, and third amino acid substitutions are selected from:
a) the first amino acid substitution being a valine, threonine, glutamine, cysteine or methionine for the amino acid at the position corresponding to position 122 of SEQ ID NO:1 or position 90 of SEQ ID NO:2; the second amino acid substitution being an alanine, glutamic acid, serine, phenylalanine, threonine, aspartic acid, cysteine or asparagine for the amino acid at the position corresponding to position 199 of SEQ ID NO:1 or position 167 of SEQ ID NO:2; and the third amino acid substitution being a phenylalanine, asparagine, threonine, glycine, valine or tryptophan for the amino acid at the position corresponding to position 653 of SEQ ID NO:1 or position 621 of SEQ ID NO:2; or
b) the first amino acid substitution being a valine, threonine, glutamine, cysteine or methionine for the amino acid at the position corresponding to position 122 of SEQ ID NO:1 or position 90 of SEQ ID NO:2; the second amino acid substitution being a serine, alanine, glutamic acid, leucine, glutamine, arginine, valine, tryptophan, tyrosine or isoleucine for the amino acid at the position corresponding to position 197 of SEQ ID NO:1 or position 165 of SEQ ID NO:2; and the third amino acid substitution being a phenylalanine, asparagine, threonine, glycine, valine or tryptophan for the amino acid at the position corresponding to position 653 of SEQ ID NO:1 or position 621 of SEQ ID NO:2;
wherein said plant is selected from *Arabidopsis thaliana*, maize, wheat, rye, oat, triticale, rice, barley, sorghum, millet, sugar beet, sugarcane, soybean, peanut, cotton, rapeseed, canola, *Brassica* species, manihot, tagetes, solanaceous plants, potato, sweet potato, tobacco, eggplant, tomato, *Vicia* species, pea, alfalfa, coffee, cacao, tea, *Salix* species, oil palm, coconut, and perennial grass.

22. The plant of any one of claims 18, 19 and 21, wherein said plant is selected from *Arabidopsis thaliana*, maize, rye, oat, triticale, rice, barley, sorghum, millet, sugar beet, sugarcane, soybean, peanut, cotton, rapeseed, canola, *Brassica* species, manihot, tagetes, solanaceous plants, potato, sweet potato, tobacco, eggplant, tomato, *Vicia* species, pea, alfalfa, coffee, cacao, tea, *Salix* species, oil palm, coconut, and perennial grass.

23. The plant of any one of claims 17 and 20, wherein the plant is a monocot.

24. The plant of any one of claims 17 and 20, wherein the plant is a dicot.

25. The plant of any one of claims 17-24, wherein at least one of said polynucleotide(s) is mutagenized.

26. A plant seed produced by the plant of any one of claims 17-25, wherein the seed comprises the AHASL polynucleotide(s) of the plant of any one of claims 17-25.

27. The plant of any one of claims 17 and 20, wherein the plant is selected from *Arabidopsis thaliana*, maize, wheat, rye, oat, triticale, rice, barley, sorghum, millet, sugar beet, sugarcane, soybean, peanut, cotton, rapeseed, canola, *Brassica* species, manihot, tagetes, solanaceous plants, potato, sweet potato, tobacco, eggplant, tomato, *Vicia* species, pea, alfalfa, coffee, cacao, tea, *Salix* species, oil palm, coconut, and perennial grass.

28. A method of producing a transgenic plant comprising the steps of:
i) transforming a plant cell with the expression vector of claim 9; and
ii) regenerating from the plant cell a transgenic plant that expresses the AHASL mutant polypeptide.

29. A method of identifying or selecting a plant cell, plant tissue, plant seed, plant or part thereof comprising:
i) providing a plant cell, plant tissue, plant seed, plant or part thereof, wherein said plant cell, plant tissue, plant seed, plant or part thereof comprises the polynucleotide of any one of claims 1-8 or the polynucleotide(s) of the plant of any one of claims 10-13, 15, 17-25 and 27;
ii) contacting the plant cell, plant tissue, plant seed, plant or part thereof with at least one AHAS-inhibiting compound; and
iii) determining whether the plant cell, plant tissue, plant seed, plant or part thereof is affected by the AHAS-inhibiting compound, thereby identifying or selecting the plant cell, plant tissue, plant seed, plant or part thereof.

30. A seed of the plant of any one of claims 10-13, 15, 17-25 and 27, wherein said seed is treated with an AHAS-inhibiting herbicide.

31. A method for preparing a seed for growing a plant, while controlling weeds in the vicinity of said plant, the method comprising contacting a seed of the plant of any one of claims 10-13, 15, 17-25 and 27 with an AHAS-inhibiting herbicide before sowing and/or after pregermination of the seed.

32. Use of a plant of any one of claims 10-13, 15, 17-25 and 27 in a method for controlling weeds.

33. The use according to claim 32, wherein said method for controlling weeds comprises contacting said plant and weeds in the vicinity of said plant with an effective amount of an AHAS-inhibiting herbicide to which said plant is tolerant and which would inhibit the growth of a corresponding wild-type plant, thereby controlling the weeds.

34. A method for identifying a plant according to any one of claims 10-13, 15, 17-25 and 27, said method comprising:
(A) providing biological material from a plant,
(B) performing PCR or hybridization testing of the AHASL genes in said biological material to determine if the biological material comprises the polynucleotide(s) of the plant of any one of claims 10-13, 15, 17-25 and 27, and
(C) identifying, based on the results of step (B), that the plant of step (A) is a plant according to any one of claims 10-13, 15, 17-25 and 27.

35. The method of claim 34, wherein said biological material is a plant seed.

36. Use of the plant of any one of claims 10-13, 15, 17-25 and 27 or the seed of any one of claims 14, 26 and 30 in a method for producing seed oil.

## Patentansprüche

1. Polynukleotid, das ein Aminosäure-substituiertes Acetohydroxysäure-Synthase-große Untereinheit (AHASL)-Polypeptid codiert, das eine Valin-, Threonin-, Glutamin-, Cystein- oder Methionin-Substitution an einer Position aufweist, die Position 122 von SEQ ID NO: 1 oder Position 90 von SEQ ID NO: 2 entspricht, und eine Glycin- oder Tryptophan-Substitution an einer Position, die Position 653 von SEQ ID NO: 1 oder Position 621 von SEQ ID NO: 2 entspricht.

2. Polynukleotid nach Anspruch 1, wobei das Polynukleotid ein AHASL-Polypeptid mit einer dritten Substitution codiert, die aus Folgenden ausgewählt ist:
a) einer Alanin-, Glutaminsäure-, Serin-, Phenylalanin-, Threonin-, Asparaginsäure-, Cystein- oder Asparagin-Substitution an einer Position, die Position 199 von SEQ ID NO: 1 oder Position 167 von SEQ ID NO: 2 entspricht, und
b) einer Serin-, Alanin-, Glutaminsäure-, Leucin-, Glutamin-, Arginin-, Valin-, Tryptophan-, Tyrosin- oder Isoleucin-Substitution an einer Position, die Position 197 von SEQ ID NO: 1 oder Position 165 von SEQ ID NO: 2 entspricht.

3. Polynukleotid nach Anspruch 1 oder 2, wobei das AHASL-Polypeptid eine Aminosäuresequenz besitzt, die zu mindestens 80% identisch mit einer der in den SEQ ID NO: 1-25 dargestellten Aminosäuresequenzen ist.

4. Polynukleotid, das ein Aminosäure-substituiertes Acetohydroxysäure-Synthase-große Untereinheit (AHASL)-Polypeptid codiert, das eine Valin-, Threonin-, Glutamin-, Cystein- oder Methionin-Substitution an einer Position aufweist, die Position 122 von SEQ ID NO: 1 oder Position 90 von SEQ ID NO: 2 entspricht, und eine Phenylalanin-, Asparagin-, Threonin-, Glycin-, Valin-, Isoleucin- oder Tryptophan-Substitution an einer Position, die Position 653 von SEQ ID NO: 1 oder Position 621 von SEQ ID NO: 2 entspricht,
wobei das AHASL-Polypeptid eine Aminosäuresequenz besitzt, die zu mindestens 80% identisch mit einer der in den SEQ ID NO: 1-4 und 8-25 dargestellten Aminosäuresequenzen ist.

5. Polynukleotid nach Anspruch 4, wobei das codierte AHASL-Polypeptid eine Threonin-Substitution an einer Position aufweist, die Position 122 von SEQ ID NO: 1 oder Position 90 von SEQ ID NO: 2 entspricht, und eine Asparagin-Substitution an einer Position, die Position 653 von SEQ ID NO: 1 oder Position 621 von SEQ ID NO: 2 entspricht.

6. Polynukleotid nach Anspruch 4 oder 5, wobei das Polynukleotid ein AHASL-Polypeptid mit einer dritten Substitution codiert, die aus Folgenden ausgewählt ist:
a) einer Alanin-, Glutaminsäure-, Serin-, Phenylalanin-, Threonin-, Asparaginsäure-, Cystein- oder Asparagin-Substitution an einer Position, die Position 199 von SEQ ID NO: 1 oder Position 167 von SEQ ID NO: 2 entspricht, und
b) einer Serin-, Alanin-, Glutaminsäure-, Leucin-, Glutamin-, Arginin-, Valin-, Tryptophan-, Tyrosin- oder Isoleucin-Substitution an einer Position, die Position 197 von SEQ ID NO: 1 oder Position 165 von SEQ ID NO: 2 entspricht.

7. Polynukleotid nach einem der Ansprüche 4-6, wobei das codierte AHASL-Polypeptid eine Aminosäuresequenz besitzt, die zu mindestens 80% identisch mit einer der in den SEQ ID NO: 1-4, 8-20, 24 und 25 dargestellten Aminosäuresequenzen ist.

8. Polynukleotid nach einem der Ansprüche 1-7, wobei das Polynukleotid rekombinant oder mutagenisiert ist.

9. Expressionsvektor, der das Polynukleotid nach einem der Ansprüche 1-8 umfasst und zur Expression des Polynukleotids in einer Wirtszelle fähig ist.

10. Transgene Pflanze, umfassend den Expressionsvektor nach Anspruch 9.

11. Transgene Pflanze nach Anspruch 10, wobei die Pflanze eine Monokotyledone ist.

12. Transgene Pflanze nach Anspruch 10, wobei die Pflanze eine Dikotyledone ist.

13. Transgene Pflanze nach Anspruch 10, wobei die Pflanze aus *Arabidopsis thaliana,* Mais, Roggen, Hafer, Triticale, Reis, Gerste, Sorghum, Hirse, Zuckerrohr, Sojabohne, Zuckerrübe, Erdnuss, Baumwolle, Raps, Canola, *Brassica-Spezies,* Maniok, Melone, Kürbis, Pfeffer, Tagetes, Solanaceen, Kartoffel, Süßkartoffel, Tabak, Aubergine, Tomate, *Vicia*-Spezies, Erbse, Alfalfa, Kaffee, Kakao, Tee, *Salix*-Spezies, Ölpalme, Kokosnuss und mehrjährigem Gras ausgewählt ist.

14. Pflanzensamen, der von der transgenen Pflanze nach einem der Ansprüche 10-13 produziert wird, wobei der Samen das Polynukleotid nach einem der Ansprüche 1-8 umfasst.

15. Transgene Pflanze nach einem der Ansprüche 10-14, wobei die Expression des Polynukleotids in der Pflanze zu Toleranz gegenüber einem Herbizid führt, das aus Imidazolinonen, Sulfonylharnstoffen, Triazolopyrimidinen und Pyrimidinyloxybenzoaten ausgewählt ist.

16. Gereinigtes AHASL-Protein, das von dem Polynukleotid nach einem der Ansprüche 1-8 codiert wird.

17. Pflanze, umfassend:
a) ein erstes Polynukleotid, das ein erstes AHASL-Polypeptid mit einer ersten Aminosäuresubstitution codiert, und ein zweites Polynukleotid, das ein zweites AHASL-Polypeptid mit einer zweiten Aminosäuresubstitution codiert, oder
b) ein Polynukleotid, das ein AHASL-Polypeptid mit sowohl der ersten als auch der zweiten Aminosäuresubstitution codiert,
wobei die erste Aminosäuresubstitution ein Valin, Threonin, Glutamin, Cystein oder Methionin für die Aminosäure an der Position ist, die Position 122 von SEQ ID NO : 1 oder Position 90 von SEQ ID NO: 2 entspricht, und die zweite Aminosäuresubstitution ein Glycin oder Tryptophan für die Aminosäure an der Position ist, die Position 653 von SEQ ID NO: 1 oder Position 621 von SEQ ID NO: 2 entspricht.

18. Nicht-transgene Pflanze, umfassend:
a) ein erstes Polynukleotid, das ein erstes AHASL-Polypeptid mit einer ersten Aminosäuresubstitution codiert, und ein zweites Polynukleotid, das ein zweites AHASL-Polypeptid mit einer zweiten Aminosäuresubstitution codiert, oder
b) ein Polynukleotid, das ein AHASL-Polypeptid mit sowohl der ersten als auch der zweiten Aminosäuresubstitution codiert,
wobei die erste Aminosäuresubstitution ein Valin, Threonin, Glutamin, Cystein oder Methionin für die Aminosäure an der Position ist, die Position 122 von SEQ ID NO : 1 oder Position 90 von SEQ ID NO: 2 entspricht, und die zweite Aminosäuresubstitution ein Phenylalanin, Asparagin, Threonin, Glycin, Valin oder Tryptophan für die Aminosäure an der Position ist, die Position 653 von SEQ ID NO: 1 oder Position 621 von SEQ ID NO: 2 entspricht;
wobei die Pflanze aus *Arabidopsis thaliana*, Mais, Weizen, Roggen, Hafer, Triticale, Reis, Gerste, Sorghum, Hirse, Zuckerrübe, Zuckerrohr, Sojabohne, Erdnuss, Baumwolle, Raps, Canola, *Brassica-*Spezies*,* Maniok, Tagetes, Solanaceen, Kartoffel, Süßkartoffel, Tabak, Aubergine, Tomate, *Vicia*-Spezies, Erbse, Alfalfa, Kaffee, Kakao, Tee, *Salix*-Spezies, Ölpalme, Kokosnuss und mehrjährigem Gras ausgewählt ist.

19. Pflanze nach Anspruch 18, wobei die erste Aminosäuresubstitution ein Threonin für die Aminosäure an der Position ist, die Position 122 von SEQ ID NO : 1 oder Position 90 von SEQ ID NO: 2 entspricht, und die zweite Aminosäuresubstitution ein Asparagin für die Aminosäure an der Position ist, die Position 653 von SEQ ID NO: 1 oder Position 621 von SEQ ID NO: 2 entspricht.

20. Pflanze, umfassend:
i) ein erstes Polynukleotid, das ein erstes AHASL-Polypeptid mit einer ersten Aminosäuresubstitution codiert, ein zweites Polynukleotid, das ein zweites AHASL-Polypeptid mit einer zweiten Aminosäuresubstitution codiert, und ein drittes Polynukleotid, das ein drittes AHASL-Polypeptid mit einer dritten Aminosäuresubstitution codiert;
ii) ein erstes Polynukleotid, das ein erstes AHASL-Polypeptid mit der ersten Aminosäuresubstitution und der zweiten Aminosäuresubstitution codiert, und ein zweites Polynukleotid, das ein zweites AHASL-Polypeptid mit der dritten Aminosäuresubstitution codiert;
iii) ein erstes Polynukleotid, das ein erstes AHASL-Polypeptid mit der ersten Aminosäuresubstitution und der dritten Aminosäuresubstitution codiert, und ein zweites Polynukleotid, das ein zweites AHASL-Polypeptid mit der zweiten Aminosäuresubstitution codiert; oder
iv) ein Polynukleotid, das ein AHASL-Polypeptid mit der ersten, der zweiten und der dritten Aminosäuresubstitution codiert;
wobei die erste, zweite und dritte Aminosäuresubstitution aus den Folgenden ausgewählt sind:
a) die erste Aminosäuresubstitution ein Valin, Threonin, Glutamin, Cystein oder Methionin für die Aminosäure an der Position ist, die Position 122 von SEQ ID NO: 1 oder Position 90 von SEQ ID NO: 2 entspricht; die zweite Aminosäuresubstitution Alanin, Glutaminsäure, Serin, Phenylalanin, Threonin, Asparaginsäure, Cystein oder Asparagin für die Aminosäure an der Position ist, die Position 199 von SEQ ID NO: 1 oder Position 167 von SEQ ID NO: 2 entspricht, und die dritte Aminosäuresubstitution ein Glycin oder Tryptophan für die Aminosäure an der Position ist, die Position 653 von SEQ ID NO: 1 oder Position 621 von SEQ ID NO: 2 entspricht; oder
b) die erste Aminosäuresubstitution ein Valin, Threonin, Glutamin, Cystein oder Methionin für die Aminosäure an der Position ist, die Position 122 von SEQ ID NO: 1 oder Position 90 von SEQ ID NO: 2 entspricht, die zweite Aminosäuresubstitution Serin, Alanin, Glutaminsäure, Leucin, Glutamin, Arginin, Valin, Tryptophan, Tyrosin oder Isoleucin für die Aminosäure an der Position ist, die Position 197 von SEQ ID NO: 1 oder Position 165 von SEQ ID NO: 2 entspricht, und die dritte Aminosäuresubstitution ein Glycin oder Tryptophan für die Aminosäure an der Position ist, die Position 653 von SEQ ID NO: 1 oder Position 621 von SEQ ID NO: 2 entspricht.

21. Nicht-transgene Pflanze, umfassend:
i) ein erstes Polynukleotid, das ein erstes AHASL-Polypeptid mit einer ersten Aminosäuresubstitution codiert, und ein zweites Polynukleotid, das ein zweites AHASL-Polypeptid mit einer zweiten Aminosäuresubstitution und einer dritten Aminosäuresubstitution codiert;
ii) ein erstes Polynukleotid, das ein erstes AHASL-Polypeptid mit der ersten Aminosäuresubstitution und der zweiten Aminosäuresubstitution codiert, und ein zweites Polynukleotid, das ein zweites AHASL-Polypeptid mit der dritten Aminosäuresubstitution codiert;
iii) ein erstes Polynukleotid, das ein erstes AHASL-Polypeptid mit der ersten Aminosäuresubstitution und der dritten Aminosäuresubstitution codiert, und ein zweites Polynukleotid, das ein zweites AHASL-Polypeptid mit der zweiten Aminosäuresubstitution codiert;
iv) ein Polynukleotid, das ein AHASL-Polypeptid mit der ersten, der zweiten und der dritten Aminosäuresubstitution codiert;
wobei die erste, zweite und dritte Aminosäuresubstitution aus den Folgenden ausgewählt sind:
a) die erste Aminosäuresubstitution ein Valin, Threonin, Glutamin, Cystein oder Methionin für die Aminosäure an der Position ist, die Position 122 von SEQ ID NO: 1 oder Position 90 von SEQ ID NO: 2 entspricht; die zweite Aminosäuresubstitution Alanin, Glutaminsäure, Serin, Phenylalanin, Threonin, Asparaginsäure, Cystein oder Asparagin für die Aminosäure an der Position ist, die Position 199 von SEQ ID NO: 1 oder Position 167 von SEQ ID NO: 2 entspricht, und die dritte Aminosäuresubstitution ein Phenylalanin, Asparagin, Threonin, Glycin, Valin oder Tryptophan für die Aminosäure an der Position ist, die Position 653 von SEQ ID NO: 1 oder Position 621 von SEQ ID NO: 2 entspricht; oder
b) die erste Aminosäuresubstitution ein Valin, Threonin, Glutamin, Cystein oder Methionin für die Aminosäure an der Position ist, die Position 122 von SEQ ID NO: 1 oder Position 90 von SEQ ID NO: 2 entspricht, die zweite Aminosäuresubstitution Serin, Alanin, Glutaminsäure, Leucin, Glutamin, Arginin, Valin, Tryptophan, Tyrosin oder Isoleucin für die Aminosäure an der Position ist, die Position 197 von SEQ ID NO: 1 oder Position 165 von SEQ ID NO: 2 entspricht, und die dritte Aminosäuresubstitution ein Phenylalanin, Asparagin, Threonin, Glycin, Valin oder Tryptophan für die Aminosäure an der Position ist, die Position 653 von SEQ ID NO: 1 oder Position 621 von SEQ ID NO: 2 entspricht,
wobei die Pflanze aus *Arabidopsis thaliana*, Mais, Weizen, Roggen, Hafer, Triticale, Reis, Gerste, Sorghum, Hirse, Zuckerrübe, Zuckerrohr, Sojabohne, Erdnuss, Baumwolle, Raps, Canola, *Brassica*-Spezies, Maniok, Tagetes, Solanaceen, Kartoffel, Süßkartoffel, Tabak, Aubergine, Tomate, *Vicia*-Spezies, Erbse, Alfalfa, Kaffee, Kakao, Tee, *Salix*-Spezies, Ölpalme, Kokosnuss und mehrjährigem Gras ausgewählt ist.

22. Pflanze nach einem der Ansprüche 18, 19 und 21, wobei die Pflanze aus *Arabidopsis thaliana*, Mais, Roggen, Hafer, Triticale, Reis, Gerste, Sorghum, Hirse, Zuckerrübe, Zuckerrohr, Sojabohne, Erdnuss, Baumwolle, Raps, Canola, *Brassica*-Spezies, Maniok, Tagetes, Solanaceen, Kartoffel, Süßkartoffel, Tabak, Aubergine, Tomate, *Vicia*-Spezies, Erbse, Alfalfa, Kaffee, Kakao, Tee, *Salix*-Spezies, Ölpalme, Kokosnuss und mehrjährigem Gras ausgewählt ist.

23. Pflanze nach einem der Ansprüche 17 und 20, wobei die Pflanze eine Monokotyledone ist.

24. Pflanze nach einem der Ansprüche 17 und 20, wobei die Pflanze eine Dikotyledone ist.

25. Pflanze nach einem der Ansprüche 17-24, wobei mindestens eines des/der Polynukleotid(e) mutagenisiert ist.

26. Pflanzensamen, der von der Pflanze nach einem der Ansprüche 17-25 produziert wird, wobei der Samen das/die AHASL-Polynukleotid(e) der Pflanze nach einem der Ansprüche 17-25 umfasst.

27. Pflanze nach einem der Ansprüche 17 und 20, wobei die Pflanze aus *Arabidopsis thaliana*, Mais, Weizen, Roggen, Hafer, Triticale, Reis, Gerste, Sorghum, Hirse, Zuckerrübe, Zuckerrohr, Sojabohne, Erdnuss, Baumwolle, Raps, Canola, Brassica-Spezies, Maniok, Tagetes, Solanaceen, Kartoffel, Süßkartoffel, Tabak, Aubergine, Tomate, *Vicia*-Spezies, Erbse, Alfalfa, Kaffee, Kakao, Tee, *Salix*-Spezies, Ölpalme, Kokosnuss und mehrjährigem Gras ausgewählt ist.

28. Verfahren zur Herstellung einer transgenen Pflanze, umfassend die folgenden Schritte:
i) Transformieren einer Pflanzenzelle mit dem Expressionsvektor nach Anspruch 9 und
ii) Regenerieren einer transgenen Pflanze, die das mutierte AHASL-Polypeptid exprimiert, aus der Pflanzenzelle.

29. Verfahren zum Identifizieren oder Selektieren einer Pflanzenzelle, eines Pflanzengewebes, eines Pflanzensamens, einer Pflanze oder eines Teils davon, das Folgendes umfasst:
i) Bereitstellen einer Pflanzenzelle, eines Pflanzengewebes, eines Pflanzensamens, einer Pflanze oder eines Teils davon, wobei die Pflanzenzelle, das Pflanzengewebe, der Pflanzensamen, die Pflanze oder der Teil davon das Polynukleotid nach einem der Ansprüche 1-8 oder das/die Polynukleotid(e) der Pflanze nach einem der Ansprüche 10-13, 15, 17-25 und 27 umfasst;
ii) Inkontaktbringen der Pflanzenzelle, des Pflanzengewebes, des Pflanzensamens, der Pflanze oder des Teils davon mit mindestens einer AHAS-inhibierenden Verbindung und
iii) Bestimmen, ob die Pflanzenzelle, das Pflanzengewebe, der Pflanzensamen, die Pflanze oder der Teil davon durch die AHAS-inhibierende Verbindung beeinflusst wird, wodurch die Pflanzenzelle, das Pflanzengewebe, der Pflanzensamen, die Pflanze oder der Teil davon identifiziert oder selektiert wird.

30. Samen der Pflanze nach einem der Ansprüche 10-13, 15, 17-25 und 27, wobei der Samen mit einem AHAS-inhibierenden Herbizid behandelt wird.

31. Verfahren zur Vorbereitung eines Samens zum Züchten einer Pflanze, während Unkräuter in der Nähe der Pflanze bekämpft werden, wobei das Verfahren das Inkontaktbringen eines Samens der Pflanze nach einem der Ansprüche 10-13, 15, 17-25 und 27 mit einem AHAS-inhibierenden Herbizid vor der Aussaat und/oder nach der Vorkeimung des Samens umfasst.

32. Verwendung einer Pflanze nach einem der Ansprüche 10-13, 15, 17-25 und 27 bei einem Verfahren zur Bekämpfung von Unkräutern.

33. Verwendung nach Anspruch 32, wobei das Verfahren zur Bekämpfung von Unkräutern das Inkontaktbringen der Pflanze und von Unkräutern in der Nähe der Pflanze mit einer wirksamen Mengen eines AHAS-inhibierenden Herbizids, gegenüber dem die Pflanze tolerant ist und welches das Wachstum einer entsprechenden Wildtyp-Pflanze hemmen würden, umfasst, wodurch die Unkräuter bekämpft werden.

34. Verfahren zu Identifizieren einer Pflanze nach einem der Ansprüche 10-13, 15, 17-25 und 27, wobei das Verfahren Folgendes umfasst:
(A) Bereitstellen von biologischem Material von einer Pflanze,
(B) Durchführen von PCR- oder Hybridisierungstests der AHASL-Gene in dem biologischen Material, um zu bestimmen, ob das biologische Material das/die Polynukleotid(e) der Pflanze nach einem der Ansprüche 10-13, 15, 17-25 und 27 umfasst, und
(C) Identifizieren auf der Basis der Ergebnisse von Schritt (B), dass die Pflanze von Schritt (A) eine Pflanze nach einem der Ansprüche 10-13, 15, 17-25 und 27 ist.

35. Verfahren nach Anspruch 34, wobei es sich bei dem biologischen Material um einen Pflanzensamen handelt.

36. Verwendung der Pflanze nach einem der Ansprüche 10-13, 15, 17-25 und 27 oder des Samens nach einem der Ansprüche 14, 26 und 30 bei einem Verfahren zur Herstellung von Samenöl.

## Revendications

1. Polynucléotide codant pour un polypeptide à substitution d'acides aminés de la grande sous-unité de l'acétohydroxyacide synthase (AHASL) ayant une substitution valine, thréonine, glutamine, cystéine ou méthionine au niveau d'une position correspondant à la position 122 de SEQ ID n° 1 ou la position 90 de SEQ ID n° 2 et une substitution glycine ou tryptophane au niveau d'une position correspondant à la position 653 de SEQ ID n° 1 ou la position 621 de SEQ ID n° 2.

2. Polynucléotide selon la revendication 1, où ledit polynucléotide code pour un polypeptide AHASL ayant une troisième substitution choisie parmi :
a) une substitution alanine, acide glutamique, sérine, phénylalanine, thréonine, acide aspartique, cystéine ou asparagine au niveau d'une position correspondant à la position 199 de SEQ ID n° 1 ou la position 167 de SEQ ID n° 2 ; et
b) une substitution sérine, alanine, acide glutamique, leucine, glutamine, arginine, valine, tryptophane, tyrosine ou isoleucine au niveau d'une position correspondant à la position 197 de SEQ ID n° 1 ou la position 165 de SEQ ID n° 2.

3. Polynucléotide selon la revendication 1 ou 2, où le polypeptide AHASL possède une séquence d'acides aminés qui possède au moins 80% d'identité par rapport à l'une quelconque parmi les séquences d'acides aminés exposées dans SEQ ID n° 1-25.

4. Polynucléotide codant pour un polypeptide à substitution d'acides aminés de la grande sous-unité de l'acétohydroxyacide synthase (AHASL) ayant une substitution valine, thréonine, glutamine, cystéine ou méthionine au niveau d'une position correspondant à la position 122 de SEQ ID n° 1 ou la position 90 de SEQ ID n° 2 et une substitution phénylalanine, asparagine, thréonine, glycine, valine, isoleucine ou tryptophane au niveau d'une position correspondant à la position 653 de SEQ ID n° 1 ou la position 621 de SEQ ID n° 2 ;
où le polypeptide AHASL possède une séquence d'acides aminés qui possède au moins 80% d'identité par rapport à l'une quelconque parmi les séquences d'acides aminés exposées dans SEQ ID n° 1-4 et 8-25.

5. Polynucléotide selon la revendication 4, où le polypeptide AHASL codé possède une substitution thréonine au niveau d'une position correspondant à la position 122 de SEQ ID n° 1 ou la position 90 de SEQ ID n° 2 et une substitution asparagine au niveau d'une position correspondant à la position 653 de SEQ ID n° 1 ou la position 621 de SEQ ID n° 2.

6. Polynucléotide selon la revendication 4 ou 5, où ledit polynucléotide code pour un polypeptide AHASL ayant une troisième substitution choisie parmi :
a) une substitution alanine, acide glutamique, sérine, phénylalanine, thréonine, acide aspartique, cystéine ou asparagine au niveau d'une position correspondant à la position 199 de SEQ ID n° 1 ou la position 167 de SEQ ID n° 2 ; et
b) une substitution sérine, alanine, acide glutamique, leucine, glutamine, arginine, valine, tryptophane, tyrosine ou isoleucine au niveau d'une position correspondant à la position 197 de SEQ ID n° 1 ou la position 165 de SEQ ID n° 2.

7. Polynucléotide selon l'une quelconque des revendications 4-6, où le polypeptide AHASL codé possède une séquence d'acides aminés qui possède au moins 80% d'identité par rapport à l'une quelconque parmi les séquences d'acides aminés exposées dans SEQ ID n° 1-4, 8-20, 24 et 25.

8. Polynucléotide selon l'une quelconque des revendications 1-7, où ledit polynucléotide est recombiné ou mutagénisé.

9. Vecteur d'expression comprenant le polynucléotide selon l'une quelconque des revendications 1-8 et capable d'exprimer ledit polynucléotide dans une cellule hôte.

10. Plante transgénique comprenant le vecteur d'expression selon la revendication 9.

11. Plante transgénique selon la revendication 10, où la plante est une monocotylédone.

12. Plante transgénique selon la revendication 10, où la plante est une dicotylédone.

13. Plante transgénique selon la revendication 10, où la plante est choisie parmi *Arabidopsis thaliana*, le maïs, le seigle, l'avoine, le triticale, le riz, l'orge, le sorgho, le millet, la canne à sucre, le soja, la betterave sucrière, l'arachide, le coton, le colza, le canola, les espèces de *Brassica,* le manioc, le melon, la courge, le poivron, le tagète, les plantes de type Solanacées, la pomme de terre, la patate douce, le tabac, l'aubergine, la tomate, les espèces de *Vicia,* le pois, la luzerne, le café, le cacao, le thé, les espèces de *Salix*, le palmier à huile, la noix de coco, et les graminées vivaces.

14. Graine végétale produite par la plante transgénique selon l'une quelconque des revendications 10-13, où la graine comprend le polynucléotide selon l'une quelconque des revendications 1-8.

15. Plante transgénique selon l'une quelconque des revendications 10-14, dans laquelle l'expression du polynucléotide dans la plante entraîne une tolérance vis-à-vis d'un herbicide choisi parmi les imidazolinones, les sulfonylurées, les triazolopyrimidines, et les oxybenzoates de pyrimidinyle.

16. Protéine AHASL purifiée codée par le polynucléotide selon l'une quelconque des revendications 1-8.

17. Plante comprenant :
a) un premier polynucléotide codant pour un premier polypeptide AHASL ayant une première substitution d'acides aminés et un deuxième polynucléotide codant pour un deuxième polypeptide AHASL ayant une première substitution d'acides aminés, ou
b) un polynucléotide codant pour un polypeptide AHASL ayant les deux, ladite première et ladite deuxième, substitutions d'acides aminés ;
ladite première substitution d'acides aminés étant une valine, une thréonine, une glutamine, une cystéine ou une méthionine pour l'acide aminé au niveau de la position correspondant à la position 122 de SEQ ID n° 1 ou la position 90 de SEQ ID n° 2 ; et ladite deuxième substitution d'acides aminés étant une glycine ou un tryptophane pour l'acide aminé au niveau de la position correspondant à la position 653 de SEQ ID n° 1 ou la position 621 de SEQ ID n° 2.

18. Plante non transgénique, comprenant :
a) un premier polynucléotide codant pour un premier polypeptide AHASL ayant une première substitution d'acides aminés et un deuxième polynucléotide codant pour un deuxième polypeptide AHASL ayant une deuxième substitution d'acides aminés, ou
b) un polynucléotide codant pour un polypeptide AHASL ayant les deux, ladite première et ladite deuxième, substitutions d'acides aminés ;
ladite première substitution d'acides aminés étant une valine, une thréonine, une glutamine, une cystéine ou une méthionine pour l'acide aminé au niveau de la position correspondant à la position 122 de SEQ ID n° 1 ou la position 90 de SEQ ID n° 2 ; et ladite deuxième substitution d'acides aminés étant une phénylalanine, une asparagine, une thréonine, une glycine, une valine ou un tryptophane pour l'acide aminé au niveau de la position correspondant à la position 653 de SEQ ID n° 1 ou la position 621 de SEQ ID n° 2 ;
où ladite plante est choisie parmi *Arabidopsis thaliana*, le maïs, le blé, le seigle, l'avoine, le triticale, le riz, l'orge, le sorgho, le millet, la betterave sucrière, la canne à sucre, le soja, l'arachide, le coton, le colza, le canola, les espèces de *Brassica,* le manioc, le tagète, les plantes de type Solanacées, la pomme de terre, la patate douce, le tabac, l'aubergine, la tomate, les espèces de *Vicia*, le pois, la luzerne, le café, le cacao, le thé, les espèces de *Salix*, le palmier à huile, la noix de coco, et les graminées vivaces.

19. Plante selon la revendication 18, dans laquelle la première substitution d'acides aminés est une thréonine pour l'acide aminé au niveau de la position correspondant à la position 122 de SEQ ID n° 1 ou la position 90 de SEQ ID n° 2 ; et la deuxième substitution est une asparagine pour l'acide aminé au niveau de la position correspondant à la position 653 de SEQ ID n° 1 ou la position 621 de SEQ ID n° 2.

20. Plante comprenant :
i) un premier polynucléotide codant pour un premier polypeptide AHASL ayant une première substitution d'acides aminés, un deuxième polynucléotide codant pour un deuxième polypeptide AHASL ayant une deuxième substitution d'acides aminés, et un troisième polynucléotide codant pour un troisième polypeptide AHASL ayant une troisième substitution d'acides aminés ;
ii) un premier polynucléotide codant pour un premier polypeptide AHASL ayant la première substitution d'acides aminés et la deuxième substitution d'acides aminés, et un deuxième polynucléotide codant pour un deuxième polypeptide AHASL ayant la troisième substitution d'acides aminés ;
iii) un premier polynucléotide codant pour un premier polypeptide AHASL ayant la première substitution d'acides aminés et la troisième substitution d'acides aminés, et un deuxième polynucléotide codant pour un deuxième polypeptide AHASL ayant la deuxième substitution d'acides aminés ; ou
iv) un polynucléotide codant pour un polypeptide AHASL ayant la première, la deuxième et la troisième substitution d'acides aminés ;
où lesdites première, deuxième et troisième substitutions d'acides aminés sont choisies parmi :
a) la première substitution d'acides aminés étant une valine, une thréonine, une glutamine, une cystéine ou une méthionine pour l'acide aminé au niveau de la position correspondant à la position 122 de SEQ ID n° 1 ou la position 90 de SEQ ID n° 2 ; la deuxième substitution d'acides aminés étant une alanine, un acide glutamique, une sérine, une phénylalanine, une thréonine, un acide aspartique, une cystéine ou une asparagine pour l'acide aminé au niveau de la position correspondant à la position 199 de SEQ ID n° 1 ou la position 167 de SEQ ID n° 2 ; et la troisième substitution d'acides aminés étant une glycine ou un tryptophane pour l'acide aminé au niveau de la position correspondant à la position 653 de SEQ ID n° 1 ou la position 621 de SEQ ID n° 2 ; ou
b) la première substitution d'acides aminés étant une valine, une thréonine, une glutamine, une cystéine ou une méthionine pour l'acide aminé au niveau de la position correspondant à la position 122 de SEQ ID n° 1 ou la position 90 de SEQ ID n° 2 ; la deuxième substitution d'acides aminés étant une sérine, une alanine, un acide glutamique, une leucine, une glutamine, une arginine, une valine, un tryptophane, une tyrosine ou une isoleucine pour l'acide aminé au niveau de la position correspondant à la position 197 de SEQ ID n° 1 ou la position 165 de SEQ ID n° 2 ; et la troisième substitution d'acides aminés étant une glycine ou un tryptophane pour l'acide aminé au niveau de la position correspondant à la position 653 de SEQ ID n° 1 ou la position 621 de SEQ ID n° 2.

21. Plante non transgénique, comprenant :
i) un premier polynucléotides codant pour un premier polypeptide AHASL ayant une première substitution d'acides aminés, et un deuxième polynucléotide codant pour un deuxième polypeptide AHASL ayant une deuxième substitution d'acides aminés et une troisième substitution d'acides aminés ;
ii) un premier polynucléotide codant pour un premier polypeptide AHASL ayant la première substitution d'acides aminés et la deuxième substitution d'acides aminés, et un deuxième polynucléotide codant pour un deuxième polypeptide AHASL ayant la troisième substitution d'acides aminés ;
iii) un premier polynucléotide codant pour un premier polypeptide AHASL ayant la première substitution d'acides aminés et la troisième substitution d'acides aminés, et un deuxième polynucléotide codant pour un deuxième polypeptide AHASL ayant la deuxième substitution d'acides aminés ;
iv) un polynucléotide codant pour un polypeptide AHASL ayant la première, la deuxième et la troisième substitution d'acides aminés ;
où lesdites première, deuxième et troisième substitutions d'acides aminés sont choisies parmi :
a) la première substitution d'acides aminés étant une valine, une thréonine, une glutamine, une cystéine ou une méthionine pour l'acide aminé au niveau de la position correspondant à la position 122 de SEQ ID n° 1 ou la position 90 de SEQ ID n° 2 ; la deuxième substitution d'acides aminés étant une alanine, un acide glutamique, une sérine, une phénylalanine, une thréonine, un acide aspartique, une cystéine ou une asparagine pour l'acide aminé au niveau de la position correspondant à la position 199 de SEQ ID n° 1 ou la position 167 de SEQ ID n° 2 ; et la troisième substitution d'acides aminés étant une phénylalanine, une asparagine, une thréonine, une glycine, une valine ou un tryptophane pour l'acide aminé au niveau de la position correspondant à la position 653 de SEQ ID n° 1 ou la position 621 de SEQ ID n° 2 ; ou
b) la première substitution d'acides aminés étant une valine, une thréonine, une glutamine, une cystéine ou une méthionine pour l'acide aminé au niveau de la position correspondant à la position 122 de SEQ ID n° 1 ou la position 90 de SEQ ID n° 2 ; la deuxième substitution d'acides aminés étant une sérine, une alanine, un acide glutamique, une leucine, une glutamine, une arginine, une valine, un tryptophane, une tyrosine ou une isoleucine pour l'acide aminé au niveau de la position correspondant à la position 197 de SEQ ID n° 1 ou la position 165 de SEQ ID n° 2 ; et la troisième substitution d'acides aminés étant une phénylalanine, une asparagine, une thréonine, une glycine, une valine ou un tryptophane pour l'acide aminé au niveau de la position correspondant à la position 653 de SEQ ID n° 1 ou la position 621 de SEQ ID n° 2 ;
où ladite plante est choisie parmi *Arabidopsis thaliana*, le maïs, le blé, le seigle, l'avoine, le triticale, le riz, l'orge, le sorgho, le millet, la betterave sucrière, la canne à sucre, le soja, l'arachide, le coton, le colza, le canola, les espèces de *Brassica*, le manioc, le tagète, les plantes de type Solanacées, la pomme de terre, la patate douce, le tabac, l'aubergine, la tomate, les espèces de *Vicia*, le pois, la luzerne, le café, le cacao, le thé, les espèces de *Salix*, le palmier à huile, la noix de coco, et les graminées vivaces.

22. Plante selon l'une quelconque des revendications 18, 19 et 21, où ladite plante est choisie parmi *Arabidopsis thaliana*, le maïs, le seigle, l'avoine, le triticale, le riz, l'orge, le sorgho, le millet, la betterave sucrière, la canne à sucre, le soja, l'arachide, le coton, le colza, le canola, les espèces de *Brassica,* le manioc, le tagète, les plantes de type Solanacées, la pomme de terre, la patate douce, le tabac, l'aubergine, la tomate, les espèces de *Vicia,* le pois, la luzerne, le café, le cacao, le thé, les espèces de *Salix*, le palmier à huile, la noix de coco, et les graminées vivaces.

23. Plante selon l'une quelconque des revendications 17 et 20, où la plante est une monocotylédone.

24. Plante selon l'une quelconque des revendications 17 et 20, où la plante est une dicotylédone.

25. Plante selon l'une quelconque des revendications 17-24, dans laquelle au moins l'un parmi ledit ou lesdits polynucléotides est mutagénisé.

26. Graine végétale produite par la plante selon l'une quelconque des revendications 17-25, où la graine comprend le ou les polynucléotides AHASL de la plante selon l'une quelconque des revendications 17-25.

27. Plante selon l'une quelconque des revendications 17 et 20, où la plante est choisie parmi *Arabidopsis thaliana*, le maïs, le blé, le seigle, l'avoine, le triticale, le riz, l'orge, le sorgho, le millet, la betterave sucrière, la canne à sucre, le soja, l'arachide, le coton, le colza, le canola, les espèces de *Brassica*, le manioc, le tagète, les plantes de type Solanacées, la pomme de terre, la patate douce, le tabac, l'aubergine, la tomate, les espèces de *Vicie*, le pois, la luzerne, le café, le cacao, le thé, les espèces de *Salix,* le palmier à huile, la noix de coco, et les graminées vivaces.

28. Méthode de production d'une plante transgénique, comprenant les étapes consistant à :
i) transformer une cellule végétale par le vecteur d'expression selon la revendication 9 ; et
ii) régénérer, à partir de la cellule végétale, une plante transgénique qui exprime le polypeptide mutant AHASL.

29. Méthode d'identification ou de sélection d'une cellule végétale, d'un tissu végétal, d'une graine végétale, d'une plante ou d'une partie de celle-ci, comprenant les étapes consistant à :
i) fournir une cellule végétale, un tissu végétal, une graine végétale, une plante ou une partie de celle-ci, où ladite cellule végétale, ledit tissu végétal, ladite graine végétale, ladite plante ou ladite partie de celle-ci comprend le polynucléotide selon l'une quelconque des revendications 1-8 ou le ou les polynucléotides de la plante selon l'une quelconque des revendications 10-13, 15, 17-25 et 27 ;
ii) mettre en contact la cellule végétale, le tissu végétal, la graine végétale, la plante ou la partie de celle-ci, avec au moins un composé d'inhibition d'AHAS ; et
iii) déterminer si la cellule végétale, le tissu végétal, la graine végétale, la plante ou la partie de celle-ci est affecté(e) par le composé d'inhibition d'AHAS, identifiant ou sélectionnant ainsi la cellule végétale, le tissu végétal, la graine végétale, la plante ou la partie de celle-ci.

30. Graine de la plante selon l'une quelconque des revendications 10-13, 15, 17-25 et 27, où ladite graine est traitée par un herbicide inhibiteur d'AHAS.

31. Méthode de préparation d'une graine destinée à la croissance d'une plante, tout en contrôlant les adventices dans l'entourage de ladite plante, la méthode comprenant la mise en contact d'une graine de la plante selon l'une quelconque des revendications 10-13, 15, 17-25 et 27, avec un herbicide inhibiteur d'AHAS, avant semis et/ou après prégermination de la graine.

32. Utilisation d'une plante selon l'une quelconque des revendications 10-13, 15, 17-25 et 27, dans une méthode de contrôle d'adventices.

33. Utilisation selon la revendication 32, dans laquelle ladite méthode destinée au contrôle d'adventices comprend la mise en contact de ladite plante et desdites adventices dans l'entourage de ladite plante, avec une quantité efficace d'un herbicide inhibiteur d'AHAS vis-à-vis duquel ladite plante est tolérante, et qui inhiberait la croissance d'une plante de type sauvage correspondante, contrôlant ainsi les adventices.

34. Méthode d'identification d'une plante selon l'une quelconque des revendications 10-13, 15, 17-25 et 27, ladite méthode comprenant :
(A) la fourniture d'un matériel biologique à partir d'une plante,
(B) la mise en oeuvre d'une PCR ou d'un test d'hybridation des gènes d'AHASL dans ledit matériel biologique afin de déterminer si le matériel biologique comprend le ou les polynucléotides de la plante selon l'une quelconque des revendications 10-13, 15, 17-25 et 27, et
(C) l'identification, sur la base des résultats de l'étape (B), que la plante de l'étape (A) est une plante selon l'une quelconque des revendications 10-13, 15, 17-25 et 27.

35. Méthode selon la revendication 34, dans laquelle ledit matériel biologique est une graine végétale.

36. Utilisation de la plante selon l'une quelconque des revendications 10-13, 15, 17-25 et 27 ou de la graine selon l'une quelconque des revendications 14, 26 et 30, dans une méthode de production d'huile de graines.
